(19) Europäisches Patentamt
European Patent Office
Office européen des brevets

(11) EP 1 359 142 A1

(12) **EUROPEAN PATENT APPLICATION**
published in accordance with Art. 158(3) EPC

(43) Date of publication:
**05.11.2003 Bulletin 2003/45**

(21) Application number: **02711282.0**

(22) Date of filing: **01.02.2002**

(51) Int Cl.[7]: **C07C 311/24**, C07C 303/36, C07F 7/12, C08F 214/26, C08F 216/14, H01M 8/02

(86) International application number:
**PCT/JP02/00854**

(87) International publication number:
**WO 02/062749 (15.08.2002 Gazette 2002/33)**

(84) Designated Contracting States:
**AT BE CH CY DE DK ES FI FR GB GR IE IT LI LU MC NL PT SE TR**
Designated Extension States:
**AL LT LV MK RO SI**

(30) Priority:
**01.02.2001 JP 2001025018**
**07.02.2001 JP 2001030955**
**13.09.2001 JP 2001278418**
**07.11.2001 JP 2001342172**
**08.11.2001 JP 2001343780**
**08.11.2001 JP 2001343931**

(71) Applicant: **Asahi Kasei Kabushiki Kaisha**
**Osaka-shi, Osaka 530-8205 (JP)**

(72) Inventors:
- **IKEDA, Masanori**
**Fuji-shi, Shizuoka 417-0801 (JP)**
- **HOSHI, Nobuto**
**Fuji-shi, Shizuoka 416-0939 (JP)**
- **UEMATSU, Nobuyuki**
**Fuji-shi, Shizuoka 416-0939 (JP)**
- **KOGA, Takehiro**
**Fuji-shi, Shizuoka 416-0939 (JP)**

(74) Representative: **Strehl Schübel-Hopf & Partner**
**Maximilianstrasse 54**
**80538 München (DE)**

(54) **PERFLUOROVINYL ETHER MONOMER HAVING SULFONAMIDE GROUP**

(57) A perfluorovinyl ether monomer represented by the following formula (1):

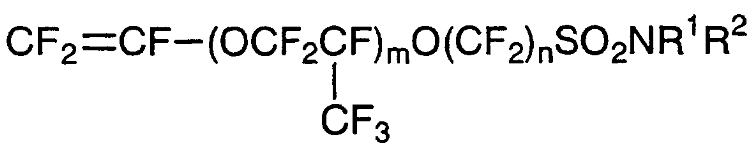

(1)

wherein: m is an integer of from 0 to 5; n is an integer of from 1 to 5; and each of $R^1$ and $R^2$ independently represents a hydrogen atom, an unsubstituted or substituted $C_1$-$C_{10}$ hydrocarbon group, or a substituted silyl group, with the proviso that, when each of $R^1$ and $R^2$ is independently the hydrocarbon group or the substituted silyl group, $R^1$ and $R^2$ are optionally bonded together, thereby forming a ring. Also disclosed are a method for producing the perfluorovinyl ether monomer; a fluorinated polymer obtained from the monomer and a method for producing the same; a polymer film obtained from the polymer; a modified or crosslinked polymer film obtained from the polymer film; and a solid polymer electrolyte membrane obtained from the modified or crosslinked polymer film.

EP 1 359 142 A1

Fig.1

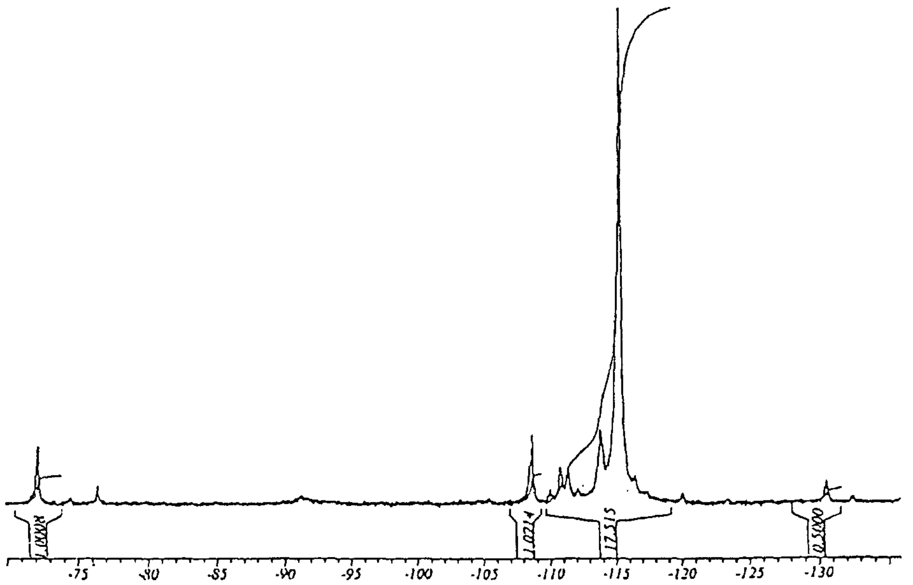


Chemical shift (ppm)

**Description**

BACKGROUND OF THE INVENTION

Field of the Invention

The present invention relates to a perfluorovinyl ether monomer. More particularly, the present invention is concerned with a perfluorovinyl ether monomer represented by the following formula (1):

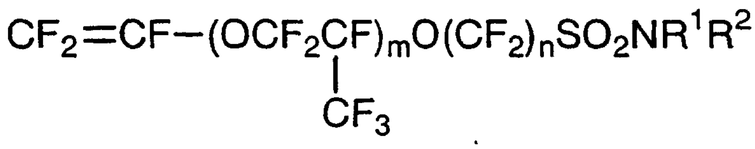

(1)

wherein:

m is an integer of from 0 to 5;
n is an integer of from 1 to 5; and
each of $R^1$ and $R^2$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the proviso that, when each of $R^1$ and $R^2$ is independently the unsubstituted or substituted $C_1$-$C_{10}$ hydrocarbon group or the substituted silyl group, $R^1$ and $R^2$ are optionally bonded together to form a divalent group, thereby forming a saturated or unsaturated nitrogen-containing heterocyclic ring in cooperation with a nitrogen atom which is bonded to $R^1$ and $R^2$.

The perfluorovinyl ether monomer of the present invention can be used for producing a fluorinated polymer which exhibits excellent properties. The fluorinated polymer can be used in various fields; for example, the fluorinated polymer can be advantageously used as a raw material for producing a solid polymer electrolyte. The solid polymer electrolyte obtained from the perfluorovinyl ether monomer of the present invention exhibits not only excellent durability, but also excellent heat resistance and high proton conductivity, and, hence, the solid polymer electrolyte can be advantageously used in a fuel cell.

The present invention is also concerned with: a method for producing -the above-mentioned perfluorovinyl ether monomer; a fluorinated polymer obtained from the perfluorovinyl ether monomer and a method for producing the fluorinated polymer; a polymer film obtained from the above-mentioned fluorinated polymer; a modified or crosslinked polymer film obtained from the above-mentioned polymer film; and a solid polymer electrolyte membrane obtained from the above-mentioned modified or crosslinked polymer film.

Prior Art

Fluororesins are used in various fields. In recent years, the use of a fluororesin as a material for use in a fuel cell is attracting attention.

In recent years, the studies for putting fuel cells to practical use have been becoming active. Especially, a fuel cell which uses a solid polymer electrolyte membrane is attracting attention because such fuel cell is advantageous not only in that it can be reduced in size and weight, but also in that high power density can be obtained at a relatively low temperature. The fuel cell using a solid polymer electrolyte membrane is now being developed especially for use in the fields relating to automobiles.

A polymer used as a raw material for producing such a solid polymer electrolyte membrane is required to exhibit an excellent proton conductivity, an appropriate water regain and a high gas barrier property against hydrogen, oxygen and the like. In order to obtain a material which satisfies the above-mentioned requirements, studies have been made on various polymers having a sulfonic acid group or a phosphonic acid group in the main chain or at the terminals of the side chain thereof, and various materials have been proposed (see, for example, O. Savadogo, Journal of New

Materials for Electrochemical Systems I, 47-66 (1998) (Canada)).

When a fuel cell using a solid polymer electrolyte membrane is operated, active oxygen species are generated at an electrode positioned near the solid polymer electrolyte membrane, and thus, the solid polymer electrolyte membrane is exposed to stringent oxidative conditions. Therefore, for stably operating such a fuel cell for a long period of time, it is necessary that the solid polymer electrolyte membrane used in the fuel cell be made of a material which exhibits excellent durability under oxidative conditions.

Many of the materials which have heretofore been proposed as a raw material for producing a solid polymer electrolyte membrane are hydrocarbon materials. Hydrocarbon materials do not have a satisfactory durability under the above-mentioned oxidative conditions. Therefore, although a fuel cell which uses a solid polymer electrolyte membrane made of a hydrocarbon material generally exhibits excellent properties in the early stage of the operation, it has frequently been pointed out that the performance of such fuel cell is likely to become poor when the fuel cell is operated for a long period of time. For this reason, at present, in the studies for putting fuel cells to practical use, fluororesins are mainly used as a raw material for producing a solid polymer electrolyte membrane. Among the fluororesins used as a raw material for producing a solid polymer electrolyte membrane, a perfluoropolymer represented by the following formula (I) is widely used:

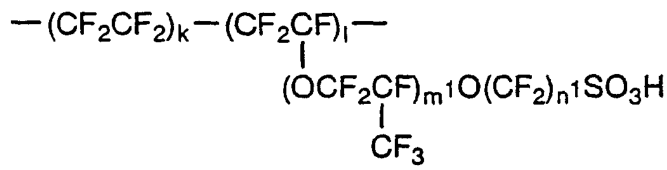

(I)

wherein each of $m^1$ and $n^1$ is a positive integer. The polymer (I) is obtained by subjecting a copolymer of a perfluorovinyl ether monomer represented by the following formula (II) and tetrafluoroethylene (TFE) to a hydrolysis reaction:

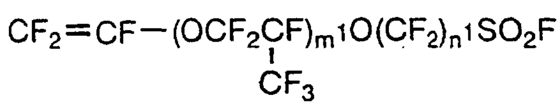

(II).

wherein each of $m^1$ and $n^1$ is a positive integer. The above-mentioned hydrolysis reaction is performed by treating the above-mentioned copolymer with an alkaline substance, such as NaOH and KOH, to thereby convert the $-SO_2F$ group at the terminal of the side chain thereof to an $-SO_3Na$ group, an $-SO_3K$ group or the like, followed by treatment with an acid, such as hydrochloric acid, to thereby further convert the terminal group of the side chain thereof to a free sulfonic acid group ($-SO_3H$).

When the above-mentioned polymer (I) is used as a raw material for producing a solid polymer electrolyte membrane, the solid polymer electrolyte membrane is generally produced by a method in which the above-mentioned copolymer is melt-molded to thereby form a film, and the obtained film is subjected to the above-mentioned hydrolysis reaction.

Among the polymers of the above-mentioned formula (I), a polymer in which $m^1$ = 1 and $n^1$ = 2 to 3, is widely used. Such polymer is produced by using as a raw material the above-mentioned monomer (II) in which $m^1$ = 1 and $n^1$ = 2 to 3. Such monomer is produced by a method as shown in the scheme below:

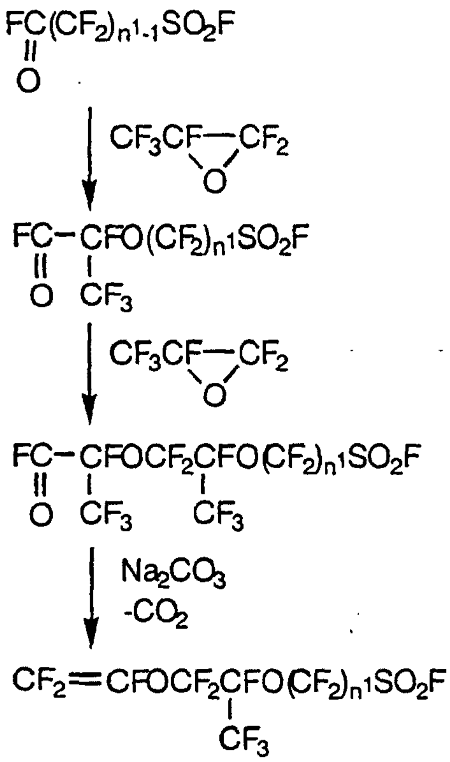


Specifically, first, an acyl fluoride having a fluorosulfonyl group is reacted with two molecules of hexafluoropropylene oxide to thereby obtain an intermediate. The intermediate is then treated with sodium carbonate, to thereby eliminate one fluoroformyl group and one fluorine atom from the intermediate and form a perfluorovinyl group. Thus, the desired perfluorovinyl ether monomer is obtained (hereinafter, such a reaction in which a carboxyl group or a group similar to a carboxyl group, and another leaving group are eliminated from an intermediate to thereby form a C=C double bond, is referred to as "decarboxylation-vinylation").

It is preferred that the above-mentioned polymer (I) has a small $m^1$ value. When such polymer (I) having a small $m^1$ value is used for producing a polymer film, the obtained film exhibits satisfactorily high strength even when the density of the sulfonic acid groups is high, wherein the sulfonic acid groups function as ion exchange groups. Therefore, such polymer as mentioned above is suitable for producing a solid polymer electrolyte membrane which has excellent properties with respect to ion conductivity and mechanical strength.

From the above, it is considered that it is especially preferred that $m^1 = 0$ in the above-mentioned polymer (I). Such polymer can be produced by copolymerizing a perfluorovinyl ether monomer represented by the following formula (III) with TFE:

$$CF_2=CFO(CF_2)_nSO_2F \qquad \text{(III)}$$

wherein n is 2 or 3.

From the above-mentioned method using decarboxylation-vinylation, it is presumed that the above-mentioned polymer (III) can be obtained by subjecting a compound represented by the following formula (IV) to decarboxylation-vinylation:

$$CF_3CF(COF)O(CF_2)_nSO_2F \qquad \text{(IV)}$$

wherein n is 2 or 3.

However, it is known that, in actuality, when the above-mentioned compound (IV) is treated with sodium carbonate, a cyclization reaction proceeds as a side reaction, thus forming a large amount of by-products having 5- or 6-membered rings. Therefore, the yield of the desired monomer (III) becomes extremely low. Indeed, when n = 3 in formula (III), the yield of monomer (III) is at most only about 50 % because of the occurrence of the cyclization reaction. Further, when n = 2 in formula (III), substantially no reaction occurs except the cyclization reaction, and substantially no monomer (III) can be obtained.

Various methods have been proposed in which the cyclization is suppressed to thereby improve the yield of monomer (III). For example, in Unexamined Japanese Patent Application Laid-Open Specification No. Sho 57-28024, a method is disclosed in which an acyl fluoride represented by the following formula:

$$FCOCF_2SO_2F$$

is reacted with an epoxide represented by the following formula (V):

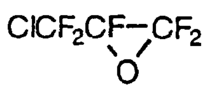

(V)

thereby obtaining a compound represented by the following formula:

$$ClCF_2CF(COF)OCF_2CF_2SO_2F,$$

and the obtained compound is subjected to decarboxylation-vinylation to thereby obtain the above-mentioned monomer (III) (n = 2). However, this method has a problem in that the method for producing the above-mentioned epoxide (V) is complicated.

In WO98/43952, a method is disclosed in which the above-mentioned compound (IV) (n = 2) is reacted with NaOH, thereby converting the compound (IV) into a compound represented by the following formula:

$$CF_3CF(CO_2Na)OCF_2CF_2SO_3Na.$$

The thus obtained compound is heated to thereby cause decarboxylation-vinylation and obtain a sulfonate represented by the following formula (VI):

$$CF_2{=}CFOCF_2CF_2SO_3Na \text{ (VI).}$$

However, the sulfonate (VI) cannot be purified by distillation, so that it is difficult to produce a highly purified sulfonate (VI) product on a commercial scale. In this document, the sulfonate (VI) is purified simply by washing with a solvent. However, by this purification method, the sulfonate (VI) cannot be purified to a satisfactory level.

Further, the above-mentioned document describes a method in which the above-mentioned sulfonate (VI) is copolymerized with TFE to thereby obtain a copolymer having a sulfonic acid group in the form of a salt (i.e., a sulfonate group). Such a sulfonate group can be converted into a free sulfonic acid group by treating the copolymer with an acid. However, each of the sulfonate group-containing copolymer and the free sulfonic acid group-containing copolymer has poor properties with respect to melt fluidity and heat stability, and, hence, it is difficult to formulate the copolymer into a film.

The above document further describes that the above-mentioned sulfonate (VI) can be converted to the above-mentioned monomer (III) (n = 2) by a conventional process. However, this conventional process is disadvantageous not only in that it is a complicated process involving a plurality of steps, but also in that the yield in each of the steps of the process is low, and the purification of the monomer (III) is difficult. Thus, it is not practical to employ the above conventional process to obtain the monomer (III).

In Examined Japanese Patent Application Publication No. Sho 47-2083, a method is disclosed in which the above-mentioned compound (IV) (n = 2) is treated with sodium carbonate to perform the above-mentioned cyclization

reaction selectively, thereby obtaining a cyclic product represented by the following formula:

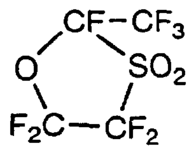

Then, the obtained cyclic product is reacted with $NaOCH_3$ to thereby obtain the above-mentioned sulfonate (VI). However, this method has the same problems as in the method disclosed in the above-mentioned WO98/43952.

As is apparent from the above, there has conventionally been no method which can be used for efficiently producing the above-mentioned valuable monomer (III). Therefore, heretofore, there has been no practical method for producing the above-mentioned polymer (I) ($m^1 = 0$) which is a copolymer of the monomer (III) with TFE.

Thus, conventionally, it has been impossible to improve the performance of a fuel cell to a satisfactory level.

With respect to the above-mentioned fuel cell, it is considered to be advantageous to operate the fuel cell at high temperatures. More specifically, such operation of the fuel cell at high temperatures is considered to be advantageous, for example, in that, theoretically, the electricity generation efficiency can be improved, and that heat can be recovered from the exhausted high temperature gas, and the recovered heat can be used for room heating and the like. Therefore, it is required that a polymeric material, which is used for producing a solid polymer electrolyte membrane used in a fuel cell, exhibits excellent properties at high temperatures.

However, with respect to the polymers which have conventionally been used as a raw material for producing a solid polymer electrolyte membrane used in a fuel cell, the properties thereof at high temperatures, especially mechanical strength, are unsatisfactory. For example, when the above-mentioned polymer (I), in which k/l = 3 to 10, $m^1 = 1$ and $n^1 = 2$, is used as a raw material for producing a solid polymer electrolyte membrane, the obtained solid polymer electrolyte membrane exhibits an unsatisfactory mechanical strength at high temperatures. Therefore, when such a solid polymer electrolyte membrane is used in a fuel cell, it is difficult to operate the fuel cell at high temperatures, for example, at 100 °C or more. Thus, at present, it is desired to improve the high temperature properties (i.e., the heat resistance) of the polymeric material used for producing a solid polymer electrolyte membrane.

As an example of effective methods for improving the heat resistance of the polymer, there can be mentioned a method in which a crosslinked structure is introduced into the polymer to obtain a crosslinked polymer. As an example of a crosslinked polymer film composed of a crosslinked form of a polymer having a sulfonic acid group, such as the above-mentioned polymer (I), there can be mentioned a crosslinked polymer film which is crosslinked through a bis-sulfonyl imide linkage ($-SO_2NHSO_2-$). With respect to the method for producing such a crosslinked polymer film, various proposals have been made as mentioned below.

In Unexamined Japanese Patent Application Laid-Open Specification Nos. 2000-188013 and 2001-319521, and Japanese Patent Application prior-to-examination Publication (Tokuhyo) No. 2001-522401, a method is disclosed in which a crosslinked polymer film is obtained by reacting a polymer (in the form of a film) having $-SO_2F$ groups at terminals of its side chains with a crosslinking agent, thereby introducing the bis-sulfonyl imide linkage into the polymer film.

Specifically, in the above-mentioned Unexamined-Japanese Patent Application Laid-Open Specification Nos. 2000-188013 and Japanese Patent Application prior-to-examination Publication (Tokuhyo) No. 2001-522401, a method is disclosed in which a polymer (in the form of a film) having $-SO_2F$ groups at terminals of its side chains is treated with a specific bifunctional crosslinking agent mentioned below to thereby produce a crosslinked polymer film. In this method, as shown in the following formula (VII), each of the two $(CH_3)_3SiN(Na)$ groups of a bifunctional crosslinking agent (A-2) is reacted with a $-SO_2F$ group at a terminal of the side chain of a polymer (A-1) to thereby form two bis-sulfonyl imide linkages, so that a crosslinked polymer (A-3) is obtained.

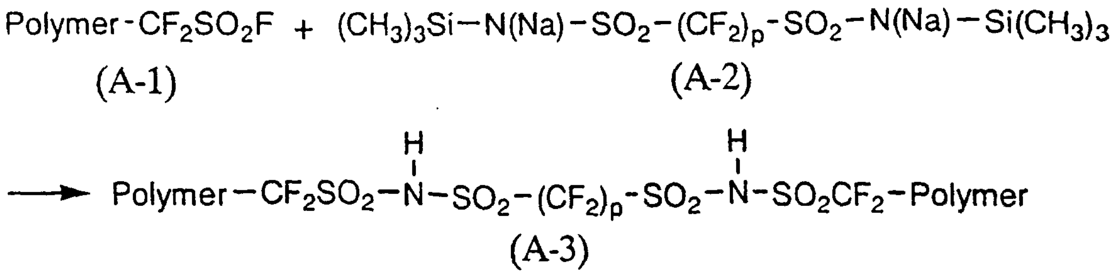

(VII).

In this method, a crosslinked structure is introduced into the polymer by utilizing the reactivity of the $-SO_2F$ group. However, when this method is practiced on a commercial scale, there arises a problem that the compound represented by the following formula (VIII), which is used as a raw material for producing the bifunctional crosslinking agent (A-2), is difficult to obtain in an amount sufficient to practice the production of the crosslinked polymer on a commercial scale:

$$FSO_2\text{-}(CF_2)_p\text{-}SO_2F \qquad \text{(VIII).}$$

Further, the bifunctional crosslinking agent (A-2) is a highly polar substance having ionic bonds. On the other hand, a perfluoropolymer, such as the polymer (I), is a low polarity substance, and such a perfluoropolymer scarcely dissolves or swells in any solvents other than a fluorine-containing solvent having a low polarity. Therefore, the crosslinking agent (A-2) has low affinity to the polymer (A-1). For this reason, it is difficult to cause the crosslinking agent (A-2) to permeate throughout the polymer (I) uniformly within a short period of time, so that a crosslinked structure cannot be efficiently introduced into the polymer (I).

Further, the above-mentioned Unexamined Japanese Patent Application Laid-Open Specification Nos. 2000-188013 and 2001-319521, and Japanese Patent Application prior-to-examination Publication (Tokuhyo) No. 2001-522401 describe the use of $NaN(SiMe_3)_2$, $LiN(SiMe_3)_2$ and the like as a crosslinking agent used for introducing a crosslinked structure into a polymer having $-SO_2F$ groups at the terminals of its side chains. However, as in the case of the above-mentioned crosslinking agent (A-2), the above-mentioned compounds, such as $NaN(SiMe_3)_2$ and $LiN(SiMe_3)_2$, also have low affinity to low polarity polymers, such as a perfluoropolymer. Therefore, it is difficult to cause the crosslinking agent (A-2) to permeate throughout the polymer (I) uniformly within a short period of time, so that a crosslinked structure cannot be efficiently introduced into the polymer (I). As a result, a crosslinking reaction occurs only on and near the surface of the polymer, and, hence, a uniformly crosslinked polymer cannot be obtained. In actuality, in the above-mentioned Unexamined Japanese Patent Application Laid-Open Specification No. 2001-319521, data are shown which indicate that a crosslinking reaction is likely to occur only on and near the surface of the polymer.

Furthermore, in the above-mentioned Japanese Patent Application prior-to-examination Publication (Tokuhyo) No. 2001-522401, a method is disclosed in which an ionic crosslinking agent as mentioned above is kneaded with a polymer having $-SO_2F$ groups at the terminals of its side chains, thereby obtaining a composition, and the obtained composition is used for forming a film. However, by this method, it is difficult to disperse the ionic crosslinking agent having high polarity uniformly throughout the low polarity polymer. Further, by this method, a crosslinking reaction proceeds during the kneading of the crosslinking agent and the polymer at high temperatures, thereby forming a crosslinked polymer. In general, it is difficult to formulate a crosslinked polymer into a film. Thus, in actuality, it is difficult to employ this method for producing a crosslinked polymer film.

The above-mentioned Unexamined Japanese Patent Application Laid-Open Specification No. 2000-188013 further describes a method in which a perfluoropolymer having $-SO_2F$ groups is directly reacted with a perfluropolymer having an NH group-containing sulfonamido group (this polymer is obtained by reacting the former perfluoropolymer with ammonia or a primary amine) to thereby obtain a crosslinked polymer. However, as a result of the studies of the present inventors, it has been found that it is extremely difficult to perform this reaction efficiently. The reason for this difficulty is not clear, but this difficulty is considered to be mainly caused due to the poor compatibility between the two polymers.

The above-mentioned Unexamined Japanese Patent Application Laid-Open Specification No. 2001-319521 further describes a method in which ammonia is used as a crosslinking agent. However, as a result of the studies of the present inventors, it has been found that, since ammonia exhibits low reactivity, excess amount of ammonia is needed to practice this method. Therefore, in this method, it is extremely difficult to control the amount of bis-sulfonyl imide linkages formed in the polymer (i.e., the crosslinking density of the polymer).

In addition, when water gets mixed into a reaction system involved in the above-mentioned method using ammonia as a crosslinking agent, a reaction of the water with the -$SO_2F$ group becomes predominant over the crosslinking reaction, thereby forming a sulfonic acid group in the form of an ammonium salt, while suppressing the formation of bis-sulfonyl imide linkage. Thus, the above-mentioned method using ammonia as a crosslinking agent has a problem in that, even when only a trace amount of water gets mixed into the reaction system, a sulfonic acid group becomes more likely to be formed than a bis-sulfonyl imide linkage, and thus, it becomes impossible to introduce a crosslinked structure into the polymer effectively.

In WO 01/27167, a method is disclosed in which a film of a polymer having -$SO_2F$ groups at the terminals of its side chains is subjected to amidation, thereby converting all -$SO_2F$ groups to sulfonamido groups. The converted sulfonamido groups are, then, reacted with the above-mentioned compound (VIII) to thereby obtain a crosslinked polymer film.

However, when a crosslinked structure is introduced into a polymer, in general, the movement of the polymer chain is suppressed. Therefore, it is difficult to react all of the sulfonamido groups in a polymer with the compound (VIII) by the method described in WO 01/27167 under conditions which are advantageous for a commercial scale production of the crosslinked polymer polymer. For this reason, it is considered that the crosslinked polymer obtained by this method has unreacted sulfonamido groups. As explained below in more detail, a polymer having a sulfonamido group exhibits a low proton conductivity, and hence, a crosslinked polymer (having unreacted sulfonamido groups) obtained by this method is not suitable as a raw material for producing a solid polymer electrolyte.

When the above-mentioned compound (VIII) is used in a largely excess amount in an attempt to increase the conversion of the sulfonamido groups, only the conversion of one of the -$SO_2F$ groups of the compound (VIII) is increased, and, hence, a crosslinked structure cannot be effectively introduced into the polymer.

Further, as mentioned above, the compound (VIII) is difficult to obtain in an amount sufficient to practice the production of a crosslinked polymer on a commercial scale.

In each of the above-mentioned four patent documents, namely, Unexamined Japanese Patent Application Laid-Open Specification Nos. 2000-188013 and 2001-319521, Japanese Patent Application prior-to-examination Publication (Tokuhyo) No. 2001-522401 and WO 01/27167, a method is disclosed in which a solid polymer film is reacted with a crosslinking agent. However, in general, in such a method as described in these patent documents, which involves a reaction in a heterogeneous system, the rate of diffusion of the crosslinking agent into the polymer is low. Therefore, the crosslinking reaction cannot be performed at a high rate, so that it becomes difficult to improve the productivity of the crosslinked polymer film.

Further, there is a large difference in susceptivity to the reaction between the surface of the polymer and the inside of the polymer. In general, the crosslinking reaction occurs only on the surface of the polymer, and, hence, it is likely that crosslinked structure is formed only on the surface of the polymer, and not inside the polymer. Therefore, by the above-mentioned method, it is difficult to obtain a uniformly crosslinked polymer having high quality.

When a low polarity polymer, such as a perfluoropolymer having $SO_2F$ groups, is reacted with a high polarity reagent, such as the above-mentioned crosslinking agents, it is very difficult to cause the crosslinking reaction uniformly throughout the polymer. In such a case, the crosslinking reaction tends to proceed only around a portion of the polymer at which the crosslinking reaction has first started. The reason for this is as follows.

In a reaction of a polymer with a reagent, in which the resultant reaction product exhibits a higher polarity than that of the unreacted polymer prior to the reaction, the reaction product has a higher affinity to the reagent than that of the unreacted polymer prior to the reaction. As a result, the unreacted reagent concentratedly comes around the site at which the reaction has first occurred, and, hence, the reaction around this site is biasedly promoted.

Thus, by the above-mentioned methods in which a solid polymer film is reacted with a crosslinking agent, it is difficult to obtain a uniformly crosslinked polymer film having high quality.

In Unexamined Japanese Patent Application Laid-Open Specification No. Sho 50-92339, a method is disclosed in which a crosslinked structure is introduced into a polymer by reacting a polymer having a halosulfonyl group with a diamine or a polyamine to form a sulfonamido group.

In Unexamined Japanese Patent Application Laid-Open Specification No. Sho 54-43192, a method is disclosed in which a polymer having a sulfonamido group with a nitrogen atom thereof bonded to an unsaturated hydrocarbon group is polymerized in the presence of a vinyl compound, thereby introducing a crosslinked structure into the polymer.

However, in both of the above-mentioned patent documents, it is not intended to use the crosslinked polymer film as a raw material for producing a solid polymer electrolyte membrane used in a fuel cell. Therefore, a hydrocarbon

group is introduced into the obtained crosslinked polymer. The hydrocarbon group suffers oxidative degradation by active oxygen. Therefore, the crosslinked polymer exhibits unsatisfactory durability under oxidative conditions, and it is difficult to operate a fuel cell using a solid polymer electrolyte membrane formed from the above-mentioned crosslinked polymer having a hydrocarbon group.

U.S. Patent No. 3784399 discloses a film which is suitable for use in the electrolysis of NaCl, wherein the film is produced by treating, with ammonia gas, the surface of a fluorine-containing polymer film having $-SO_2F$ groups as side chains, to thereby convert the $-SO_2F$ groups only on the surface of the film to $-SO_2NH_2$ groups.

However, the polymer film obtained in U.S. Patent No. 3784399 does not have a structure in which both $-SO_2F$ and $-SO_2NH_2$ groups are uniformly dispersed throughout the film, but has a multilayer structure composed of a polymer layer having $-SO_2F$ groups and a polymer layer having $-SO_2NH_2$ groups. Therefore, it is impossible to modify such a polymer film uniformly throughout the film by the interaction between the mutually adjacent $-SO_2F$ groups and $-SO_2NH_2$ groups.

The $-SO_2NH_2$ group is a weakly acidic group. However, as a result of the studies of the present inventors, it has been found that a polymer film having $-SO_2NH_2$ groups, as an ion exchange group, exhibits poor proton conductivity which is only 1/25 of the proton conductivity of a polymer film having free sulfonic acid groups as an ion exchange group. Therefore, in actuality, the above-mentioned polymer layer having $-SO_2NH_2$ groups functions as an insulating layer, so that the polymer film of U.S. Patent No. 3784399 is not suitable as a solid polymer electrolyte membrane.

It is considered that the above-mentioned polymer having $-SO_2NH_2$ groups can be obtained by copolymerizing monomers having sulfonamido groups. Such monomers having sulfonamido groups are mentioned in some documents; however, none of the documents describe the production of such monomers.

For example, Unexamined Japanese Patent Application Laid-Open Specification No. Sho 57-28119 discloses the structural formulae of various perfluorovinyl ether monomers which are used as a raw material for producing a fluorinated polymer having an acidic group. The structural formulae encompass a wide variety of compounds, including a perfluorovinyl ether monomer having a sulfonamido group. However, in this patent document, there is no description about the specific structure and properties of the above-mentioned monomers (including monomers having a sulfonamido group) and the method for producing the monomers. Further, this patent document has no description about a fluorinated polymer which is obtained from the monomers having a sulfonamido group. Needless to say, this patent document has no description about a method for improving the heat resistance of the fluorinatd polymer.

Further, U.S. Patent No. 3282875 discloses a perfluorovinyl ether monomer represented by the following formula (IX) and a copolymer obtained therefrom:

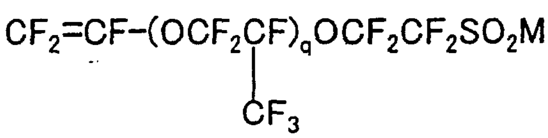

(IX)

wherein q is an integer of from 1 to 3 and M represents F, an OH group, an amino group, an ONa group or the like.

Further, this patent document discloses a method for producing a copolymer having a $-SO_2NH_2$ group, in which a copolymer obtained from monomers including the monomer (IX) having a fluorine atom as substituent M is treated with ammonia. However, in this patent document, there is no description about specific examples of monomer (IX) having an amino group as substituent M and the method for synthesizing such monomer (IX).

In the Examples of the above-mentioned U.S. patent, the monomer (IX) having a hydroxyl group or a -ONa group as substituent M is produced from the monomer (IX) having a fluorine atom as substituent M. In accordance with such method, the present inventors reacted the monomer (IX) having a fluorine atom as substituent M with ammonia or diethylamine in an attempt to produce the monomer (IX) having an amino group or a diethylamino group as substituent M. However, the present inventors could obtain no desired product, but obtain only a complex mixture. The present inventors analyzed the obtained complex mixture. From the results of the analysis, it is presumed that the complex mixture was formed by the reaction of ammonia or diethylamine with a perfluorovinyl group of the monomer (IX).

Thus, a method for efficiently producing a perfluorovinyl ether having a sulfonamido group, such as the perfluorovinyl ether monomer (1) of the present invention, has not been known at all.

As apparent from the above, there has conventionally not been obtained a fluororesin which has high heat resistance and high proton conductivity and which can be advantageously used as a raw material for producing a solid polymer electrolyte membrane for use in a fuel cell.

## SUMMARY OF THE INVENTION

In this situation, the present inventors have made extensive and intensive studies with a view toward developing a fluororesin which has excellent heat resistance and high proton conductivity and which can be advantageously used as a raw material for producing a solid polymer electrolyte membrane for use in a fuel cell and toward developing a monomer used for producing the fluororesin. As a result, unexpectedly, the present inventors have not only for the first time succeeded in producing a perfluorovinyl ether monomer which has a specific novel structure containing a sulfonamido group, but have also found that, from this monomer, there can be obtained a fluorinated polymer which exhibits excellent heat resistance and high proton conductivity and that a solid polymer electrolyte membrane having excellent heat resistance can be obtained by subjecting the fluorinated polymer to appropriate treatment. The present invention has been completed, based on these successes and findings.

Accordingly, it is an object of the present invention to provide a perfluorovinyl ether monomer which has a specific novel structure and which can be used as a raw material for producing a fluororesin which can be advantageously used for producing a solid polymer electrolyte membrane for use in a fuel cell.

It is another object of the present invention to provide a method for producing the above-mentioned perfluorovinyl ether monomer.

It is still another object of the present invention to provide a fluorinated polymer which is produced using the above-mentioned perfluorovinyl ether monomer.

It is a further object of the present invention to provide a method for producing the above-mentioned fluorinated polymer.

It is still a further object of the present invention to provide a polymer film obtained from the above-mentioned fluorinated polymer.

It is still a further object of the present invention to provide a solid polymer electrolyte membrane obtained from the above-mentioned fluorinated polymer.

The foregoing and other objects, features and advantages of the present invention will be apparent from the following detailed description taken in connection with the accompanying drawings and the appended claims.

## BRIEF DESCRIPTION OF THE DRAWINGS

In the drawings:

Fig. 1 is a chart showing the $^{19}$F-NMR spectrum of the binary polymer which is synthesized in Example 2; and
Fig. 2 is a chart showing the $^{19}$F-NMR spectrum of the unmodified terpolymer which is synthesized in Example 16.

## DETAILED DESCRIPTION OF THE INVENTION

In the present invention, there is provided a perfluorovinyl ether monomer represented by the following formula (1).

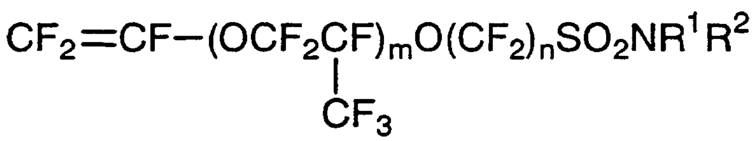
(1)

wherein:

m is an integer of from 0 to 5;
n is an integer of from 1 to 5; and
each of $R^1$ and $R^2$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the proviso that, when each of $R^1$ and $R^2$ is independently the unsubstituted or substituted $C_1$-$C_{10}$ hydrocarbon group or the substituted silyl group, $R^1$ and $R^2$ are optionally bonded together to form a divalent group, thereby forming a saturated or unsaturated nitrogen-containing heterocyclic ring in cooperation with a nitrogen

atom which is bonded to $R^1$ and $R^2$ .

For easy understanding of the present invention, the essential features and various preferred embodiments of the present invention are enumerated below.

1. A perfluorovinyl ether monomer represented by the following formula (1).

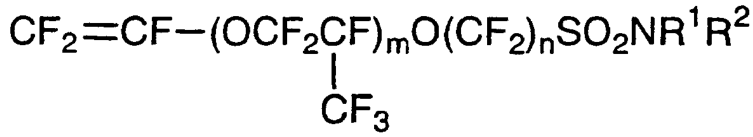

(1)

wherein:

m is an integer of from 0 to 5;
n is an integer of from 1 to 5; and
each of $R^1$ and $R^2$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the proviso that, when each of $R^1$ and $R^2$ is independently theunsubstitutedorsubstitutedCi-$C_{10}$ hydrocarbon group or the substituted silyl group, $R^1$ and $R^2$ are optionally bonded together to form a divalent group, thereby forming a saturated or unsaturated nitrogen-containing heterocyclic ring in cooperation with a nitrogen atom which is bonded to $R^1$ and $R^2$.

2. The monomer according to item 1 above, wherein $R^1$ in formula (1) is a hydrogen atom, the unsubstituted or substituted $C_1$-$C_{10}$ hydrocarbon group or the substituted silyl group and $R^2$ in formula (1) is a hydrogen atom or the substituted silyl group.

3. The monomer according to item 1 above, wherein at least one of $R^1$ and $R^2$ in formula (1) is the substituted silyl group.

4. The monomer according to item 1 above, wherein at least one of $R^1$ and $R^2$ in formula (1) is a hydrogen atom.

5. The monomer according to item 1 above, wherein each of $R^1$ and $R^2$ in formula (1) is a hydrogen atom.

6. A method for producing the monomer of item 1 above, which comprises:

(i) converting an acyl fluoride represented by the following formula (2):

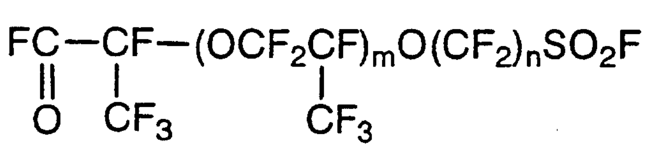

(2)

wherein m and n are as defined above for formula (1),
to a carboxylate represented by the following formula (3):

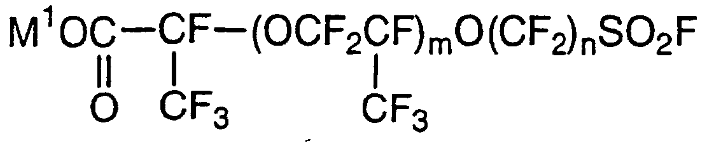

(3)

wherein:

m and n are as defined above for formula (1); and
$M^1$ is an alkali metal, an alkaline earth metal, a quaternary ammonium group or a quaternary phosphonium group;

(ii) effecting an amidation reaction of the fluorosulfonyl group of the carboxylate (3) to thereby obtain a sulfonamide represented by the following formula (4):

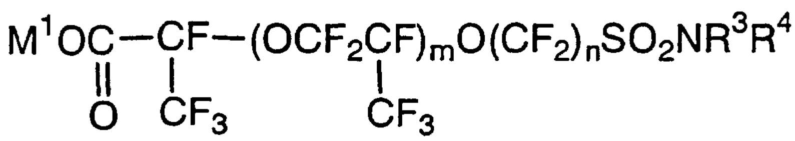

(4)

wherein:

m and n are as defined above for formula (1);
$M^1$ is as defined above for formula (3); and
each of $R^3$ and $R^4$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; a substituted silyl group containing as a substituent at least one $C_1$-$C_{10}$ hydrocarbon group so as to have up to 10 carbon atoms in total; an alkali metal; an alkaline earth metal; an ammonium group; or a phosphonium group, with the proviso that, when each of $R^3$ and $R^4$ is independently the unsubstituted or substituted $C_1$-$C_{10}$ hydrocarbon group or the substituted silyl group, $R^3$ and $R^4$ are optionally bonded together to form a divalent group, thereby forming a saturated or unsaturated nitrogen-containing heterocyclic ring in cooperation with a nitrogen atom which is bonded to $R^3$ and $R^4$, and that $R^3$ and $R^4$ are not simultaneously hydrogen atoms,
optionally followed by treatment with an alkaline compound; and

(iii) subjecting the sulfonamide (4) to decarboxylation-vinylation, optionally followed by treatment with a protic compound.

7. The method according to item 6 above, wherein each m in formulae (1), (2), (3) and (4) is 0.

8. A sulfonamide represented by the following formula (4):

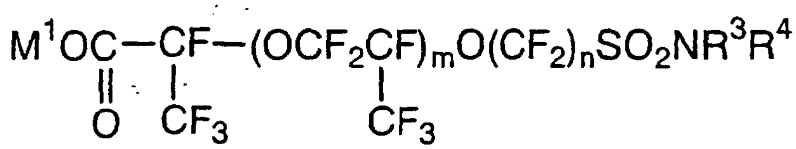

(4)

wherein:

m is an integer of from 0 to 5;
n is an integer of from 1 to 5;
$M^1$ is an alkali metal, an alkaline earth metal, a quaternary ammonium group or a quaternary phosphonium group; and
each of $R^3$ and $R^4$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; a substituted silyl group containing as a substituent at least one $C_1$-$C_{10}$ hydrocarbon group so as to have up to 10 carbon atoms in total; an alkali metal; an alkaline earth metal; an ammonium group; or a phosphonium group, with the proviso that, when each of $R^3$ and $R^4$ is independently the unsubstituted or substituted $C_1$-$C_{10}$ hydrocarbon group or the substituted silyl group, $R^3$ and $R^4$ are optionally bonded together to form a divalent group, thereby forming a saturated or unsaturated nitrogen-containing heterocyclic ring in cooperation with a nitrogen atom which is bonded to $R^3$ and $R^4$, and that $R^3$ and $R^4$ are not simultaneously hydrogen atoms.

9. The sulfonamide according to item 8 above, wherein m in formula (4) is 0.

10. A method for producing the monomer of item 1 above, wherein each of $R^1$ and $R^2$ in formula (1) is a hydrogen atom, or wherein each of $R^1$ and $R^2$ in formula (1) is independently a hydrogen atom; a $C_1$-$C_{10}$ hydrocarbon group which is unsubstituted or substituted with at least one substituent selected from the group consisting of a N,N-disubstituted amino group containing as substituents two hydrocarbon groups, An alkoxy group and an ether group, wherein the substituted $C_1$-$C_{10}$ hydrocarbon group has up to 15 carbon atoms in total; or the substituted silyl group, with the proviso that at least one of $R^1$ and $R^2$ in formula (1) is a $C_3$-$C_{10}$ secondary or tertiary alkyl group or the substituted silyl group,

the method comprising subjecting a sulfonyl fluoride represented by the following formula (5):

$$CF_2{=}CF{-}(OCF_2\underset{\substack{|\\CF_3}}{C}F)_mO(CF_2)_nSO_2F \qquad (5)$$

wherein m and n are as defined above for formula (1),
to amidation, optionally followed by treatment with a protic compound,

wherein the amidation is performed by reacting the sulfonyl fluoride (5) with an amine or metal amide, which is represented by the following formula (6):

$$M^2NR^5R^6 \qquad (6)$$

wherein:

$M^2$ is a hydrogen atom, an alkali metal or an alkaline earth metal; and
each of $R^5$ and $R^6$ independently represents a $C_1$-$C_{10}$ hydrocarbon group which is unsubstituted or substituted with at least one substituent selected from the group consisting of a N,N-di-substituted amino group containing as substituents two hydrocarbon groups, an alkoxy group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the proviso that at least one of $R^5$ and $R^6$ is a $C_3$-$C_{10}$ secondary or tertiary alkyl group or the substituted silyl group,

wherein $R^5$ and $R^6$ are optionally bonded together to form a divalent group, thereby forming a saturated or unsaturated nitrogen-containing heterocyclic ring in cooperation with a nitrogen atom which is bonded to $R^5$ and $R^6$ .

11. A method for producing the monomer of item 1 above, which comprises subjecting a compound represented by the following formula (7):

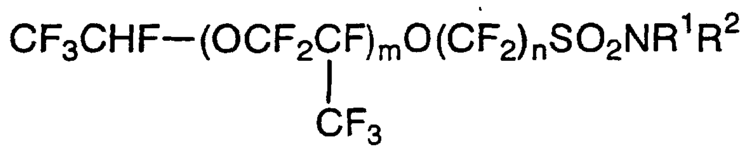

(7)

wherein m, n, $R^1$ and $R^2$ are as defined above for formula (1),
to dehydrofluorination, optionally followed by treatment with a protic compound,

wherein the dehydrofluorination is performed by contacting the compound (7) with a metal amide, which is represented by the following formula (8):

$$M^3NR^xR^y \quad (8)$$

wherein:

$M^3$ is an alkali metal or an alkaline earth metal; and
each of $R^x$ and $R^y$ independently represents a $C_1$-$C_{10}$ hydrocarbon group which is unsubstituted or substituted with at least one substituent selected from the group consisting of a N,N-disubstituted amino group containing as substituents two hydrocarbon groups, an alkoxy group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the proviso that at least one of $R^x$ and $R^y$ is a $C_3$-$C_{10}$ secondary or tertiary alkyl group or the substituted silyl group,

wherein $R^x$ and $R^y$ are optionally bonded together to form a divalent group, thereby forming a saturated or unsaturated nitrogen-containing heterocyclic ring in cooperation with a nitrogen atom which is bonded to $R^x$ and $R^y$.

12. A compound represented by the following formula (7):

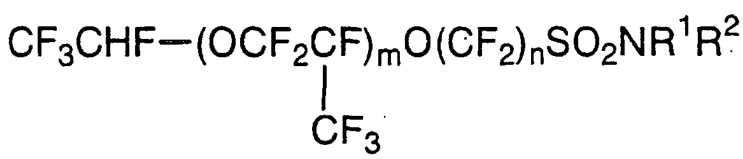

(7)

wherein:

m is an integer of from 0 to 5;
n is an integer of from 1 to 5; and
each of $R^1$ and $R^2$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the proviso that, when each of $R^1$ and $R^2$ is independently the unsubstituted or substituted $C_1$-$C_{10}$ hydrocarbon group or the substituted silyl group, $R^1$ and $R^2$ are optionally bonded together to form a divalent group, thereby forming a saturated or unsaturated nitrogen-containing heterocyclic ring in cooperation with a nitrogen atom which is bonded to $R^1$ and $R^2$.

13. A method for producing the monomer of item 1 above, which comprises subjecting a compound represented by the following formula (9):

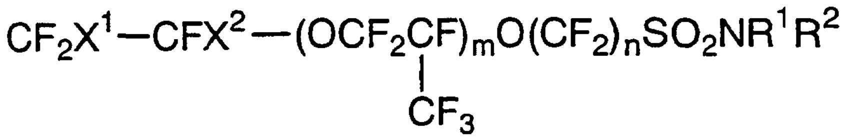 (9)

wherein:

m, n, $R^1$ and $R^2$ are as defined above for formula (1); and
each of $X^1$ and $X^2$ is independently a chlorine atom, a bromine atom or an iodine atom,
to dehalogenation, optionally followed by treatment with a protic compound.

14. A compound represented by the following formula (9):

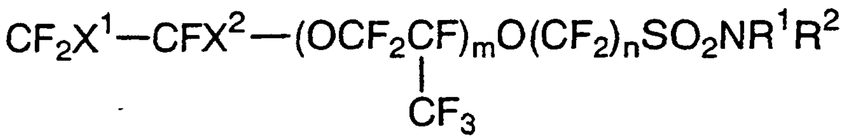 (9)

wherein:

m is an integer of from 0 to 5;
n is an integer of from 1 to 5;
each of $R^1$ and $R^2$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the pro-viso that, when each of $R^1$ and $R^2$ is independently the unsubstituted or substituted $C_1$-$C_{10}$ hydrocarbon group or the substituted silyl group, $R^1$ and $R^2$ are optionally bonded together to form a divalent group, thereby forming a saturated or unsaturated nitrogen-containing heterocyclic ring in cooperation with a nitrogen atom which is bonded to $R^1$ and $R^2$; and
each of $X^1$ and $X^2$ is independently a chlorine atom, a bromine atom or an iodine atom.

15. A method for producing a fluorinated polymer, which comprises subjecting a perfluorovinyl ether monomer represented by the following formula (1):

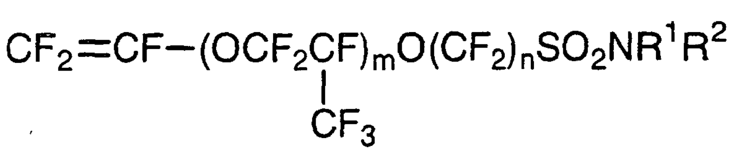 (1)

wherein:

m is an integer of from 0 to 5;
n is an integer of from 1 to 5; and
each of $R^1$ and $R^2$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group

so as to have up to 10 carbon atoms in total, with the proviso that, when each of $R^1$ and $R^2$ is independently the unsubstituted or substituted $C_1$-$C_{10}$ hydrocarbon group or the substituted silyl group, $R^1$ and $R^2$ are optionally bonded together to form a divalent group, thereby forming a saturated or unsaturated nitrogen-containing heterocyclic ring in cooperation with a nitrogen atom which is bonded to $R^1$ and $R^2$,
to homopolymerization or copolymerization with at least one comonomer having an olefinic unsaturated bond.

16. The method according to item 15 above, wherein the monomer (1) is copolymerized with a comonomer comprising tetrafluoroethylene.

17. A fluorinated polymer produced by the method of item 15 or 16 above.

18. A fluorinated polymer comprising monomer units derived from at least one perfluorovinyl ether monomer represented by the following formula (10):

$$CF_2=CFO(CF_2)_pSO_2NR^aR^b \quad (10)$$

wherein:

p is an integer of from 1 to 5; and
each of $R^a$ and $R^b$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the proviso that, when each of $R^a$ and $R^b$ is independently the unsubstituted or substituted $C_1$-$C_{10}$ hydrocarbon group or the substituted silyl group, $R^a$ and $R^b$ are optionally bonded together to form a divalent group, thereby forming a saturated or unsaturated nitrogen-containing heterocyclic ring in cooperation with a nitrogen atom which is bonded to $R^a$ and $R^b$.

19. The fluorinated polymer according to item 18 above, which is a fluorinated copolymer comprising monomer units each derived from the monomer (10) and comonomer units each derived from tetrafluoroethylene.

20. A method for producing a fluorinated copolymer, which comprises subjecting to copolymerization:

(a) at least one monomer having a partially fluorinated or perfluorinated vinyl group and a group represented by the following formula (11):

$$-SO_2NR^7R^8 \quad (11)$$

wherein:

$R^7$ represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total; and
$R^8$ represents a hydrogen atom or the substituted silyl group;

(b) at least one monomer having a partially fluorinated or perfluorinated vinyl group and a group represented by the following formula (12):

$$-SO_2X^3 \quad (12)$$

wherein $X^3$ represents a fluorine atom, a chlorine atom or a $-OR^9$ group, wherein $R^9$ represents the unsubstituted or substituted $C_1$-$C_{10}$ hydrocarbon group or the substituted silyl group; and optionally

(c) at least one monomer other than the monomers (a) and (b), which has an olefinic unsaturated bond.

21. The method according to item 20 above, wherein the monomer (a) is a monomer represented by the following formula (13):

$$CF_2{=}CF\text{-}Rf\text{-}SO_2NR^7R^8 \qquad (13)$$

wherein:

$R^7$ and $R^8$ are as defined above for formula (11); and

Rf is a single bond; a $C_1$-$C_{20}$ fluoroalkylene group represented by the following formula (14):

$$-C_qX^4{}_{2q}- \qquad (14)$$

wherein:

q is an integer of from 1 to 20; and

each $X^4$ independently is a fluorine atom; or a monovalent substituent selected from the group consisting of a hydrogen atom, chlorine atom and an alkoxy group, with the proviso that the number of the monovalent substituent is 35 % or less, based on the number of $X^4$; or

a $C_1$-$C_{20}$ oxyfluoroalkylene group represented by the following formula (15):

$$-OC_qX^4{}_{2q}- \qquad (15)$$

wherein q and $X^4$ are as defined above for formula (14),

wherein at least one single bond between two adjacent carbon atoms of the $C_1$-$C_{20}$ fluoroalkylene group (14) or $C_1$-$C_{20}$ oxyfluoroalkylene group (15) is optionally substituted with at least one divalent substituent selected from the group consisting of an oxygen atom, a carbonyl group, a sulfonyl group, a biscarbonylimide group, a bissulfonylimide group and a carbonylsulfonylimide group, with the proviso that the number of the divalent substituent is 50 % or less, based on the number q.

22. The method according to item 20 above, wherein the monomer (a) is a monomer represented by the following formula (16):

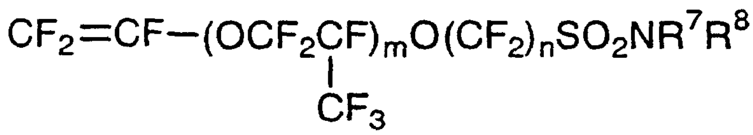

(16)

wherein:

m is an integer of from 0 to 5;

n is an integer of from 1 to 5; and

$R^7$ and $R^8$ are as defined above for formula (11).

23. The method according to any one of items 20 to 22 above, wherein the monomers (a), (b) and (c) are subjected to copolymerization, the monomer (c) comprising tetrafluoroethylene.

24. A fluorinated copolymer obtained by the method of any one of items 20 to 23 above.

25. A fluorinated copolymer comprising the following sulfonyl group-containing monomer units (A) and (B):

(A) monomer units derived from at least one monomer having a partially fluorinated or perfluorinated vinyl group and a group represented by the following formula (11):

$$-SO_2NR^7R^8 \quad (11)$$

wherein:

$R^7$ represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total; and
$R^8$ represents a hydrogen atom or the substituted silyl group, and

(B) monomer units derived from at least one monomer having a partially fluorinated or perfluorinated vinyl group and a group represented by the following formula (12):

$$-SO_2X^3 \quad (12)$$

wherein $X^3$ represents a fluorine atom, a chlorine atom or a $-OR^9$ group, wherein $R^9$ represents the unsubstituted or substituted $C_1$-$C_{10}$ hydrocarbon group or the substituted silyl group.

26. The copolymer according to item 25 above, which comprises the monomer units (A) and (B) and comonomer units each derived from tetrafluoroethylene.

27. The copolymer according to item 25 or 26 above, wherein the amount of the monomer unit (A) is from 0.001 to 50 mol %, based on the total molar amount of the monomer units (A) and (B).

28. The copolymer according to any one of items 25 to 27 above, wherein the weight of the copolymer per mole of sulfonyl groups in the monomer units (A) and (B), which is obtained by dividing the weight (g) of the copolymer by the total molar amount of the monomer units (A) and (B), is from 400 to 1400 g/mol.

29. The copolymer according to any one of items 25 to 28 above, wherein each of the monomer units (A) is derived from a monomer represented by the following formula (13):

$$CF_2{=}CF\text{-}Rf\text{-}SO_2NR^7R^8 \quad (13)$$

wherein:

$R^7$ and $R^8$ are as defined above for formula (11); and
Rf is a single bond; a $C_1$-$C_{20}$ fluoroalkylene group represented by the following formula (14):

$$-C_qX^4{}_{2q}- \quad (14)$$

wherein:

q is an integer of from 1 to 20; and
each $X^4$ independently is a fluorine atom; or a monovalent substituent selected from the group consisting of a hydrogen atom, chlorine atom and an alkoxy group, with the proviso that the number of the monovalent substituent is 35 % or less, based on the number of $X^4$; or
a $C_1$-$C_{20}$ oxyfluoroalkylene group represented by the following formula (15):

$$-OC_qX^4{}_{2q}- \quad (15)$$

wherein q and $X^4$ are as defined above for formula (14),

wherein at least one single bond between two adjacent carbon atoms of the $C_1$-$C_{20}$ fluoroalkylene group (14) or $C_1$-$C_{20}$ oxyfluoroalkylene group (15) is optionally substituted with at least one divalent substituent selected from the group consisting of an oxygen atom, a carbonyl group, a sulfonyl group, a biscarbonylimide group, a bissulfonylimide group and a carbonylsulfonylimide group, with the proviso that the number of the divalent substituent is 50 % or less of the integer q.

30. The copolymer according to any one of items 25 to 28 above, wherein each of the monomer units (A) is derived from at least one monomer represented by the following formula (16):

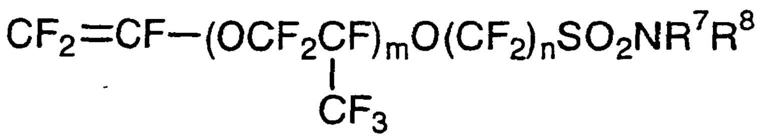 (16)

wherein:

m is an integer of from 0 to 5;
n is an integer of from 1 to 5; and
$R^7$ and $R^8$ are as defined above for formula (11).

31. A copolymer film produced from the copolymer of any one of items 24 to 30 above or a composition comprising the copolymer of any one of items 24 to 30 above.

32. A method for producing the copolymer film of item 31 above, which comprises forming the copolymer of any one of items 24 to 30 above or a composition comprising the copolymer of any one of items 24 to 30 above by melt processing.

33. A copolymer film produced by the method of item 32 above.

34. The copolymer film according to item 31 or 33 above, which is in the form of a single-layer film.

35. A method for producing a modified copolymer film, which comprises subjecting the copolymer film of any one of items 31, 33 and 34 above to treatment with a basic compound.

36. A modified copolymer film produced by the method of item 35 above.

37. A method for producing a solid polymer electrolyte membrane, which comprises subjecting the modified copolymer film of item 36 above to at least one treatment selected from the group consisting of alkali treatment and acid treatment.

38. A solid polymer electrolyte membrane produced by the method of item 37 above.

39. A method for producing a crosslinked copolymer film, which comprises subjecting the copolymer film of any one of items 31, 33 and 34 above to treatment with a basic compound.

40. A crosslinked copolymer film produced by the method of item 39 above.

41. A method for producing a crosslinked solid polymer electrolyte membrane, which comprises subjecting the crosslinked copolymer film of item 40 above to at least one treatment selected from the group consisting of alkali treatment and acid treatment.

42. A crosslinked solid polymer electrolyte membrane produced by the method of item 41 above.

Hereinbelow, the present invention will be described in detail.

The perfluorovinyl ether monomer of the present invention is represented by the following formula (1):

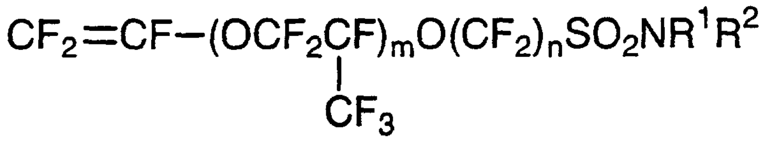

(1)

In formula (1), m is an integer of from 0 to 5. From the viewpoint of increasing the mechanical strength of a polymer which is produced from the above-mentioned perfluorovinyl ether monomer (1) (hereinafter frequently referred to as "monomer (1)"), and the viewpoint of increasing the ion-exchange capacity obtained when such polymer is used as an ion-exchange resin, m is preferably 0 to 2, more preferably 0 or 1, most preferably 0.

In formula (1), n is an integer of from 1 to 5. From the viewpoint of increasing the chemical stability of the monomer (1) itself and a polymer which is produced from the monomer (1), and from the viewpoint of increasing the ion-exchange capacity obtained when such polymer is used as an ion-exchange resin, n is preferably 2 or 3, most preferably 2.

Each of $R^1$ and $R^2$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total.

Each of the unsubstituted hydrocarbon groups $R^1$ and $R^2$ independently has 1 to 10 carbon atoms, preferably 1 to 7 carbon atoms, more preferably 1 to 4 carbon atoms. With respect to the structure of the unsubstituted hydrocarbon group, there is no particular limitation, and the structure can be any of, for example, a linear structure, a branched structure, a cyclic structure, and a combination thereof. Examples of unsubstituted hydrocarbon groups include an alkyl group, an alkenyl group, an aryl group, an aralkyl group and the like. An alkyl group is especially preferred. Specific examples of unsubstituted hydrocarbon groups include a methyl group, an ethyl group, a propyl group, an isopropyl group, a butyl group, an isobutyl group, a sec-butyl group, a t-butyl group, a pentyl group, a hexyl group, a heptyl group, a vinyl group, an allyl group, a butenyl group, a cyclohexenyl group, a phenyl group, a tolyl group, a xylyl group, a benzyl group, a phenetyl group and the like. Among them, a $C_1$-$C_4$ lower alkyl group, such as a methyl group, an ethyl group, a propyl group, an isopropyl group or the like, is especially preferred.

Each of the substituted hydrocarbon groups $R^1$ and $R^2$ independently has a structure in which at least one hydrogen atom of the above-mentioned unsubstituted hydrocarbon group is replaced by at least one substituent selected from the group consisting of a halogen atom, a hydroxyl group, an amino group, an alkoxy group, a carbonyl group, an ester group, an acid amido group, a sulfonyl group and an ether group. When the substituted hydrocarbon group has a substituent containing a carbon atom, such as an alkoxy group or the like, the total number of carbon atoms in the substituted hydrocarbon group is 1 to 15, preferably 1 to 10. Specific examples of substituted hydrocarbon groups include a 2,2,2-trifluoroethyl group, a 3-methoxypropyl group and the like.

Each of the substituted silyl groups $R^1$ and $R^2$ independently contains as a substituent at least one $C_1$-$C_{10}$ hydrocarbon group, preferably 2 or more $C_1$-$C_{10}$ hydrocarbon groups, more preferably 3 $C_1$-$C_{10}$ hydrocarbon groups, so as to have a total number of carbon atoms of 10 or less, preferably 6 or less, more preferably 3. With respect to the structure of the hydrocarbon group in the substituted silyl group, there is no particular limitation, and the structure can be any of, for example, a linear structure, a branched structure, a cyclic structure, and a combination thereof. Examples of hydrocarbon groups include an alkyl group, an alkenyl group, an aryl group, an aralkyl group and the like. An alkyl group is especially preferred. Specific examples of substituted silyl groups include a trimethylsilyl group, a triethylsilyl group, a tripropylsilyl group, a dimethylphenylsilyl group, a dimethylsilyl group and the like. A trimethylsilyl group is especially preferred.

In the monomer (1), when each of $R^1$ and $R^2$ is independently the unsubstituted or substituted $C_1$-$C_{10}$ hydrocarbon group or the substituted silyl group (i.e., when any of $R^1$ and $R^2$ is not a hydrogen atom), $R^1$ and $R^2$ are optionally bonded together to form a divalent group, thereby forming a saturated or unsaturated nitrogen-containing heterocyclic ring in cooperation with a nitrogen atom which is bonded to $R^1$ and $R^2$ . The nitrogen-containing heterocyclic ring may contain a plurality of nitrogen atoms and may contain a hetero atom other than a nitrogen atom, such as an oxygen atom or a sulfur atom. The number of carbon atoms contained in the nitrogen-containing heterocyclic ring is 20 or less, preferably 8 or less, more preferably 4 or less.

When the nitrogen-containing heterocyclic ring is formed, especially when the nitrogen-containing heterocyclic ring is an imidazole ring or a pyrrole ring, the nitrogen-containing heterocyclic ring is susceptive to a substitution reaction (e.g., hydrolysis). Therefore, when the $-SO_2NR^1R^2$ group in the monomer (1) is required to be converted finally to a free sulfonic acid group, it is preferred that the monomer (1) contains the nitrogen-containing heterocyclic ring.

Further, when at least one of $R^1$ and $R^2$ in the monomer (1) is the substituted silyl group, the acidity of the proton in the $-SO_2NH-$ group is lowered and the acid dissociation is suppressed. Therefore, when it is not desired that the monomer (1) is contacted with an acid, it is especially preferred that at least one of $R^1$ and $R^2$ in the monomer (1) is the substituted silyl group. The substituted silyl group in the monomer (1) can be easily converted to a $-SO_2NH-$ group or the below-described bissulfonylimido group even after the monomer (1) has been (co)polymerized to form a polymer.

On the other hand, when at least one of $R^1$ and $R^2$ in the monomer (1) is a hydrogen atom, the hydrogen atom exhibits a weak acidity. Therefore, a polymer which is obtained by a (co)polymerization of such monomer (1) can be used as a weakly acidic resin. Further, such monomer (1) is advantageous in that, as described below, the $-SO_2NR^1R^2$ group can be easily converted to a bissulfonylimido group.

As described below, when both $R^1$ and $R^2$ in the monomer (1) are hydrogen atoms, an advantage can be obtained in that, in the case where the $-SO_2NR^1R^2$ group is converted to a bissulfonylimido group after the polymerization of the monomer (1), there is no necessity for eliminating any of the $R^1$ groups and the $R^2$ groups which remain on the nitrogen atom. Therefore, when the $-SO_2NR^1R^2$ group is converted to a bissulfonylimido group after the polymerization of the monomer (1), it is preferred that both $R^1$ and $R^2$ are hydrogen atoms.

Specific examples of $-SO_2NR^1R^2$ groups (which do not contain the above-mentioned nitrogen-containing heterocyclic ring) in the monomer (1) of the present invention are enumerated below:

$-SO_2NH_2$,
$-SO_2NHCH_3$,
$-SO_2N(CH_3)_2$,
$-SO_2NHC_2H_5$,
$-SO_2NHCH_2CH_2CH_3$,
$-SO_2NHCH(CH_3)_2$,
$-SO_2N(CH_3)C_2H_5$,
$-SO_2N(C_2H_5)_2$,
$-SO_2NHCH_2CH_2CH_2CH_3$,
$-SO_2NHCH_2CH(CH_3)_2$,
$-SO_2NHCH(CH_3)CH_2CH_3$,
$-SO_2NHC(CH_3)_3$,
$-SO_2N(CH_3)CH_2CH_2CH_3$,
$-SO_2N(CH_2CH_2CH_3)_2$,
$-SO_2N[CH(CH_3)_2]_2$,
$-SO_2NHCH_2CH_2CH_2CH_2CH_3$,
$-SO_2NHCH_2C(CH_3)_3$,
$-SO_2NHCH_2CH_2CH_2CH_2CH_2CH_3$,
$-SO_2N(CH_2CH_2CH_2CH_3)_2$,
$-SO_2N[Si(CH_3)_3]_2$,
$-SO_2NHSi(CH_3)_3$,
$-SO_2N(CH_3)Si(CH_3)_3$,

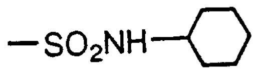,

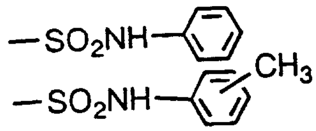,

,

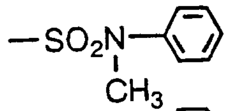,

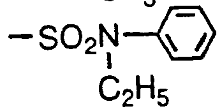, and

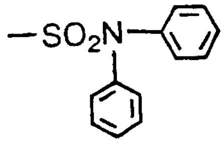.

Further, examples of -$SO_2NR^1R^2$ groups in which $R^1$ and $R^2$ are bonded together to form a divalent group, thereby forming a saturated or unsaturated nitrogen-containing heterocyclic ring in cooperation with a nitrogen atom which is bonded to $R^1$ and $R^2$, are enumerated below:

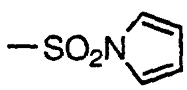,

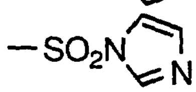,

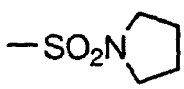,

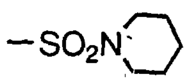,

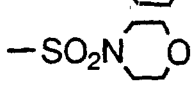,

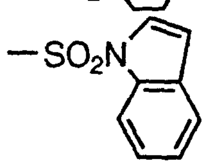,

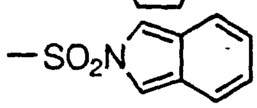, and

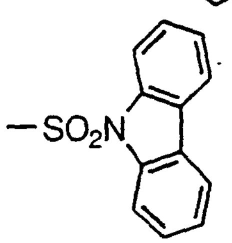.

Hereinbelow, explanations are made with respect to the methods for producing the perfluorovinyl ether monomer (1) of the present invention.

The perfluorovinyl ether monomer (1) of the present invention can be produced by various methods. However, the monomer (1) can be especially efficiently produced by the below-described production methods 1 to 4.

Explanations are made below with respect to the production methods 1 to 4.

·Production method 1

Production method 1 is a method comprising:

(i) converting an acyl fluoride represented by the following formula (2):

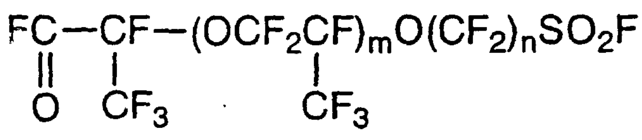

(2)

wherein m and n are as defined above for formula (1), to a carboxylate represented by the following formula (3):

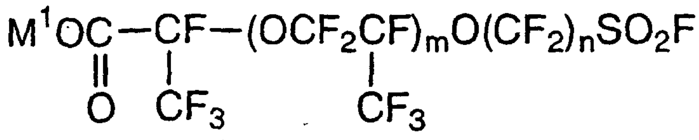

(3)

wherein:

m and n are as defined above for formula (1); and
$M^1$ is an alkali metal, an alkaline earth metal, a quaternary ammonium group or a quaternary phosphonium group;

(ii) effecting an amidation reaction of a fluorosulfonyl group of the carboxylate (3) to thereby obtain a sulfonamide represented by the following formula (4):

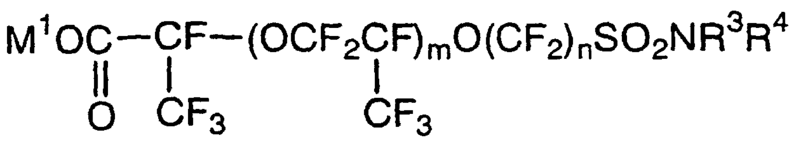

(4)

wherein:

m and n are as defined above for formula (1);
$M^1$ is as defined above for formula (3); and
each of $R^3$ and $R^4$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; a substituted silyl group containing as a substituent at least one $C_1$-$C_{10}$ hydrocarbon group so as to have up to 10 carbon atoms in total; an alkali metal; an alkaline earth metal; an ammonium group; or a phosphonium group, with the proviso that, when each of $R^3$ and $R^4$ is independently the unsubstituted or substituted $C_1$-$C_{10}$ hydrocarbon group or the substituted silyl group, $R^3$ and $R^4$ are optionally bonded together

to form a divalent group, thereby forming a saturated or unsaturated nitrogen-containing heterocyclic ring in cooperation with a nitrogen atom which is bonded to $R^3$ and $R^4$, and $R^3$ and $R^4$ are not simultaneously hydrogen atoms,

optionally followed by treatment with an alkaline compound; and

(iii) subjecting the sulfonamide (4) to decarboxylation-vinylation, optionally followed by treatment with a protic compound.

This method has a very advantageous feature that, even if m in each of the formulae (1), (2), (3) and (4) is 0, the method is substantially free from the above-mentioned unfavorable cyclization reaction which occurs as a side reaction in the conventional methods, so that the monomer (1) can be obtained in a very high yield.

First, explanations are made below with respect to the method for converting the above-mentioned acyl fluoride (2) to the above-mentioned carboxylate (3).

The acyl fluoride (2) can be converted to the carboxylate (3) by a conventional method. Examples of conventional methods include:

a method in which the acyl fluoride (2) is contacted with a basic substance containing $M^1$ (which is as defined above for formula (3)) to perform a neutralization reaction, and

a method in which the acyl fluoride (2) is reacted with an appropriate alcohol (e.g., an alcohol having up to 10 carbon atoms) to obtain an ester, and the obtained ester is saponified with a basic substance containing $M^1$ (which is as defined above for formula (3)).

Of these two methods, the former involving a neutralization reaction is preferred. The "neutralization reaction" mentioned herein means a reaction to convert an acyl halide, such as an acyl fluoride, to a corresponding carboxylate.

Examples of basic substances containing the above-mentioned $M^1$ include a hydroxide, a carbonate, a carboxylate and a phosphate. Examples of quarternary ammonium groups used as the above-mentioned $M^1$ include a tetramethylammonium group, a tetraethylammonium group and a tetrabutylammonium group. Examples of quarternary phosphonium groups used as the above-mentioned $M^1$ include a tetramethylphosphonium group, a tetraethylphosphonium group and a tetrabutylphosphonium group. When a quarternary ammonium group or a quarternary phosphonium group is used as the above-mentioned $M^1$, it is preferred to use a quarternary ammonium hydroxide or a quarternary phosphonium hydroxide as the above-mentioned basic substance.

Among the above-mentioned basic substances, a carbonate of an alkali metal or an alkaline earth metal is preferred, because such a carbonate exhibits high selectivity in the above-mentioned neutralization reaction.

In the above-mentioned neutralization reaction, a solvent can be used to improve a reaction efficiency. When a solvent is used, it is possible to use a protic solvent, but it is more preferred to use an aprotic solvent. Whether the protic solvent or the aprotic solvent is used, it is preferred that the solvent has high polarity.

Examples of protic solvents include water; and alcohols, such as methanol, ethanol and propanol. Examples of aprotic solvents include ethers, such as diethyl ether, tetrahydrofuran, dioxane, ethylene glycol dimethyl ether, and diethylene glycol dimethyl ether; nitriles, such as acetonitrile, propionitrile, butyronitrile, malononitrile, and adiponitrile; and amides, such as dimethylformamide, and dimethylacetoamide.

When the above-mentioned neutralization reaction is performed without using a solvent, the reaction temperature is generally 120 °C or less, preferably 100 °C or less, more preferably 80 °C or less. On the other hand, when the above-mentioned neutralization reaction is performed using a solvent, the reaction temperature is generally 100 °C or less, preferably 80 °C or less, more preferably 60 °C or less. When the reaction temperature is too high, a decarboxlation reaction proceeds to thereby form by-products. For example, when m = 0, cyclization reaction products are co-produced (hereinafter, the side reaction to produce the cyclization reaction products is referred to as "decarboxylation-cyclization reaction"). As a result, the yield of the desired carboxylate (3) is lowered.

With respect to the reaction temperature, there is no particular limitation so long as the above-mentioned neutralization reaction proceeds; however, the reaction is generally performed at 0 °C or higher. There is also no particular limitation with respect to the reaction pressure; however, the reaction is generally performed under atmospheric pressure.

In the above-mentioned neutralization reaction, the above-mentioned basic substance is generally used in an amount equivalent to the amount of the acyl fluoride (2); however, if desired, the basic substance may be used in an excess amount.

Hereinbelow, explanations are made with respect to the methods for effecting an amidation reaction of the fluorosulfonyl group of the carboxylate (3) to thereby obtain the above-mentioned sulfonamide (4).

The amidation reaction of the fluorosulfonyl group of the carboxylate (3) can be effected by the conventional methods. Examples of conventional methods include the following methods:

(1-1) a method in which the carboxylate (3) is reacted with ammonia, a primary amine or a secondary amine (these compounds used as an amidation agent are, hereinafter, collectively referred to simply as "amine"),
(1-2) a method in which the carboxylate (3) is reacted with a metal amide of the above-mentioned amine, and
(1-3) a method in which the carboxylate (3) is reacted with an aminosilane (having one amino group selected from an unsubstituted amino group, an N-monosubstituted amino group or an N,N-disubstituted amino group) in the presence of a fluoride ion-containing compound.

Examples of amines used as the amidation agent in the method (1-1) include amines represented by the following formula (X):

$$HNR^1 R^2 \qquad (X)$$

wherein $R^1$ and $R^2$ are as defined above for formula (1). However, the amine used as the amidation agent in the method (1-1) is not limited to those represented by the formula (X).

In the method (1-1), hydrogen fluoride is co-produced. Therefore, for promoting the desired reaction, an appropriate basic substance may be used as a hydrogen fluoride-scavenger. Examples of basic substances usable as the hydrogen fluoride-scavenger include tertiary amines, such as triethylamine and pyridine; and carbonates of alkali metals. Further, it is also possible to use the above-mentioned amine as the amidation agent in an excess amount such that the surplus amine (which is not reacted with the carboxylate (3)) functions as a hydrogen fluoride-scavenger. The hydrogen fluoride reacts with the hydrogen fluoride-scavenger to form a fluoride ion-containing salt, and this salt is removed from the reaction system. The removal of the salt can be conducted by an appropriate method, such as filtration.

Examples of solvents usable in the above-mentioned amidation reaction include hydrocarbons, hydrocarbon halides, ethers, nitriles and amides. Alternatively, when the above-mentioned amine used as the amidation agent is a liquid, the amine may be used as a solvent.

The reaction temperature varies depending on the type of the amine used; however, the reaction temperature is generally in the range of from -50 to 150 °C, preferably in the range of from 0 °C to 100 °C. With respect to the reaction pressure, there is no particular limitation; however, the reaction is performed generally under atmospheric pressure.

In the method (1-1), when ammonia or a primary amine is used as the above-mentioned amidation agent, it is possible that a sulfonamide (4) having an ammonium group as $R^3$ and/or $R^4$ is obtained. The reaction scheme of the reaction to form the sulfonamide (4) having an ammonium group as $R^3$ and/or $R^4$ is shown below, taking as an example the case where methylamine is used as the amidation agent.

$$-SO_2F \ + \ 3NH_2CH_3$$

$$\downarrow \ -NH_3(CH_3)\cdot F$$

$$-SO_2NHCH_3 \ + \ NH_2CH_3$$

$$\downarrow$$

$$-SO_2NCH_3(NH_3CH_3)$$

In the method (1-2), the same operation is performed as in the above-mentioned method (1-1) except that a metal amide is used as the amidation agent instead of the above-mentioned amine. In this method, the use of the hydrogen fluoride-scavenger (optionally used in the method (1-1)) is not necessary.

Examples of metal amides include a metal amide having a structure in which a hydrogen atom bonded to a nitrogen atom of the above-mentioned amine (X) is replaced by a metal, such as an alkali metal or an alkaline earth metal. However, the metal amide usable in the method (1-2) is not limited to those exemplified above. In the method (1-2), lithium, sodium or potassium is generally used as the above-mentioned metal.

When the metal amide used in the method (1-2) contains a silyl group which is bonded to a nitrogen atom of the metal amide, the above-mentioned silyl group is sometimes eliminated during the amidation reaction. Such elimination of the silyl group is considered to be caused by a fluoride ion which is eliminated from the fluorosulfonyl group

of the carboxylate (3). The reaction scheme of the reaction to eliminate the silyl group is as follows.

$$-SO_2F + NaN(SiMe_3)_2 \longrightarrow -SO_2NNa(SiMe_3) + Me_3SiF$$

In the method (1-3), the same operation is performed as in the above-mentioned method (1-1) except that an aminosilane is used as the amidation agent instead of the above-mentioned amine, and the reaction is performed in the presence of a floride ion-containing compound. Also in the method (1-3), the use of the hydrogen fluoride-scavenger (optionally used in the method (1-1)) is not necessary.

Examples of aminosilanes include a metal amide having a structure in which a hydrogen atom which is bonded to a nitrogen atom of the above-mentioned amine (X) is replaced by an unsubstituted or substituted silyl group. However, the aminosilane used in the method (1-3) is not limited to those exemplified above. Examples of substituted silyl groups include a substituted silyl group containing as a substituent at least one $C_1$-$C_{10}$ hydrocarbon group so as to have up to 10 carbon atoms in total.

Examples of fluoride ion-containing compounds include cesium fluoride and potassium fluoride.

In the method (1-3), it is preferred to use as a solvent any of hydrocarbons, ethers, nitriles and amides.

The conversion of the acyl fluoride (2) to the carboxylate (3) and the amidation of a fluorosulfonyl group of the carboxylate (3) may be performed individually in different reactors or in a single reactor successively. That is, the conversion of the acyl group and the amidation of the fluorosulfonyl group can be performed by either of the following two methods,

a method in which the carboxylate (3) is produced in a first reactor, and the obtained carboxylate (3) is isolated and then, amidated in a second reactor; and

a method in which the carboxylate (3) is produced in a reactor and then, the obtained carboxylate (3) is in situ amidated in the reactor without isolating the carboxylate (3).

Further, when a protic compound (such as water, an alcohol, a primary amine or a secondary amine) is used or co-produced in either of the two reaction steps (i.e., a step for conversion of the acyl group and a step for amidation of the fluorosulfonyl group), it is necessary to dry the resultant reaction product sufficiently so as to remove the protic compound. When the protic compound is not removed sufficiently, a proton-substituted compound represented by the following formula (17) (i.e., a compound formed by replacing a $-COOM^1$ group of the sulfonamide (4) by a hydrogen atom) is co-produced during the below-described decarboxylation-vinylation reaction, thereby lowering the yield of the monomer (1):

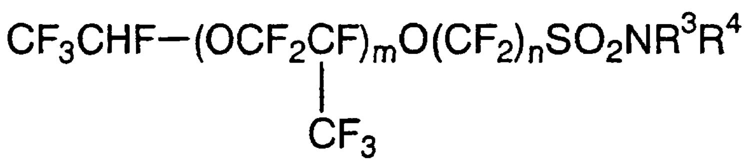

(17)

wherein m, n, $R^3$ and $R^4$ are as defined above for formula (4).

Further, when both $R^3$ and $R^4$ of the sulfonamide (4) are hydrogen atoms, the above-mentioned proton-substituted compound (17) becomes a main product in the decarboxylation-vinylation reaction, whereas almost no monomer (1) of the present invention is obtained (see Comparative Example 6).

Therefore, at least one of $R^3$ and $R^4$ in the sulfonamide (4) should not be a hydrogen atom. It is more preferred that both $R^3$ and $R^4$ are not a hydrogen atom.

So long as one of $R^3$ and $R^4$ is not a hydrogen atom, the other may be a hydrogen atom. In the case where one of $R^3$ and $R^4$ is a hydrogen atom, it is preferred that the other one of $R^3$ and $R^4$ is an aryl group, a secondary or tertiary alkyl group or a substituted silyl group.

In general, the hydrogen atom of a $-SO_2NH-$ group is acidic, so that, when the hydrogen atom is contacted with an alkaline compound, the hydrogen atom may be dissociated. Accordingly, when the sufonamide (4) in which one of $R^3$ and $R^4$ is a hydrogen atom is treated with an alkaline compound, the hydrogen atom as $R^3$ or $R^4$ is dissociated to form a salt with the alkaline compound. In other words, the hydrogen atom as $R^3$ or $R^4$ of the sufonamide (4) is replaced by an alkali metal, an ammonium group or the like. By using such sulfonamide (4) having an alkali metal, an

ammonium group or the like as $R^3$ or $R^4$, the above-mentioned side reaction can be suppressed.

Examples of alkaline compounds include hydroxides, carbonates, carboxylates, phosphates and the like of alkali metals and alkaline earth metals; ammonium hydroxides; and phosphonium hydroxides. By using any of these compounds, the hydrogen atom as $R^3$ or $R^4$ can be replaced by an alkali metal, an alkaline earth metal, an ammonium group or a phosphonium group.

Further, when one of $R^3$ and $R^4$ is a hydrogen atom, the other may be the above-mentioned $M^1$.

The above-mentioned sulfonamide (4) is a novel compound and a key material in the production of the monomer (1) of the present invention. The present invention also provides such valuable sulfonamide (4).

With respect to the method for producing the sulfonamide (4) from the acyl fluoride (2), it is possible to employ methods other than the above-mentioned method in which the carboxylate (3) is formed as an intermediate product. However, as a result of the studies made by the present inventors with respect to various methods, it has been found that the above-mentioned method (in which the carboxylate (3) is formed as an intermediate product) is most excellent with respect to the yield of the sulfonamide (4).

As another method for producing the sulfonamide (4) from the acyl fluoride (2), there can be mentioned a method in which the $-SO_2F$ group of the acyl fluoride (2) is amidated first to obtain a sulfonamide, and the obtained sulfonamide is subjected to neutralization reaction to obtain the sulfonamide (4). By this method, however, substantially no sulfonamide (4) can be obtained. As still another possible method, there can be mentioned a method in which the acyl fluoride (2) is esterified, the $-SO_2F$ group of the resultant ester is amidated to obtain a sulfonamide, and an ester group of the obtained sulfonamide is saponified to obtain the sulfonamide (4). However, this method involves complicated operations, and the yield of the sulfonamide (4) which is achieved by this method is low.

Next, explanations are made with respect to the methods for effecting the decarbonation-vinylation of the sulfonamide (4). The "decarboxylation-vinylation" mentioned herein means that a $-COOM^1$ group and a fluorine atom in the sulfonamide (4) are eliminated to form a perfluorovinyl group ($CF_2$=CF-) shown in formula (1) above.

The decarboxylation-vinylation of the sulfonamide (4) can be performed by heating the sulfonamide (4) in the absence or presence of a solvent.

As the solvent used, an aprotic solvent, especially an aprotic polar-solvent, is preferred. On the other hand, it is not preferred to use protic solvents, such as water and an alcohol, because the protic solvents cause co-production of the above-mentioned proton-substituted compound (17). Examples of preferred solvents include ethers, such as ethylene glycol dimethyl ether, diethylene glycol dimethyl ether, triethylene glycol dimethyl ether and dioxane; nitriles, such as acetonitrile, propionitrile, adiponitrile and malononitrile; amides, such as dimethylformamide, dimethylacetoamide and N-methylpyrolidone.

With respect to the reaction temperature, there is no particular limitation so long as the decarboxylation-vinylation proceeds; however, the reaction temperature is generally in the range of from 50 °C to 350 °C, preferably from 80 °C to 300 °C.

A preferable reaction temperature varies depending on other reaction conditions (especially, the presence or absence of a solvent, and the type of the solvent, if any). When the decarboxylation-vinylation is performed in the absence of a solvent, the reaction temperature is preferably in the range of from 120 °C to 300 °C. When the decarboxylation-vinylation is performed in the presence of a solvent, the reaction temperature is preferably in the range of from 80 °C to 220 °C. However, when a nonpolar solvent is used, the preferred reaction temperature is almost the same as mentioned above in connection with the decarboxylation-vinylation performed in the absence of a solvent.

When both $R^3$ and $R^4$ of the sulfonamide (4) are groups falling within the definition of $R^1$ and $R^2$ in the above-mentioned formula (1), the monomer (1) of the present invention can be obtained as a reaction product.

In the case where at least one of $R^3$ and $R^4$ of the sulfonamide (4) is an alkali metal, an alkaline earth metal, an ammonium group or a phosphonium group (that is, as mentioned above, in the case where a silyl group bonded to a nitrogen atom of the metal amide (amidation agent) is eliminated during the amidation of the fluorosulfonyl group of the carbonate (3), or in the case where a hydrogen atom as $R^3$ or $R^4$ is replaced by an alkali metal, an ammonium group or the like), the resultant reaction product is treated with a protic compound to replace the above-mentioned metal or group as $R^3$ or $R^4$ of the reaction product by a hydrogen atom, to thereby obtain the monomer (1) of the present invention.

Further, by treating the monomer (1) containing a substituted silyl group as $R^1$ or $R^2$ with a protic compound, the substituted silyl group can be replaced by a hydrogen atom.

Examples of protic compounds include water; acids, such as hydrochloric acid, sulfuric acid, phosphoric acid, acetic acid, trifluoromethanesulfonic acid and oxalic acid; alcohols, such as methanol, ethanol, isopropanol, n-butanol and t-butanol; and phenol. Each of these protic compounds is generally used in the form of an aqueous solution.

The treatment with a protic compound can be easily conducted by contacting the reaction product obtained by the above-mentioned decarboxlation-vinylation with a protic compound at room temperature.

Examples of reactions to exchange the substituent(s) ($R^3$ and/or $R^4$ of the sulfonamide (4), or $R^1$ and/or $R^2$ of the monomer (1)) bonded to the nitrogen atom with a hydrogen atom by the above-mentioned treatment with a protic

compound are shown below.

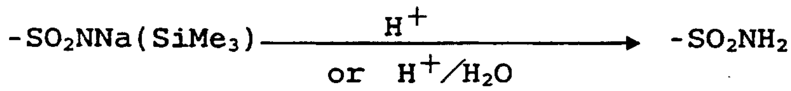


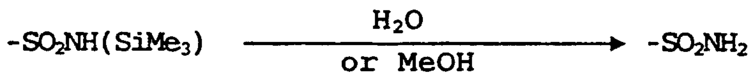


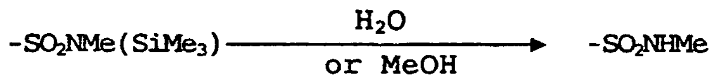


·Production method 2

Production method 2 is a method which can be used only to produce the monomer (1) of the present invention, wherein each of $R^1$ and $R^2$ in formula (1) is a hydrogen atom, or wherein each of $R^1$ and $R^2$ in formula (1) is independently a hydrogen atom; a $C_1$-$C_{10}$ hydrocarbon group which is unsubstituted or substituted with at least one substituent selected from the group consisting of a N,N-disubstituted amino group containing as substituents two hydrocarbon groups, an alkoxy group and an ether group, wherein the substituted $C_1$-$C_{10}$ hydrocarbon group has up to 15 carbon atoms in total; or the substituted silyl group, with the proviso that at least one of $R^1$ and $R^2$ in formula (1) is a $C_3$-$C_{10}$ secondary or tertiary alkyl group or the substituted silyl group. Specifically, the production method 2 comprises subjecting a sulfonyl fluoride represented by the following formula (5):

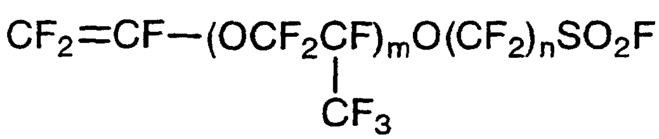
(5)

wherein m and n are as defined above for formula (1), to amidation, optionally followed by the treatment with a protic compound.

In this method, the amidation of the sufonyl fluoride (5) is performed by reacting the sufonyl fluoride (5) with an amine or metal amide which is represented by the following formula (6):

$$M^2NR^5R^6. \qquad (6)$$

wherein:

$M^2$ is a hydrogen atom, an alkali metal or an alkaline earth metal; and
each of $R^5$ and $R^6$ independently represents a $C_1$-$C_{10}$ hydrocarbon group which is unsubstituted or substituted with at least one substituent selected from the group consisting of an N,N-disubstituted amino group containing as substituents two hydrocarbon groups, an alkoxy group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the proviso that at least one of $R^5$ and $R^6$ is a $C_3$-$C_{10}$ secondary or tertiary alkyl group or the substituted silyl group,

wherein $R^5$ and $R^6$ are optionally bonded together to form a divalent group, thereby forming a saturated or unsaturated nitrogen-containing heterocyclic ring in cooperation with a nitrogen atom which is bonded to $R^5$ and $R^6$.

The sulfonyl fluoride (5) is generally used as a raw material for conventional solid polymer electrolyte membranes and can be produced by the conventional methods.

In the amidation of the sulfonylamide (5), when the sulfonyl fluoride (5) is subjected to a reaction with an amide anion, such as $(NH_2)^-$ or $(NEt_2)^-$, the amide anion reacts with a perfluorovinyl group of the sulfonyl fluoride (5), so that substantially no desired monomer (1) can be obtained.

However, as a result of extensive studies made by the present inventors, it has been found that by using a specific amine or metal amide (represented by the above-mentioned formula (6)) having a bulky substituent, the above-mentioned reaction of the amide anion with the perfluorovinyl group is suppressed, and hence, the desired monomer (1) can be obtained efficiently.

In the above-mentioned amine or metal amide (6), at least one of $R^5$ and $R^6$ is a $C_3$-$C_{10}$ secondary or tertiary alkyl group or a substituted silyl group containing as a substituent at least one $C_1$-$C_{10}$ hydrocarbon group so as to have up to 10 carbon atoms in total. When neither $R^5$ nor $R^6$ is such a group as mentioned above, the amine or metal amide (6) reacts with a perfluorovinyl group of the monomer (1) formed by the amidation of the sulfonylamide (5), so that the desired monomer (1) can not be obtained.

Examples of secondary or tertiary alkyl groups include branched or cyclic alkyl groups, each having 3 to 10 carbon atoms, preferably 3 to 6 carbon atoms, such as an isopropyl group, a 2-butyl group, a t-butyl group, 2,4,4-trimethy-2-pentyl group, a cyclopentyl group and a cyclohexyl group.

In the amine or metal amide (6), $R^5$ and $R^6$ are optionally bonded together to form a divalent group, thereby forming a saturated or unsaturated nitrogen-containing heterocyclic ring in cooperation with a nitrogen atom which is bonded to $R^5$ and $R^6$. As an example of such a divalent group, there can be mentioned a 2,6-dimethyl-2,6-pentylene group.

Further, when a substituted silyl group is used as $R^5$ and $R^6$, the same substituted silyl group as mentioned above as $R^1$ and $R^2$ of the monomer (1) can be used. Such a substituted silyl group contains preferably 2 or more hydrocarbon groups, more preferably 3 hydrocarbon groups. Preferred examples of substituted silyl groups include a trimethylsilyl group, a triethylsilyl group and a t-butyldimethylsilyl group.

As $M^2$ of the amine or metal amide (6), it is preferred to use an alkali metal or an alkaline earth metal. It is more preferred to use an alkali metal, and it is most preferred to use lithium, sodium or potassium.

Specific examples of the amine or metal amide (6) include metal amides, such as lithium diisopropylamide, lithium dicyclohexylamide, lithium isopropylcyclohexylamide, 2,2,6,6-tetramethylpiperidine lithium amide, lithium (t-butyl) (2,4,4-trimethyl-2-pentyl) amide, lithium hexamethyldisilazide, sodium hexamethyldisilazide, potassium hexamethyldisilazide, lithium benzyltrimethylsilylamide and amines corresponding to these metal amides (i.e., amines each formed by replacing the metal atom of the metal amide by a hydrogen atom).

The above-mentioned amidation reaction is generally performed in an aprotic polar solvent, such as an ether type solvent, at a relatively low temperature which is below room temperature.

Further, when at least one of $R^5$ and $R^6$ is a substituted silyl group, the substituted silyl group is sometimes eliminated for the same reason as mentioned above in connection with the above-mentioned method (1-2). In such a case, by treating the reaction product obtained by the above-mentioned reaction with a protic compound, the monomer (1) can be obtained. The treatment with a protic compound can be conducted by the method explained above in connection with the production method 1.

Further, when the thus obtained monomer (1) contains as $R^1$ or $R^2$ a $C_1$-$C_{10}$ unsubstituted or substituted hydrocarbon group or a substituted silyl group, the hydrocarbon group or the substituted silyl group may be replaced by a hydrogen atom by subjecting the monomer (1) to the above-mentioned treatment with protic compound.

·Production method 3

Production method 3 is a method which comprises subjecting a compound represented by the following formula (7):

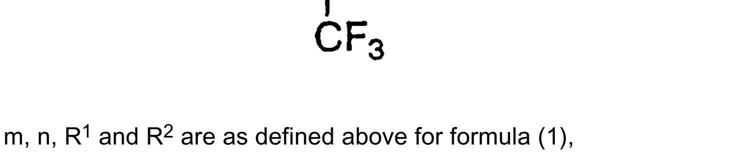

(7)

wherein m, n, $R^1$ and $R^2$ are as defined above for formula (1),

to dehydrofluorination, optionally followed by treatment with a protic compound.

In this method, the dehydrofluorination is performed by contacting the above-mentioned compound (7) with a metal amide represented by the following formula (8):

$$M^3NR^xR^y \quad (8)$$

wherein:

$M^3$ is an alkali metal or an alkaline earth metal; and
each of $R^x$ and $R^y$ independently represents a $C_1$-$C_{10}$ hydrocarbon group which is unsubstituted or substituted with at least one substituent selected from the group consisting of an N,N-disubstituted amino group containing as substituents two hydrocarbon groups, an alkoxy group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the proviso that at least one of $R^x$ and $R^y$ is a $C_3$-$C_{10}$ secondary or tertiary alkyl group or the substituted silyl group,

wherein $R^x$ and $R^y$ are optionally bonded together to form a divalent group, thereby forming a saturated or unsaturated nitrogen-containing heterocyclic ring in cooperation with a nitrogen atom which is bonded to $R^x$ and $R^y$.

As explained above in connection with the production method 1, when the decarboxylation-vinylation of the sulfonamide (4) is performed in the presence of a protic compound, the proton-substituted compound (17) is co-produced. However, the present inventors have found that a specific species of the proton-substituted compound (17), i.e., the above-mentioned compound (7), also can be used as a precursor of the monomer (1). Specifically, the proton-substituted compound (17) in which $R^3$ and $R^4$ are the groups usable as $R^1$ and $R^2$ of the monomer (1) is the compound (7).

The above-mentioned compound (7) is a novel compound and a key material in the production of the monomer (1) of the present invention. The present invention also provides such valuable compound (7).

Hereinbelow, an explanation is made with respect to the dehydrofluorination of the compound (7).

The dehydrofluorination is considered to be caused as follows. By contacting the compound (7) with a basic substance, the hydrogen atom of the $CF_3CFH$ group of the compound (7) is eliminated to thereby cause the dehydrofluorination. However, when the compound (7) is contacted with a basic substance, such as KOH, side reactions vigorously occur, so that substantially no desired monomer (1) can be obtained. The reason for this is because a basic substance reacts with a perfluorovinyl group of the monomer (1) formed by the dehydrofluorination, thereby decomposing the desired monomer (1).

However, as a result of the extensive studies made by the present inventors, it has been found that, when a specific metal amide of-formula (8) above, which has a bulky substituent, is used, the above-mentioned reaction with a perfluorovinyl group is suppressed, and the desired monomer (1) can be efficiently obtained. The metal amide (8) is a specific species of the compound (6) used in the production method 2. Specifically, the compound (6) having an alkali metal or an alkaline earth metal as $M^2$ is the compound (8).

The dehydrofluorination of the compound (7) can be performed in the same manner as in the production method 2 except that the compound (7) is used instead of the sulfonamide (5) and that the metal amide (8) is used instead of the compound (6). However, in the production method 3, the compound (7) having an $SO_2NR^1R^2$ group is used as a starting material, so that, differing from the production method 2, there is no limitation with respect to $R^1$ and $R^2$ of the monomer (1) produced in the production method 3.

Further, as described above, when at least one of $R^1$ and $R^2$ is a hydrogen atom, the hydrogen atom in the $-SO_2NH-$ group is acidic. Therefore, prior to the dehydrofluorination of the compound (7), the compound (7) can be converted into a salt (in which the hydrogen atom as $R^1$ or $R^2$ is replaced by an alkali metal ion, an alkaline earth metal ion, an ammonium ion or the like) by treating the compound (7) with an alkaline substance in the same manner as in the treatment (conducted in the production method 1) of the sulfonamide (4) with an alkaline substance. Even when the compound (7) is not converted into a salt, by using an excess amount of the metal amide (8) which is an alkaline substance, the $-SO_2NH-$ group can be neutralized prior to the dehydrofluorination of the compound (7).

As described above, the proton-substituted compound (17) in which $R^3$ and $R^4$ are the groups usable as $R^1$ and $R^2$ of the monomer (1) is the compound (7). The compound (7) is co-produced in the decarboxylation-vinylation of the compound (i.e., sulfonamide (4) in which $R^3$ and $R^4$ are replaced by $R^1$ and $R^2$ of the monomer (1)) represented by the formula (18) below, when a protic compound is presents in the reaction system.

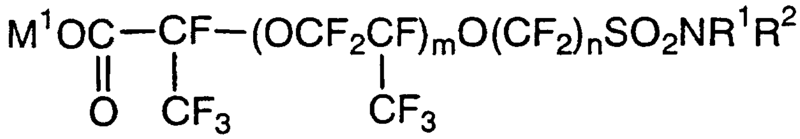

(18)

wherein:

m, n, are as defined above for formula (1); and
$M^1$ is as defined above for formula (3). In fact, the monomer (1) produced by the production method 1 frequently contains a small amount of the compound (7).

With respect to the compound (7), the difference in the boiling point between the compound (7) and the monomer (1) is generally small, and hence, it is difficult to separate the compound (7) from the compound (1) efficiently by distillation. However, by contacting the metal amide (8) with the monomer (1) containing the compound (7), it is possible to cause the metal amide (8) to react only with the compound (7) to thereby convert the compound (7) to the monomer (1). Thus, a highly purified monomer (1) can be obtained.
As is apparent from the above, the dehydrofluorination (vinylation) method using the compound (8) is also useful as a method for improving the purity of the perfluorovinyl ether (1), and this method can be applied also to the production of conventional perfluorovinyl ethers other than the monomer (1) so long as the starting material does not have any functional group which reacts with the metal amide (8).
The reaction of the compound (7) with the metal amide (8) is generally performed in an aprotic polar solvent, such as an ether, at a relative low temperature which is below room temperature. The metal amide (8) may be used in an amount equivalent to the amount of the compound (7); however, when the compound (7) contains a proton (other than a hydrogen atom in a $CF_3CFH$ group) which is easily eliminated, the metal amide (8) may be used in an excess amount.
As described above, the compound (7) can be easily obtained by the decarboxylation of the compound (18), which is conducted in the presence of a protic compound, such as water or an alcohol. For producing the compound (7) efficiently, the protic compound is preferably used in an amount equivalent to the amount of the compound (18), more preferably in an excess amount.
As another method for producing the compound (7), there can be mentioned a method which comprises:

subjecting the above-mentioned carboxylate (3) to decarboxylation in the presence of the above-mentioned protic compound, to thereby obtain a compound represented by the following formula (19):

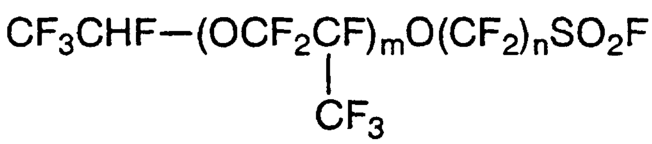

(19)

wherein:

n is as defined above for formula (1); and
amidating a fluorosulfonyl group of the obtained compound (19) by the same method as in the amidation of a fluorosulfonyl group of the carboxylate (3) in the above-mentioned production method (1).

With respect to the amidation of the compound (19), as in the case of the above-mentioned amidation methods (1-1) and (1-2), the compound (19) can be amidated by reacting the compound (19) with an amine or a metal amide. It is preferred that the above-mentioned amine or metal amide is used in an amount equivalent to the amount of the compound (19). When the amine or metal amide is used in an excess amount, the produced compound (7) further reacts with the amine or metal amide to coproduce a by-product, thereby lowering the yield of the desired compound (7).
In this method, when the above-mentioned metal amide (8) is used for the amidation of the compound (19), the above-mentioned dehydrofluorination and amidation can be performed together in a single step. More specifically,

the monomer (1) can be obtained in a single reaction step from the compound (19) without isolating the compound (7) as an intermediate. For effecting such a single reaction step, the metal amide is used in an excess amount (two equivalents or more), relative to the compound (19). Such method is simple and an excellent production method for the monomer (1). However, in this method, the same amidation reaction as in the above-mentioned production method 2 is considered to occur in the reaction system, and therefore, the monomer (1) which can be produced by this method is the same as that obtained by the production method 2.

The production method 3 is especially advantageous in the case where the co-production of the compound (7) is likely to occur during the decarboxylation-vinylation of the sulfonamide (4) due to the presence of easily dissociable protons in the sulfonamide (4), thereby rendering difficult the production of the monomer (1).

Further, when at least one of $R^x$ and $R^y$ of the compound (8) (used for the dehydrofluorination of the compound (7)) is a substituted silyl group, the substituted silyl group is sometimes eliminated for the same reason as mentioned above in connection with the above-mentioned method (1-2). In such a case, by treating the reaction product (obtained by the dehydrofluorination) with a protic compound, the monomer (1) can be obtained. The treatment with a protic compound can be conducted by the method explained above in connection with the production method 1.

Further, when the thus obtained monomer (1) contains a substituted silyl group as $R^1$ or $R^2$, by subjecting the monomer (1) to the above-mentioned treatment with a protic compound, the substituted silyl group can be replaced by a hydrogen atom.

·Production method 4

Production method 4 is a method which comprises subjecting a compound represented by the following formula (9):

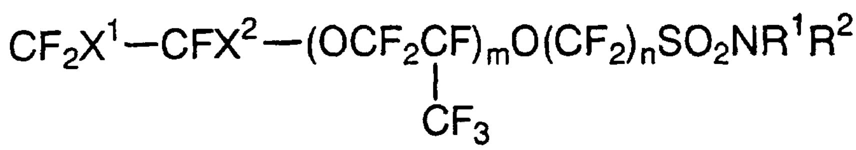

(9)

wherein m, n, $R^1$ and $R^2$ are as defined above for formula (1),

each of $X^1$ and $X^2$ is independently a chlorine atom, a bromine atom or an iodine atom.

to dehalogenation, optionally followed by a treatment with a protic compound.

The compound (9) which is used as a starting material in the production method 4 has a structure in which a halogen atom other than a fluorine atom is added to the perfluorovinyl group of the monomer (1). Specifically, each of $X^1$ and $X^2$ in the formula (9) is independently a chlorine atom (Cl), a bromine atom (Br) or an iodine atom (I).

With respect to the above-mentioned halogen atoms used as $X^1$ and $X^2$, the order of preferredness is I > Br > Cl, from the viewpoint of ease in dehalogenation. On the other hand, from the viewpoint of availability of a halogen compound used for providing the halogen atom, the order of preference is Cl > Br > I.

With respect to the method for producing the compound (9), there is no particular limitation. Examples of methods for producing the compound (9) include the following methods 1) and 2).

1) A method comprising:

adding $Cl_2$, $Br_2$ or $I_2$ to a perfluorovinyl group of a monomer having an $SO_2F$ group and a perfluorovinyl group, which monomer is produced by a conventional method, to thereby obtain an intermediate compound having an $SO_2F$ group; and

reacting the obtained intermediate compound with ammonia or a primary or secondary amine to amidate the intermediate compound.

The scheme of the reactions involved in this method is shown below.

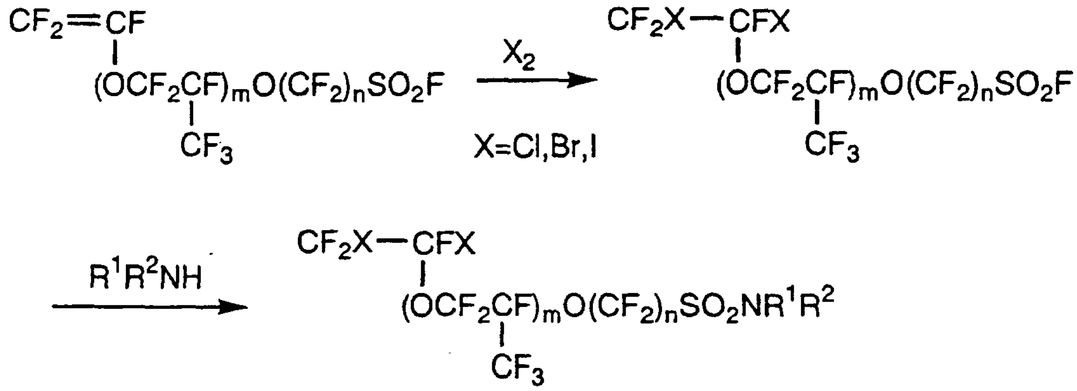


2) A method which comprises:

reacting a hypofluorite having an $SO_2F$ group with a 1,2-dihalo-1,2-difluoroethylene (see, J. Fluorine Chem., 95, 27 (1999), the Netherlands) or reacting a hypochlorite having an $SO_2F$ group with a monohalotrifluoroethylene (e.g., chlorotrifluoroethylene) (see, J. Fluorine Chem., 58, 59 (1992), the Netherlands), to thereby obtain an intermediate compound having an $SO_2F$ group; and

reacting the obtained intermediate compound with ammonia or a primary or secondary amine to amidate the intermediate compound.

The scheme of the reactions involved in this method is shown below.

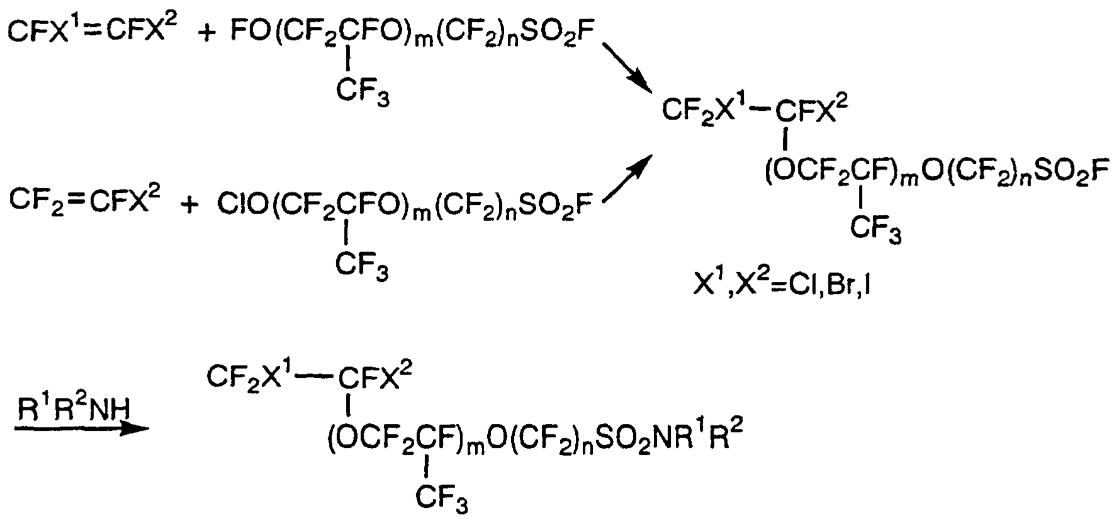


The amidation of the above-mentioned intermediate compound having an $SO_2F$ group can be performed by the same method as in the amidation of a fluorosulfonyl group of the carboxylate (3) which is explained above in connection with the above-mentioned production method 1.

The above-mentioned compound (9) is a novel compound and a key material in the production of the monomer (1) of the present invention. The present invention also provides such a valuable compound (9).

Hereinbelow, an explanation is made with respect to the dehalogenation of the compound (9).

The dehalogenation of the compound (9) is performed by contacting the compound (9) with a dehalogenating agent. Examples of dehalogenating agents generally used include metals, such as Zn, Mg, Cu, Fe and Sn, and metal

alloys each comprising at least two metals, such as Zn-Cu, and Zn-Pb. Among these, especially preferred are Zn and alloys containing Zn.

With respect to these metals or alloys, it is preferred to use one which has a large surface area. For this reason, generally, each of the above metals and metal alloys is used in a powdery, granular or particulate form. Further, it is preferred that the above metals and alloys are washed with diluted hydrochloric acid or the like, followed by drying, prior to the use thereof.

For promoting the dehalogenation reaction, a catalyst, such as bromine, may be used in combination with the dehalogenating agent.

The dehalogenation reaction is performed in a heterogeneous system (i.e., a gas-solid system or a solid-liquid system) containing a solid dehalogenating agent. Generally, the reaction is performed in a solid-liquid system using a solvent. As a solvent, a polar solvent is generally used. Examples of polar solvents include ethers, such as glyme, diglyme, triglyme and dioxane; amides, such as dimethyformamide, dimethylacetoamide and N-methylpyrrolidone; nitriles, such as acetonitrile and propionitrile; and dimethylsulfoxide. The reaction temperature is generally in the range of from room temperature to the boiling point of the solvent used.

Alternatively, the dehalogenation of the compound (9) can be performed by electrochemical method. In this method, the above-mentioned dehalogenating agent (metals and the like) is not necessary, and hence, a by-product derived from a dehalogenating agent is not formed. Therefore, the use of the electrochemical method is advantageous in that the amount of the waste accompanying the producuction of the monomer (1) can be lowered.

The electrochemical method is generally conducted as follows. The compound (9) is dissolved in an appropriate electrolytic liquid, and then, an anode and a cathode are put into the resultant solution. Then, a voltage is applied between the two electrodes to perform the dehalogenation of the compound (9) by an electrochemical reaction.

With respect to the conditions for performing the electrochemical reaction, there is no particular limitation and the reaction can be performed under conditions which are generally employed in the conventional electrolysis.

Examples of materials for the anode include carbon, platinum, ruthenium, rhodium, palladium, iridium and gold. Further, an electrode plated with any of the above metals can also be used.

Examples of materials for the cathode include nickel, copper, zinc, iron, titanium, chromium, aluminum, cobalt, tin, cadmium, antimony, mercury, lead and silver. Further, an electrode plated with any of the above metals can also be used.

If desired, a membrane, such as an ion exchange membrane, a porous resin membrane and a porous ceramic membrane, may be disposed between the two electrodes.

The electrolytic liquid used in the electrochemical method comprises a solvent generally used in the art and an electrolyte dissolved in the solvent.

The above-mentioned solvent used in this method needs to be capable of dissolving therein not only the above-mentioned electrolyte but also the compound (9). Examples of solvents include water; nitriles, such as acetonitrile and propionitrile; amides, such as dimethylformamide, N-methyl-2-pyrrolidone and hexamethylphosphorictriamide; alcohols, such as butanol and (poly)ethyleneglycol; ethers, such as tetrahydrofuran and dioxane; ketones, such as acetone and methyl ethyl ketone; polar organic solvents, such as dimethysulfoxide. These solvents can be used individually or in combination. If desired, for improving the solubility of the compound (9), a fluorine-containing solvent, such as HFC43-10mee, can be used in combination with any of the above-exemplified solvents.

Examples of electrolytes include inorganic acids, such as hydrochloric acid, sulfuric acid and tetrafluoroboric acid; organic acids, such as (fluoro)aliphatic saturated carboxylic acid, (fluoro)alkyl sulfonic acid; inorganic bases, such as sodium hydroxide; organic bases, such as trialkylamine and tetraalkylammonium-hydroxide; and salts thereof. When an organic solvent is used, it is preferred to use an electrolyte having a high solubility in the organic solvent, such as a quarternary ammonium salt or quarternary phosphonium salt of the above-mentioned organic acid or inorganic acid.

Further, as an electrolytic liquid, an ionic solution (which functions as the electrolyte as well as the solvent) can also be used. Examples of ionic solutions include 1-butyl-3-methyl-1H-imidazoliumhexafluorophosphate, and 1-ethyl-3-methyl-1H-imidazoliumtrifluoromethane sulfonate.

The voltage which is applied between the two electrodes is generally in the range of from 2.7 to. 40 V, and the current density is generally in the range of from 10 to 500 $mA/cm^2$. Further, the electrochemical reaction is generally performed under atmospheric pressure at a temperature within the range of from -20 °C to the boiling point of the solvent used.

It is preferred that the isolation of the desired monomer (1) from the reaction mixture obtained by the dehalogenation reaction is performed by subjecting the reaction mixture per se to distillation; however, if desired, the distillation can be conducted after removal of a solid from the reaction mixture by filtration or after extraction using an appropriate solvent.

Further, in the case where the monomer (1) is a liquid, and the reaction mixture separates into a phase comprised mainly of the monomer (1) and another phase when allowed to stand, the monomer (1) can be obtained by recovering the monomer (1)-containing phase from the reaction mixture, followed by purification by distillation or the like.

When the thus obtained monomer (1) has a substituted silyl group as $R^1$ or $R^2$, the substituted silyl group can be replaced by a hydrogen atom by subjecting the monomer (1) to the treatment with a protic compound, which is explained above in connection with the production method 1.

Explanations have been made on the preferred methods 1 to 4 for producing the perfluorovinyl ether monomer (1) of the present invention. However, the production method is not limited to these methods 1 to 4.

As a specific example of a production method other than the above-mentioned methods 1 to 4, there can be mentioned a method which comprises the steps of:

producing the monomer (1) by using any one of the above-mentioned methods 1 to 4, and
subjecting the obtained monomer (1) to an appropriate treatment in which $R^1$ and/or $R^2$ in the monomer (1) is modified or substituted, to thereby obtain a monomer (1) product having a structure different from the original monomer (1) with respect to the structure of $R^1$ and/or $R^2$.

Examples of modifications or substitutions of $R^1$ and/or $R^2$ include:

- replacement of the hydrogen atom as $R^1$ and/or $R^2$ by an alkyl group (i.e., N-alkylation),
- replacement of the hydrogen atom as $R^1$ and/or $R^2$ by a substituted silyl group (i.e., N-silylation),
- replacement of the alkyl group as $R^1$ and/or $R^2$ by a hydrogen atom (i.e., N-dealkylation), and
- replacement of the substituted silyl group as $R^1$ and/or $R^2$ by a hydrogen atom (i.e., N-desilylation).

As a specific example, there can be mentioned the N-silylation (using hexamethyldisilazane) represented by the following formula:

$$-SO_2NH_2 + (Me_3Si)_2NH \rightarrow -SO_2NHSiMe_3.$$

The sulfonamido group contained in the monomer (1) of the present invention exhibits high reactivity. By virtue of such property of the monomer (1), the monomer (1) can be converted into various derivatives. By the copolymerization of the thus obtained derivatives with the monomer (1), a copolymer having the desired properties can be obtained.

Hereinbelow, examples of reactions for the production of a derivative of the monomer (1) are illustrated. (In each of the following examples, the monomer (1) is expressed as the $-SO_2NR^1R^2$ group.)

$$-SO_2NH_2 + CF_3SO_2F \xrightarrow{\text{Base}} -SO_2NHSO_2CF_3,$$

$$-SO_2NH_2 + FSO_2R^{f}{}_1SO_2F \xrightarrow{\text{Base}} -SO_2NHSO_2R^{f}{}_1SO_2F$$

wherein $R^f{}_1$ represents a $C_1$-$C_{10}$ perfluoroalkylene group optionally containing an ether linkage,

$$-SO_2NH(tBu) + CF_3SO_2F \xrightarrow{\text{Base}} -SO_2N(tBu)SO_2CF_3,$$

$$-SO_2NH_2 + F(CO)CF_2SO_2F \xrightarrow{\text{Base}} -SO_2NHCOCF_2SO_2F$$

and

$$-SO_2NH_2 + CF_3COF \xrightarrow{\text{Base}} -SO_2NHCOCF_3.$$

Further, the reactions represented by the below-mentioned formulae (32) to (35) (i.e., the reactions for converting the structure of the side chain of polymer) can also be advantageously used for producing a derivative of the monomer (1).

By subjecting the thus obtained perfluorovinyl ether monomer (1) to homopolymerization or copolymerization with at least one comonomer having an olefinic unsaturated bond, a fluorinated polymer can be easily obtained. For the production of a fluorinated polymer having excellent mechanical strength, it is preferred to perform the copolymerization of the monomer (1) with at least one comonomer having an olefinic unsaturated bond.

In the copolymerization reaction between the monomer (1) of the present invention and a comonomer, with respect to the type of comonomer, there is no particular limitation, and the type of comonomer can be appropriately chosen in accordance with the properties of the desired copolymer.

Examples of comonomers include olefins, such as ethylene, propylene and alkylvinyl ether; fluorinated olefins, such as tetrafluoroethylene, trifluoroethylene, vinylidene fluoride, hexafluoropropylene, perfluoromethyl vinyl ether, perfluoropropyl vinyl ether; and chlorofluorolefins, such as chlorotrifluoroethylene. These comonomers can be used individually or in combination.

Among these comonomers, from the viewpoint of chemical stability, preferred are the comonomers containing at least one fluorine atom, especially per-fluorolefins and chlorofluorolefins. More preferred are tetrafluoroethylene and chlorotrifluoroethylene, and most preferred is tetrafluoroethylene.

In the copolymerization reaction between the monomer (1) of the present invention and a comonomer, with respect to the amount of monomer (1) and the amount of the comonomer, there is no particular limitation, and the amounts of monomer (1) and comonomer can be appropriately chosen in accordance with the properties of the desired fluorinated copolymer. In the fluorinated copolymer, the amount of the monomer unit derived from the monomer (1) is generally in the range of from 0.001 to 50 mol %, preferably from 0.005 to 30 mol %, more preferably from 0.01 to 20 mol %, based on the total molar amount of the monomer unit derived from the monomer (1) and the monomer unit derived from the comonomer.

With respect to the method for performing the homopolymerization reaction or copolymerization reaction of the monomer (1) of the present invention, there is no particular limitation, and any conventional method can be employed, such as radical polymerization or radiation polymerization. Examples of methods of polymerization include a solution polymerization as described in Unexamined Japanese Patent Application Laid-Open Specification No. Sho 57-92026, a suspension polymerization or an emulsion polymerization each of which uses water or the like as a liquid medium, a bulk polymerization, a miniemulsion polymerization and a microemulsion polymerization. In the case of a radical emulsion, the conventional radical initiators which are generally used, and also other polymerization initiators, such as per-fluoroperoxides, may be used as the polymerization initiator.

In the case where $R^1$ and/or $R^2$ in the monomer (1) of the present invention is a hydrogen atom (namely, when the monomer (1) has a structure represented by the formula $-SO_2NH-$), the hydrogen atom as $R^1$ or $R^2$ in the monomer (1) exhibits a weak acidity. When performing a (co)polymerization of such monomer (1), especially in the case of an emulsion polymerization or the like which is effected in an aqueous solvent, the (co)polymerization may be performed by a method in which a basic compound is added to the reaction system to thereby cause the hydrogen atom as $R^1$ or $R^2$ to be replaced by an alkali metal ion, an alkaline earth metal ion, an ammonium ion or the like.

As mentioned above, when the thus obtained fluorinated polymer is used as an ion-exchange resin, from the viewpoint of increasing the ion-exchange capacity thereof, the most preferred value of m in formula (1) is 0. Therefore, it is especially preferred that the fluorinated polymer comprises monomer units derived from at least one perfluorovinyl ether monomer represented by the following formula (10):

$$CF_2{=}CFO(CF_2)_pSO_2NR^aR^b \qquad (10)$$

wherein:

p is an integer of from 1 to 5; and
each of $R^a$ and $R^b$ independently represents a hydrogen atom; a $C_1$-$C_{10}$ hydrocarbon group which is unsubstituted

or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the pro-viso that, when each of $R^a$ and $R^b$ is independently the unsubstituted or substituted $C_1$-$C_{10}$ hydrocarbon group or the substituted silyl group, $R^a$ and $R^b$ are optionally bonded together to form a divalent group, thereby forming a saturated or unsaturated nitrogen-containing heterocyclic ring in cooperation with a nitrogen atom which is bonded to $R^a$ and $R^b$.

This fluorinated polymer, comprising monomer units derived from at least one perfluorovinyl ether monomer represented by the monomer (10), is a novel polymer which has for the first time been produced by the present inventors.

In the course of studying the above-mentioned fluorinated polymer, the present inventors have unexpectedly found that, with respect to a fluorinated copolymer produced by a method which comprises subjecting to copolymerization:

(a) at least one monomer having a partially fluorinated or perfluorinated vinyl group and a group represented by the following formula (11):

$$-SO_2NR^7R^8 \qquad (11)$$

wherein:

$R^7$ represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total; and

$R^8$ represents a hydrogen atom or the substituted silyl group;

(b) at least one monomer having a partially fluorinated or perfluorinated vinyl group and a group represented by the following formula (12):

$$-SO_2X^3 \qquad (12)$$

wherein $X^3$ represents a fluorine atom, a chlorine atom or a $-OR^9$ group, wherein $R^9$ represents the unsubstituted or substituted $C_1$-$C_{10}$ hydrocarbon group or the substituted silyl group; and optionally

(c) at least one monomer other than the monomers (a) and (b), which has an olefinic unsaturated bond.

The fluorinated copolymer has advantageous properties such that the heat resistance of the fluorinated copolymer can be easily improved by subjecting the fluorinated copolymer to an appropriate modification treatment with a basic compound as mentioned below. Such properties are extremely advantageous in the production of a material which is required to have excellent heat resistance, such as a material for a solid polymer electrolyte for use in a fuel cell.

Conventionally, there has not been known such a fluorinated copolymer. Such fluorinated copolymer has for the first time been produced by the present inventors. Further, as described below, a certain type of the perfluorovinyl ether monomer (1) of the present invention can be used as the above-mentioned monomer (a).

Hereinbelow, explanations are made of the monomers (a), (b) and (c).

Monomer (a)

As mentioned above, the monomer (a) is at least one monomer having a partially fluorinated or perfluorinated vinyl group and a group represented by the following formula (11):

$$-SO_2NR^7R^8 \qquad (11).$$

In the group represented by formula (11) (hereinafter referred to as "substituent (11)"), $R^7$ represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein the substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total.

The unsubstituted hydrocarbon group $R^7$ is an unsubstituted $C_1$-$C_{10}$ hydrocarbon group, preferably an unsubstituted $C_1$-$C_7$ hydrocarbon group, more preferably an unsubstituted $C_1$-$C_4$ hydrocarbon group. With respect to the structure of the above-mentioned unsubstituted hydrocarbon group, there is no particular limitation, and the structure can be any of, for example, a linear structure, a branched structure, a cyclic structure, and a combination thereof. Specific examples of unsubstituted hydrocarbon groups include an alkyl group, an alkenyl group, an aryl group and an aralkyl group. Among these hydrocarbon groups, from the viewpoint of chemical stability, preferred are an aromatic hydrocarbon group, a saturated hydrocarbon group and a hydrocarbon group having those in combination. More preferred are lower alkyl groups, such as a methyl group, an ethyl group, a propyl group, an isopropyl group, a butyl group, an isobutyl group, a sec-butyl group and a t-butyl group.

The substituted hydrocarbon group $R^7$ has a structure in which at least one hydrogen atom of the unsubstituted hydrocarbon group is replaced by at least one substituent selected from the group consisting of a halogen atom, a hydroxyl group, an amino group, an alkoxy group, a carbonyl group, an ester group, an acid amido group, a sulfonyl group and an ether group. When the substituted hydrocarbon group has a substituent containing a carbon atom, such as an alkoxy group, the total number of carbon atoms contained in the substituted hydrocarbon group is in the range of from 1 to 15, preferably from 1 to 10, inclusive of the carbon atoms contained in the substituent. Specific examples of substituted hydrocarbon groups include a 2,2,2-trifluoroethyl group and a 3-methoxypropyl group.

The substituted silyl group $R^7$ contains as a substituent at least one $C_1$-$C_{10}$ hydrocarbon group, preferably at least two $C_1$-$C_{10}$ hydrocarbon groups, more preferably three $C_1$-$C_{10}$ hydrocarbon groups, so as to have up to 10 carbon atoms in total, preferably up to 6 carbon atoms in total, more preferably three carbon atoms in total. With respect to the structure of the hydrocarbon group in the substituted silyl group, there is no particular limitation, and the structure can be any of, for example, a linear structure, a branched structure, a cyclic structure, and a combination thereof. Specific examples of hydrocarbon groups include an alkyl group, an alkenyl group, an aryl group and an aralkyl group. Especially preferred is an alkyl group. Specific examples of substituted silyl groups include a trimethyl silyl group, a triethyl silyl group, tripropyl silyl group, a dimethylphenyl silyl group and a dimethyl silyl group. Especially preferred is a trimethyl silyl group.

In the substituent (11), $R^8$ represents a hydrogen atom or the substituted silyl group.

As mentioned below, depending on the type of $R^7$ and $R^8$ in the monomer (a), when the fluorinated copolymer of the present invention is intended for use as a solid polymer electrolyte membrane, $R^7$ and/or $R^8$ needs to be replaced by a hydrogen atom. Therefore, for eliminating the necessity for replacing $R^7$ and/or $R^8$ by a hydrogen atom, it is preferred that each of $R^7$ and $R^8$ in the monomer (a) is a hydrogen atom.

As the above-mentioned partially fluorinated or perfluorinated vinyl group (hereinafter referred to as "fluorinated vinyl group"), there can be used a vinyl group in which the three hydrogen atoms thereof are partially or completely replaced by a fluorine atom, a vinyl group in which one hydrogen atom thereof is replaced by a fluorine atom, and at least one of the other two hydrogen atoms is replaced by a chlorine atom, or a vinyl group in which one hydrogen atom is replaced by a chlorine atom, and at least one of the other two hydrogen atoms is replaced by a fluorine atom. It is preferred that the fluorinated vinyl group contains at least two fluorine atoms.

The monomer (a) has a structure in which the fluorinated vinyl group and the substituent (11) are bonded together through a divalent group. With respect to the structure of the divalent group, there is no particular limitation; however, from the viewpoint of obtaining a fluorinated copolymer having excellent chemical stability and excellent thermal stability, it is preferred to use a perfluorinated divalent hydrocarbon group as the divalent group. With respect to the structure of such perfluorinated divalent hydrocarbon group, there is no particular limitation, and the structure can be any of, for example, a linear structure, a branched structure, a cyclic structure, and a combination thereof. The perfluorinated divalent hydrocarbon group may also contain an unsaturated bond or an aromatic ring.

The single bond between two adjacent carbon atoms of the perfluorinated divalent hydrocarbon group may be replaced by a divalent substituent, such as an oxygen atom, a carbonyl group, a sulfonyl group, a biscarbonylimido group, a bissulfonylimido group or a carbonylsulfonylimido group.

The fluorinated vinyl group and the substituent (11) may be bonded to the perfluorinated divalent hydrocarbon group through the divalent substituent mentioned above.

Further, the fluorine atoms of the perfluorinated divalent hydrocarbon group may be partially replaced by a monovalent substituent, such as a hydrogen atom or a chlorine atom, as long as no adverse effect is caused on the properties of the obtained copolymer.

As a specific example of such monomer (a), there can be mentioned a monomer represented by the following

formula (13):

$$CF_2=CF\text{-}Rf\text{-}SO_2NR^7R^8 \qquad (13)$$

wherein:

$R^7$ and $R^8$ are as defined above for formula (11); and
Rf is a single bond; a $C_1$-$C_{20}$ fluoroalkylene group represented by the below-mentioned formula (14); or a $C_1$-$C_{20}$ oxyfluoroalkylene group represented by the below-mentioned formula (15):

$$-C_qX^4{}_{2q}- \qquad (14)$$

$$-OC_qX^4{}_{2q}- \qquad (15)$$

wherein:

q is an integer of from 1 to 20; and
each $X^4$ is independently a fluorine atom; or a monovalent substituent selected from the group consisting of a hydrogen atom, a chlorine atom and an alkoxy group, and the number of the monovalent substituent is 35 % or less, preferably 25 % or less, more preferably 15 % or less, based on the number of $X^4$.

With respect to the structures of the fluoroalkylene group (14) and the oxyfluoroalkylene group (15), there is no particular limitation, and each of the fluoroalkylene group (14) and the oxyfluoroalkylene group (15) can have any structure, such as a linear structure, a branched structure or a cyclic structure, or a combination thereof.

Further, at least one of the single bonds, each of which is present between two adjacent carbon atoms of the $C_1$-$C_{20}$ fluoroalkylene group (14) or $C_1$-$C_{20}$ oxyfluoroalkylene group (15), may be optionally replaced by at least one divalent substituent selected from the group consisting of an oxygen atom, a carbonyl group, a sulfonyl group, a biscarbonylimido group, a bissulfonylimido group and a carbonylsulfonylimido group, with the proviso that the number of the divalent substituent is 50 % or less, based on the number q.

Examples of Rf groups include the below-mentioned divalent groups; however, the Rf group is not limited to the below-mentioned divalent groups:

$-C_aF_{2a}-$,
$-(CF_2)_a-CHFCF_2$,
$-C_aF_{2a}-O-C_cF_{2c}-$,
$-(C_aF_{2a}O)_b-C_cF_{2c}-$, and
$-(C_aF_{2a}O)_b-C_cF_{2c}SO_2NR^1SO_2C_dF_{2d}-$

wherein:

each of a, b, c and d is independently an integer of from 1 to 4; and
$R^1$ is as defined above for formula (1).

As a specific example of such monomer (a), there can be mentioned a monomer represented by the following formula (20):

$$CF_2=CFCF_2SO_2NR^7R^8 \qquad (20)$$

wherein:

$R^7$ and $R^8$ are as defined above for formula (11).

In addition, a monomer represented by the following formula (16):

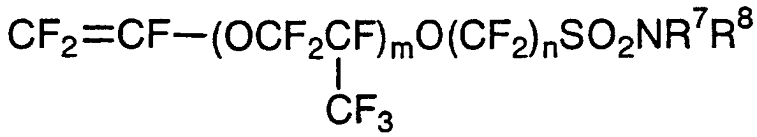

(16)

wherein:

m is an integer of from 0 to 5;
n is an integer of from 1 to 5; and
$R^7$ and $R^8$ are as defined above for formula (11), can also be advantageously used as the monomer (a). The monomer (16) corresponds to the monomer (1) having a structure in which $R^2$ is not the unsubstituted or substituted $C_1$-$C_{10}$ hydrocarbon group. The monomer (16) is more advantageous than the monomer (20), since the monomer (16) has higher polymerizability than the monomer (20).

In the monomer (16), it is preferred that m is as small as possible. In the monomer (16), m is more preferably from 0 to 2, still more preferably from 0 to 1, most preferably 0. Use of such monomer (16) is advantageous in that, even when the amount of monomer (16) is small, excellent effects can be obtained, such as the effects that an improved mechanical strength of the fluorinated copolymer can be obtained, and an improved ion-exchange capacity of the fluorinated copolymer can be obtained when the fluorinated copolymer is used as an ion-exchange resin.
n is an integer of from 1 to 5. From the viewpoint of improving the chemical stability of the monomer (16) and fluorinated copolymer obtained by using the monomer (16), and from the viewpoint of increasing the productivity of the monomer (16), n is preferably 2 or 3, and most preferably 2.
These monomers (a) can be used individually or in combination.

Monomer (b)

As mentioned above, the monomer (b) is at least one monomer having a partially fluorinated or perfluorinated vinyl group and a group (hereinafter referred to as "substituent (12)") represented by the following formula (12):

$$-SO_2X^3 \qquad (12).$$

That is, the monomer (b) has a structure in which the substituent (11) of the monomer (a) is replaced by the substituent (12).
In formula (12), $X^3$ represents a fluorine atom, a chlorine atom or an $-OR^9$ group, wherein $R^9$ represents the unsubstituted or substituted $C_1$-$C_{10}$ hydrocarbon group or the substituted silyl group.
With respect to $R^9$, the unsubstituted or substituted $C_1$-$C_{10}$ hydrocarbon group as $R^9$ is the same as the unsubstituted or substituted $C_1$-$C_{10}$ hydrocarbon group as $R^7$ in formula (11), and, on the other hand, the substituted silyl group as $R^9$ is the same as the substituted silyl group as $R^7$ or $R^8$ in formula (11).
From the viewpoint of improving the stability and handling properties of the monomer (b), $X^3$ is preferably a fluorine atom or a chlorine atom, more preferably a fluorine atom.
As a specific example of the monomer (b), there can be mentioned a monomer represented by the following formula (21):

$$CF_2=CF-Rf-SO_2X^3 \qquad (21).$$

In formula (21), $X^3$ is as defined above for formula (12), and Rf is as defined for formula (13).
In addition, the sulfonyl fluoride (5) used in the above-mentioned production method 2 may be preferably used as the monomer (b).
In the sulfonyl fluoride (5), m is an integer of from 0 to 5; however, for obtaining a fluorinated copolymer having excellent mechanical strength, it is preferred that m is from 0 to 2, more advantageously 0 or 1.
n is an integer of from 1 to 5. From the viewpoint of improving the chemical stability of the sulfonyl fluoride (5) and fluorinated copolymer obtained from the sulfonyl fluoride (5), and from the viewpoint of increasing the productivity of the sulfonyl fluoride (5), n is preferably 2 or 3, most preferably 2.

Specific examples of sulfonyl fluoride (5) are illustrated in the following formulae (22), (23), (24) and . (25).

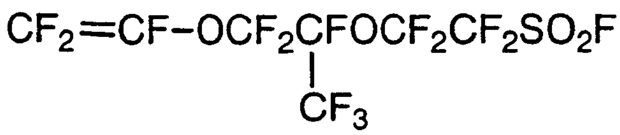

(22)

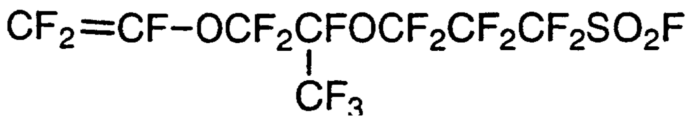

(23)

$$CF_2{=}CFOCF_2CF_2SO_2F \qquad (24)$$

$$CF_2{=}CFOCF_2CF_2CF_2SO_2F \qquad (25)$$

These monomers (b) can be used individually or in combination.

Monomer (c)

The fluorinated copolymer of the present invention can be obtained by subjecting the above-mentioned monomers (a) and (b) to copolymerization; however, as mentioned above, in addition to the monomers (a) and (b), there may also be used at least one monomer (c) other than the monomers (a) and (b), which has an olefinic unsaturated bond.

With respect to the structure of the monomer (c), there is no particular limitation, as long as the monomer (c) has an olefinic unsaturated bond and is copolymerizable with the monomers (a) and (b).

Examples of monomers (c) include olefins, such as ethylene and propylene; and halogenated olefins, especially halogenated ethylenes, such as vinylidene fluoride, tetrafluoroethylene and chlorotrifluoroethylene. These monomers (c) can be used individually or in combination.

Among these monomers (c), preferred are per-fluorolefins and chlorofluorolefins. Especially preferred are tetrafluoroethylene and chlorotrifluoroethylene, and most preferred is tetrafluoroethylene.

In the production of the fluorinated copolymer of the present invention, the type of monomers (a), (b) and (c) can be appropriately chosen in accordance with the properties of the desired copolymer. When using the above-mentioned compound (16) as the monomer (a) and the above-mentioned sulfonyl fluoride (5) as the monomer (b), there is no particular limitation; for example, it is not necessary that m and n in the compound (16) are, respectively, the same as m and n in the sulfonyl fluoride (5), that is, the structures of the compound (16) and the sulfonyl fluoride (5) can be appropriately chosen in accordance with the properties of the desired copolymer. Such freedom of the choice of the structures of the monomers cannot be obtained by the conventional amidation method in which the $-SO_2F$ group of a polymer is partially amidated.

With respect to the amounts of the above-mentioned monomers (a), (b) and (c), there is no particular limitation; however, from the viewpoint of improving the handling properties and the like of the obtained fluorinated copolymer, it is preferred that the monomer (a) is used in an amount of from 0.001 to 20 mol %, more advantageously from 0.005 to 10 mol %, most advantageously from 0.01 to 5 mol %, based on the total molar amount of the monomers (a), (b) and (c).

With respect to the monomer (b), it is preferred that the monomer (b) is used in an amount of from 3 to 95 mol %, more advantageously from 5 to 60 mol %, most advantageously from 10 to 30 mol %, based on the total molar amount of the monomers (a), (b) and (c).

With respect to the monomer (c), it is preferred that the monomer (c) is used in an amount of from 0 to 97 mol %, more advantageously from 50 to 92 mol %, most advantageously from 70 to 88 mol %, based on the total molar amount of the monomers (a), (b) and (c).

The substituent (12) of the monomer (b) is a group which can be easily converted into a free sulfonic acid group. A fluorinated copolymer containing a large number of the substituent (12) can be advantageously used as a

material for an ion-exchange resin having a large ion-exchange capacity, or as a material for a solid polymer electrolyte having excellent proton conductivity. Therefore, it is preferred that the monomer (b) is used in a relatively large amount.

On the other hand, the monomer (a) can exhibit a satisfactory effect even when it is used in a relatively small amount.

The fluorinated copolymer of the present invention, comprising monomer unit (A) derived from the monomer (a) and monomer unit (B) derived from the monomer (b), has for the first time been obtained by the present inventors. The fluorinated copolymer of the present invention is extremely advantageous not only in that it exhibits excellent properties suitable for high speed production of a film, but also in that a modification treatment for improving the mechanical strength of the fluorinated copolymer at high temperatures, can be performed efficiently.

It is preferred that the amount of monomer unit (A) is in the range of from 0.001 to 50 mol %, more advantageously from 0.005 to 30 mol %, most advantageously from 0.01 to 20 mol %, based on the total molar weight of the monomer units (A) and (B).

For the purpose of obtaining the fluorinated copolymer of the present invention exhibiting excellent handling properties and exhibiting a good balance of various properties, it is preferred that the weight of the fluorinated copolymer of the present invention per mole of sulfonyl groups in monomer unit (A) (derived from the monomer (a)) and monomer unit (B) (derived from the monomer (b)), is from 400 to 1400 g/mol, more advantageously from 600 to 1200 g/mol, most advantageously from 700 to 1100 g/mol.

Such value of the weight of the fluorinated copolymer corresponds to the "equivalent weight" of the fluorinated copolymer when the copolymer is regarded as an ion-exchange resin containing the substituents (11) and (12) as ion-exchange groups. The value is obtained by dividing the weight (g) of the fluorinated copolymer of the present invention by the total molar amount of the monomer units (A) and (B).

The melt index of the fluorinated copolymer of the present invention is preferably in the range of from 0.001 to 500, more preferably from 0.01 to 200, most preferably from 0.1 to 100, as measured under conditions wherein the load is 2.16 kg, the orifice diameter is 2.09 mm and the temperature is in the range of from the melting temperature of the copolymer to lower than the decomposition temperature of the copolymer.

The fluorinated copolymer of the present invention is a novel copolymer which has for the first time been obtained by the present inventors and which has the substituents (11) and (12) at the terminals of the side chains thereof. As mentioned above, when such copolymer is subjected to the below-mentioned modification treatment, the heat resistance of the copolymer can be remarkably improved.

In addition, the fluorinated copolymer intrinsically has relatively high heat resistance, and the fluorinated copolymer does not suffer deterioration even when the copolymer is subjected to melt molding under heating. Further, the copolymer does not have a crosslinked structure, and hence can be easily molded into a shaped article, such as a copolymer film, by various conventional molding methods, such as a melt molding method. Thus, in the present invention, a modified copolymer film can be obtained by a process which comprises the steps of producing a copolymer film by a melt molding method, and subjecting the obtained copolymer film to modification treatment. Such process is advantageous for a commercial practice.

The above-mentioned fluorinated polymer, especially the fluorinated copolymer, can be easily molded by various conventional molding methods to thereby obtain various molded articles, such as a film. If desired, the fluorinated polymer can be used in the form of a composition which is obtained by mixing the fluorinated polymer with another polymer.

A copolymer film produced from a composition containing the above-mentioned fluorinated copolymer is especially useful as a material for a solid polymer electrolyte membrane for use in a fuel cell, the film having excellent thermal stability. Hereinbelow, an explanation is made on the method for producing the fluorinated polymer film of the present invention.

With respect to the method for producing a film from the fluorinated polymer of the present invention, there is no particular limitation, and a film can be produced by the conventional methods, such as a calendar method, an inflation method, a T-die method, a casting method, a cutting method, an emulsion method and a hot-press method. The fluorinated polymer of the present invention has excellent thermal stability and does not suffer marked deterioration upon heating. From the viewpoint of increasing the productivity of a film, the methods based on melt processing, such as a T-die method and an inflation method, are especially preferred among the above-mentioned methods.

In addition, for producing a thin film of the fluorinated polymer, a casting method which employs a solution of the fluorinated polymer is also preferred.

With respect to the thickness of the polymer film, there is no particular limitation. The thickness of the polymer film can be appropriately chosen in accordance with the use of the polymer film; however, the thickness of the polymer film is preferably from 5 to 200 μm, more preferably from 10 to 150 μm, most preferably from 20 to 100 μm.

The obtained polymer film can be used in the form of a single-layer film or in the form of a multi-layer film which is obtained by laminating the polymer film with other film(s). The term "single-layer film" used herein means a film having a uniform composition, and such film is most suitable for the below-mentioned modification treatment. If

desired, the single-layer film may constitute a part of a multi-layer film comprising the single-layer film and other structure (s) or other film(s) having a composition different from the composition of the single-layer film.

The single-layer film has a structure in which the above-mentioned substituents (11) and (12) are uniformly dispersed throughout the interior portion of the film. Such single-layer film has for the first time been obtained by the present inventors.

Further, the above-mentioned polymer film may be a composite material obtained by combining the polymer film with various reinforcements, such as a fibrous reinforcement, a granular reinforcement and a porous membrane. Specific examples of reinforcements include PTFE microparticles, PTFE fibrils, a PTFE woven fabric, a PTFE porous membrane, and various inorganic reinforcements.

Unexamined Japanese Patent Application Laid-Open Specification No. 2001-319521 discloses a method in which a polymer film containing an $-SO_2F$ group is treated with ammonia (see the section "Prior Art" of this patent document). The present inventors made a study on this method, and they found that the IR spectrum of an ammonia-treated film obtained by this method shows that, in addition to $-SO_2NH_2$ groups, a large amount of sulfonic acid groups (in ammonium salt form) are present in the ammonia-treated film. The reason for this is considered to reside in that, in the case of this prior art method, the entering of even a very small amount water into the reaction system is likely to cause the conversion of the $-SO_2F$ group into a sulfonic acid group (in ammonium salt form). On the other hand, from the IR spectrum of the copolymer film of the present invention, which contains the $-SO_2NH_2$ group, it has been confirmed that the formation of a sulfonic acid group (in ammonium salt form) does not occur at all.

Among the above-mentioned polymer films, a fluorinated copolymer film produced from a fluorinated copolymer obtained by copolymerizing the above-mentioned monomers (a) and (b), and optionally the monomer (c), is advantageous in that, when the copolymer film is subjected to a modification treatment, there can be obtained a large improvement in the thermal stability of the copolymer film. Especially the mechanical strength and the elasticity both at high temperatures become improved. In addition, the occurrence of creep deformation at high temperatures becomes remarkably decreased. Further, the modified fluorinated copolymer film also has advantageous properties in that, even when the modified fluorinated copolymer film is converted into a solid polymer electrolyte membrane by the below-mentioned method, the membrane does not exhibit a lowering of the proton conductivity thereof. Hereinbelow, an explanation is made of the modification treatment for improving the thermal stability of the copolymer film.

The modification treatment is performed by contacting the fluorinated copolymer film with a basic compound. Examples of basic compounds include various Lewis bases and BrØnsted bases. Specific examples of such basic compounds include a nitrogen-containing organic Lewis base and a compound represented by the following formula:

$$Q^+Y^-$$

wherein $Q^+$ represents a quaternary ammonium group, a quaternary phosphonium group, an alkali metal, an alkaline earth metal or the like; and $Y^-$ represents an alkoxyl group, an allyloxy group, an amino group having a hindered amine structure, a fluoride ion or the like.

It is preferred that the basic compound is an anhydrous compound.

Nitrogen-containing organic Lewis bases can be used for the modification treatment of a wide variety of fluorinated copolymer films. Specific examples of nitrogen-containing organic Lewis bases include tertiary amines, such as trimethylamine, triethylamine, tripropylamine, tributylamine, tetramethylethylenediamine and dimethylaniline; partially fluorinated tertiary amines, such as $N(CH_2CH_2OCF_2CHFCF_3)_3$; and nitrogen-containing heterocyclic compounds, such as pyridine, an alkylsubstituted pyridine, N,N-dimethylaminopyridine, quinoline, 1,4-diazabicyclo[2.2.2]octane (DABCO), 1,8-diazabicyclo[5.4.0]-7-undecene (DBU), 1,5-diazabicyclo[4.3.0]-5-nonene (DBN), imidazole and the derivatives thereof. Among the above-mentioned compounds, preferred are tertiary amines, N,N-dimethylamino pyridine and superstrong bases (DABCO, DBU, DBN and the like).

When the fluorinated copolymer film is produced from a copolymer comprising monomer units (A) in which at least one of $R^7$ and $R^8$ is a substituted silyl group, among the compounds represented by the formula $Q^+Y^-$, it is also effective to employ a compound in which $Y^-$ represents a fluoride ion (i.e., a fluoride ion-containing compound). Examples of such compounds include metal fluorides, such as lithium fluoride, sodium fluoride, potassium fluoride, rubidium fluoride and cesium fluoride; quaternary ammonium fluorides, such as tetrabutylammonium fluoride and tetramethylammonium fluoride; and quaternary phosphonium fluorides, such as tetrabutylphosphonium fluoride and teramethylphosphonium fluoride. Among the above-mentioned compounds, preferred are potassium fluoride and cesium fluoride, and more preferred is potassium fluoride.

The modification treatment may be performed by using a solvent. With respect to the type of solvent used, there is no particular limitation, and there can be used various solvents. Examples of solvents include fluorine-containing solvents, such as HCFC225ca/cb and HFC43-10mee; ether type solvents, such as a glyme and a dioxane; and polar solvents, such as dimethylsulfoxide and dimethylformamide. However, when the modification treatment is performed

in the presence of a large amount of water, the substituent (12) of the copolymer becomes hydrolyzed markedly, rendering it impossible to obtain the desired improvement of the thermal stability. Therefore, it is preferred that the entering of water into the reaction system is suppressed to a level as low as possible.

The modification treatment is generally performed at a temperature which is 0 °C or higher, preferably 40 °C or higher, more preferably 60 °C or higher, and which is 200 °C or lower, preferably 150 °C or lower. When a fluoride ion-containing compound is used as a basic compound, it is preferred that the modification treatment is performed at a temperature which is slightly higher than the temperature used for the modification treatment performed with a nitrogen-containing organic Lewis base.

The reason why the thermal stability, especially the mechanical strength at high temperatures, becomes improved by the above-mentioned modification treatment has not yet been fully elucidated, but it is presumed that a crosslinking structure is formed by the reactions represented by formulae (26) and (27) below. Indeed, the formation of a bissulfonyl imide linkage shown in formulae (26) and (27) below has been confirmed from the IR spectrum of the modified copolymer film:

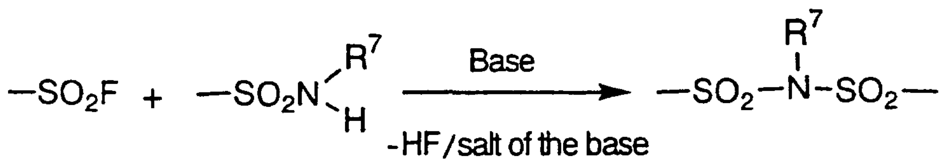

(26)

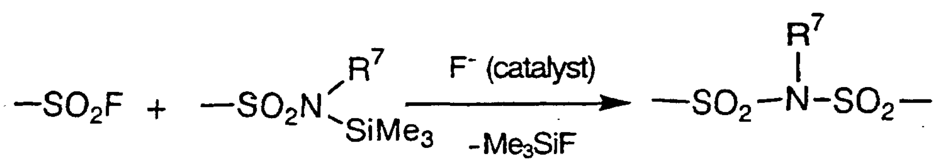

(27)

wherein $R^7$ is as defined above for formula (11).

In the case where $R^7$ in the above formulae (26) and (27) is an alkyl group or a substituted silyl group, when the modification treatment of the fluorinated copolymer film is followed by an acid treatment of the modified copolymer film, a bissulfonyl imide linkage containing an N-H group is formed, as shown in the following formula (28) and (29):

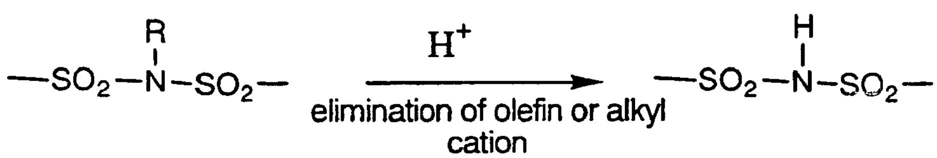

(28)

wherein R represents an alkyl group; and

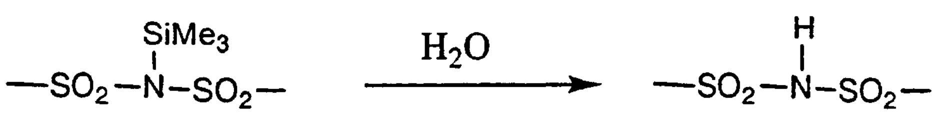

(29).

The fluorinated copolymer of the present invention can be used to formulate a resin composition having excellent dispersibility, together with another polymer containing monomer units derived from the sulfonyl fluoride (5) above (for example, a copolymer of TFE and sulfonyl fluoride (5)). Such resin composition can be processed by a method in which a copolymer film is produced from such resin composition, and the obtained copolymer film is subjected

to a modification treatment in the above-mentioned manner.

For producing a solid polymer electrolyte membrane, the fluorinated copolymer film which has been subjected to the modification treatment is further subjected to an alkali treatment and/or an acid treatment in the same manner as in the generally employed method for producing a membrane for use in a fuel cell, to thereby convert the fluorinated copolymer film into a solid polymer electrolyte membrane. In general, a solid polymer electrolyte membrane having sulfonic acid groups in the form of a salt thereof can be obtained by subjecting the modified fluorinated copolymer film (which has been modified by the above-mentioned modification treatment) to an alkali treatment. On the other hand, a solid polymer electrolyte membrane having free sulfonic acid groups can be obtained by subjecting the modified fluorinated copolymer film to an acid treatment. However, for efficiently producing a solid polymer electrolyte membrane having free sulfonic acid groups under moderate conditions, in general, a method is employed in which a modified fluorinated copolymer film is first treated with an alkaline compound to thereby obtain a solid polymer electrolyte membrane having sulfonic acid groups in the form of a salt thereof and, then, the obtained solid polymer electrolyte membrane is treated with an acid to thereby obtain a solid polymer electrolyte membrane having free sulfonic acid groups.

As an alkaline compound, there can be used inorganic alkaline compounds, such as NaOH and KOH; and amines, such as triethylamine and diethylamine. The alkaline compounds are generally used in the form of an aqueous solution thereof. The alkali treatment can be performed under conditions which are generally employed for treating a conventional polymer containing an $-SO_2F$ group. For example, the alkali treatment is performed in an aqueous NaOH solution or an aqueous KOH solution at a temperature in the range of from room temperature to 100 °C. The aqueous NaOH solution and the aqueous KOH solution may contain an organic solvent, such as an alcohol, a water-soluble ether, dimethylformamide and dimethylsulfoxide.

The acid treatment is generally performed in an aqueous solution of a strong acid, such as hydrochloric acid, sulfuric acid or trifluoromethanesulfonic acid, at a temperature in the range of from room temperature to 100 °C. After the acid treatment, the treated film is washed thoroughly with water to thereby obtain a desired solid polymer electrolyte membrane having free sulfonic acid groups.

The thus obtained solid polymer electrolyte membrane exhibits improved mechanical strength at high temperatures and can be advantageously used as a membrane for use in a fuel cell.

The fluorinated polymer obtained by using the perfluorovinyl ether monomer of the present invention, irrespective of whether or not the fluorinated polymer has been subjected to modification treatment, can be used not only for producing a membrane for use in a fuel cell, but can also be used in a wide variety of fields, such as various materials for electrochemical elements, such as a binder for a catalyst in a fuel cell, and a solid polymer electrolyte for use in a lithium ion battery; various separation membranes, such as an ion-exchange membrane for a chlor-alkali process and an anti-ozone separation membrane; and ion-exchange resins.

Hereinbelow, an explanation is made on the other uses for the fluorinated polymer obtained by using the monomer (1) of the present invention.

The fluorinated polymer obtained by using the monomer (1) of the present invention can be used as a material for various functional resins and functional films, even without subjecting the fluorinated polymer to a modification treatment.

A fluorinated polymer obtained by using the monomer (1) wherein at least one of $R^1$ and $R^2$ is a hydrogen atom, can be used as a weakly acidic ion-exchange fluorinated polymer, because the hydrogen atoms as $R^1$ and/or $R^2$ exhibit weak acidity. An example of a use of such polymer include a use in the apparatus for the electrolysis of sodium chloride, which is disclosed in U.S. Patent No. 3,784,399. Specifically, the above-mentioned polymer can be used as a material for a weakly acidic ion-exchange fluorinated polymer layer formed on the surface of a membrane for the electrolysis of sodium chloride, the polymer layer being formed for improving the efficiency of the electrolysis.

In addition, such a weakly acidic ion-exchange resin may be molded into spherical microparticles, and the obtained spherical microparticles can be used as an ion-exchange resin for separation and purification.

Further, with respect to the (co)polymer produced using the monomer (1) of the present invention, the $-SO_2NR^1R^2$ moiety thereof can be converted into various functional groups as shown in the below-mentioned items a), b) and c), in accordance with the use of the (co)polymer.

a) Conversion into a strongly acidic ion-exchange polymer

The $-SO_2NR^1R^2$ group of the fluorinated polymer produced from the monomer (1) of the present invention can be converted into a strongly acidic $-SO_3H$ group, and the resultant modified polymer can be used in a wide variety of fields, such as a membrane for use in a fuel cell, a membrane for use in an apparatus for the electrolysis of sodium chloride, a strongly acidic catalyst and an ion-exchange resin for separation and purification.

The conversion of the $-SO_2NR^1R^2$ group into the $-SO_3H$ group can be performed, for example, under acidic conditions or alkaline conditions or in an acidic atmosphere or in the presence of water. These conditions can be used individually or in combination. The difficulty of the conversion reaction varies depending on the structure of the

$-SO_2NR^1R^2$ group and, thus, the reaction conditions need to be selected in accordance with the structure of the $-SO_2NR^1R^2$ group. It is especially preferred that the $-NR^1R^2$ group is a nitrogen-containing aromatic group, such as an imidazolyl group or a pyrolyl group, because, in such case, the $-SO_2NR^1R^2$ group can be converted efficiently into the $-SO_3H$ group under moderate temperature conditions in an acidic atmosphere.

b) Conversion into a weakly acidic ion-exchange polymer

A fluorinated polymer obtained by using the monomer (1) wherein at least one of $R^1$ and $R^2$ is an alkyl group or a substituted silyl group, can be subjected to the below-mentioned treatment with a protic compound, such as an acid, an alcohol or water, to thereby dealkylate or desilylate the polymer and convert the fluorinated polymer into an NH group-containing polymer. The resultant weakly acidic, NH group-containing polymer can be used in various fields which are mentioned above in connection with the weakly acidic ion-exchange resin.

<Examples of conversions into an $-SO_2NH-$ group>

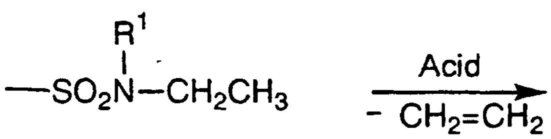


(30)

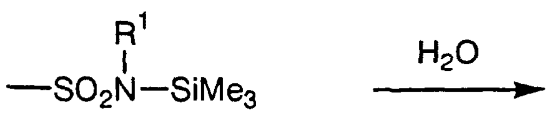


(31)

wherein $R^1$ is as defined above for formula (1).

c) Other modifications of polymer

A fluorinated polymer obtained by using the monomer (1) wherein at least one of $R^1$ and $R^2$ is a hydrogen atom or a substituted silyl group, has a highly reactive N-H group or N-Si linkage. By virtue of such a highly reactive structure, the fluorinated polymer can be reacted with various compounds to thereby modify the polymer structure of the fluorinated polymer. Further, when the $-NR^1R^2$ group is a nitrogen-containing aromatic group, such as imidazolyl group or a pyrolyl group, the $-NR^1R^2$ group can be easily converted into other functional groups by reacting the fluorinated polymer with an active hydrogen-containing compound (acidic compound), such as hydrogen chloride (HCl).

<Examples of conversions into a bissulfonyl imido group and a sulfonylcarbonyl imido group>

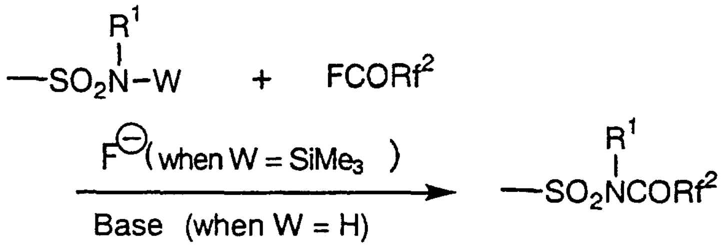


(32)

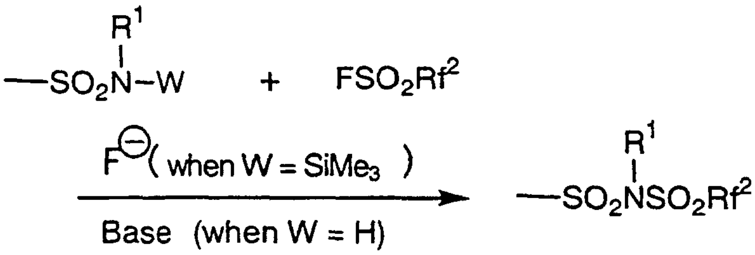


(33)

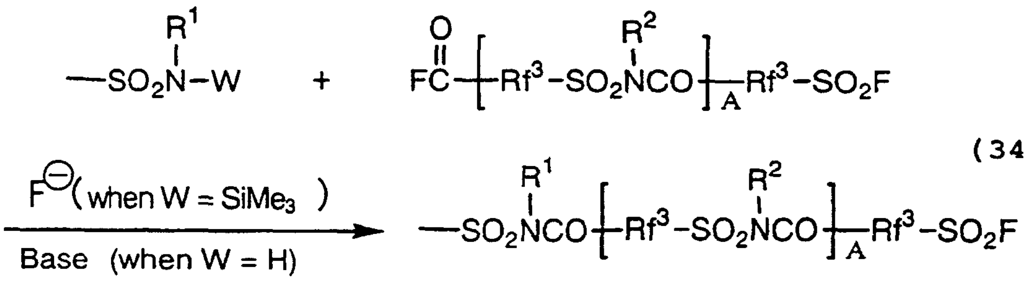


(34)

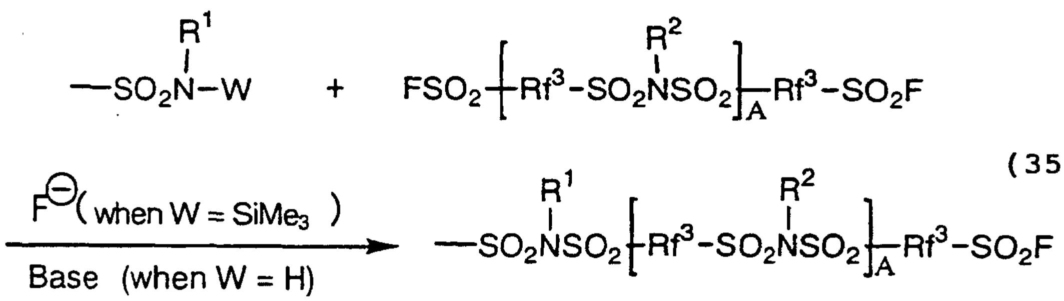


(35)

wherein:

$R^1$ and $R^2$ are as defined above for formula (1);
each $Rf^2$ independently represents a perfluoroalkyl group, a perfluoroalkoxyl group or a perfluoropolyoxyalkylene group, each having 1 to 15 carbon atoms, preferably 1 to 8 carbon atoms, more preferably 1 to 4 carbon atoms;

each $Rf^3$ independently represents a perfluoroalkylene group, a perfluorooxyalkylene group or a perfluoropolyoxyalkylene group, each having 1 to 15 carbon atoms, preferably 1 to 8 carbon atoms, more preferably 1 to 4 carbon atoms; and

A is an interger of from 0 to 20, preferably 0 to 5, more preferably 0 to 2.

In the formulae (32) to (35) above, when $R^1$ in the N-$R^1$ group of the reaction product is an alkyl group, the reaction product can be subjected to a dealkylation reaction by contacting the reaction product with an acid catalyst or by heating, thereby easily converting the N-$R^1$ group into the N-H group.

<Example of a reaction of an imidazole derivative>

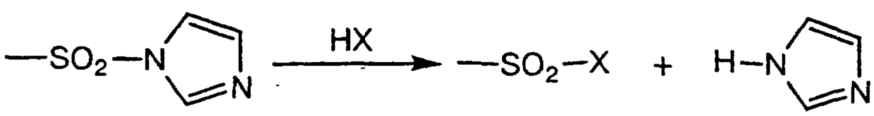


wherein HX represents an active hydrogen-containing compound (acidic compound), such as hydrogen chloride.

If desired, before the use of the monomer (1) of the present invention, the -$SO_2NR^1R^2$ group thereof may be converted into other functional groups by using the modification methods as described in items b) and c) above.

As explained hereinabove, the perfluorovinyl ether monomer (1) of the present invention can be used for producing various functional materials, such as a solid polymer electrolyte (membrane) haying high thermal stability and, thus, the perfluorovinyl ether monomer (1) of the present invention is very useful.

## BEST MODE FOR CARRYING OUT THE INVENTION

Hereinbelow, the present invention will be described in more detail with reference to the following Examples, Comparative Examples and Reference Example, but they should not be construed as limiting the scope of the present invention.

In the Examples, Comparative Examples and Reference Example, various measurements were conducted by the following methods.

1. Fluorine-19 and proton nuclear magnetic resonance ($^{19}F$- and $^1H$-NMR) spectra

The $^{19}F$-NMR spectrum of a monomer was obtained using a nuclear magnetic resonance (NMR) apparatus (Lambda-400 or GSX-400; manufactured and sold by JEOL LTD., Japan). In the $^{19}F$-NMR analysis, deuterated chloroform was used as a solvent, and Freon-11 ($CFCl_3$) was used as a standard.

The $^1H$-NMR spectrum of a monomer was obtained using a nuclear magnetic resonance (NMR) apparatus (Lambda-400 or GSX-400; manufactured and sold by JEOL LTD., Japan). In the $^1H$-NMR analysis, deuterated chloroform was used as a solvent, and tetramethylsilane (TMS) or chloroform (contained in the above-mentioned deuterated chloroform) was used as a standard.

In the NMR analyses of a monomer, a double sample tube was used.

The $^{19}F$-NMR spectrum of a solid polymer was obtained by the magic angle spinning (MAS) method using a nuclear magnetic resonance (NMR) apparatus (DSX-400; manufactured and sold by BRUKER BIOSPIN, Germany). In the $^{19}F$-NMR analysis, Freon-113 ($CFCl_2CF_2Cl$) was used as a standard.

2. Infrared absorption spectrum

The infrared absorption spectrum (IR) was obtained by the transmission method (i.e., the KBr tablet method or film method, in which a sample (neat sample) was directly coated on a window used in the IR analysis) or the attenuated total reflectance method, using a FT-IR spectrometer (2000FT-IR; manufactured and sold by Perkin Elmer, U.S.A., or FTS-6000; manufactured and sold by BIO-RAD, U.S.A.).

3. Gas chromatography (GC)

The gas chromatography was conducted under the following conditions.

Apparatus: 5890 series II (manufactured and sold by HEWLETT PACKARD, U.S.A.)

Column: capillary column DB-1 (inner diameter: 0.25 mm, column length: 30 m, film thickness: 1 μm) (manufactured and sold by J & W Scientific, U.S.A.)

Carrier gas: helium

Detector: flame ionization detector (FID)

4. Gas chromatography-Mass spectrometry (GC-MS)

The GC-MS was conducted under the following conditions.

Apparatus: Automass-Sun (manufactured and sold by JEOL LTD., Japan)

Column: capillary column DB-1 (inner diameter: 0.25 mm, column length: 30 m, film thickness: 1 μm) (manufactured and sold by J & W Scientific, U.S.A.)

Carrier gas: helium

5. Tensile modulus

A rectangular test specimen having a size of about 30 mm × 3 mm was cut out from a sample (a copolymer film). The tensile modulus of the test specimen was measured using a dynamic viscoelasticity measuring apparatus (RHEOVIBRON DDV-01FP) (tradename; manufactured and sold by A&D CO., Ltd., Japan) under conditions wherein the temperature was in the range of from room temperature to 300 °C and the frequency was 35 Hz.

6. Melt index (MI)

The melt index was measured using D4002 (manufactured and sold by Dynisco, U.S.A.) under conditions wherein the temperature was 270 °C, the load was 2.16 kg and the orifice diameter was 2.09 mm.

Example 1

(I) Neutralization reaction

103.8 g of a compound represented by the following formula: $CF_3CF(COF)OCF_2CF_2SO_2F$ was dropwise added to a slurry comprising 31.8 g of sodium carbonate and 150 ml of acetonitrile, under a stream of nitrogen gas at room temperature to thereby obtain a mixture. The obtained mixture was stirred at room temperature for 1 hour and then stirred at 40 °C for 1 hour to effect a reaction, thereby obtaining a reaction mixture. The obtained reaction mixture was subjected to filtration to thereby remove a precipitate which was formed during the reaction. Then, the solvent in the reaction mixture was distilled off under reduced pressure, thereby obtaining 96.0 g of a white solid. From the $^{19}F$-NMR spectrum of the solid, it was confirmed that the solid was a sodium carboxylate represented by the following formula: $CF_3CF(CO_2Na)OCF_2CF_2SO_2F$.

$^{19}F$-NMR: δ(ppm)-125.5(1F), -112.7(2F), -82.9(3F), -81.7, -79.7(2F), 43.7(1F).

(II) Amidation reaction

11.0 g of diethylamine was dissolved in 150 ml of anhydrous tetrahydrofuran to obtain a solution. The obtained solution was cooled to -78 °C. 100 ml of a (1.6 moles/liter) solution of n-butyllithium (BuLi) in n-hexane was dropwise added to the solution under a stream of nitrogen gas, and the resultant mixture was stirred at -78 °C for 1 hour to obtain a solution. On the other hand, 54.9 g of the above-mentioned sodium carboxylate was dissolved in 150 ml of anhydrous tetrahydrofuran to obtain a solution. The obtained solution was dropwise added to the above-mentioned solution at -78 °C to thereby obtain a mixture. The temperature of the obtained mixture was elevated to room temperature, and the obtained mixture was stirred at room temperature for 5 hours to effect a reaction, thereby obtaining a reaction mixture. The obtained reaction mixture was subjected to filtration to thereby remove a precipitate which was formed during the reaction. Then, the solvent in the reaction mixture was distilled off under reduced pressure to thereby obtain a residue. The residue was subjected to a vacuum drying at 70 °C, thereby obtaining 63.6 g of a yellow solid. From the $^{19}F$-NMR spectrum and an IR spectrum of the solid, it was confirmed that the solid was a sulfonamide represented by the following formula: $CF_3CF(CO_2Na)OCF_2CF_2SO_2N(C_2H_5)_2$.

$^{19}F$-NMR: δ(ppm)-125.2(1F), -116.1(2F), -82.2(3F), -82(1F), -79(1F).

IR(KBr): 2990, 1695, 1382, 1223, 1162 $cm^{-1}$.

(III) Decarboxylation-vinylation reaction

20.4 g of the sulfonamide obtained in the step (II) above was introduced into a flask,equipped with a distillation head, and the flask was heated under a pressure of 8 $\times$ $10^{-3}$ MPa at 200 °C, to generate a vapor. The vapor was condensed and recovered as a distillate, thereby obtaining 11.2 g of a pale yellow liquid. From the $^{19}$F-NMR and $^{1}$H-NMR spectra and the IR spectrum of the liquid and the GC-MS chart of the liquid, it was confirmed that the main component of the liquid was a perfluorovinyl ether represented by the following formula: $CF_2$=$CFOCF_2CF_2SO_2N(C_2H_5)_2$.

$^{19}$F-NMR: $\delta$ (ppm) -137 (1F), -124 (1F), -117.6 (2F), -116 (1F), -85.3 (2F).
$^{1}$H-NMR: $\delta$ (ppm) 1.27 (3H), 3.3 -3.7 (2H).
IR (neat): 2988, 1390, 1215, 1166 $cm^{-1}$.
EI-MS: m/z 136, 100, 97, 81, 44, 29.

Further, it was also confirmed that the above-mentioned liquid contained a small amount of a proton-substituted product represented by the following formula: $CF_3CHFOCF_2CF_2SO_2N(C_2H_5)_2$ (perfluorovinyl ether : proton-substituted product = 94 : 6). However, it was confirmed that the above-mentioned liquid did not contain a cyclization reaction product.

Example 2

7.5 g of the perfluorovinyl ether obtained in Example 1 (which was purified by redistillation), 22 g of HFC43-10mee and 2.2 g of a 5 % $(CF_3CF_2CF_2COO)_2$ solution in HFC43-10mee (wherein $(CF_3CF_2CF_2COO)_2$ is a polymerization initiator) were introduced into a 200 ml volume pressure resistant vessel which was made of a stainless steel and which was equipped with a gas introduction pipe. The atmosphere in the pressure resistant vessel was fully replaced by nitrogen. Tetrafluoroethylene (TFE) was introduced into the pressure resistant vessel through the gas introduction pipe so that the internal pressure of the pressure resistant vessel was elevated to 0.5 MPa. Then, a reaction was performed at 25 °C for 3.5 hours while stirring and appropriately introducing TFE so as to maintain the internal pressure of the pressure resistant vessel at 0.5 MPa.

Thereafter, the introduction of TFE was stopped and the internal pressure of the pressure resistant vessel was lowered to atmospheric pressure, to obtain a reaction mixture (a white turbid liquid). Methanol was added to the obtained reaction mixture to precipitate a solid. The solid was recovered by filtration and washed with methanol, followed by drying, to obtain 0.7 g of a white solid.

From the $^{19}$F-NMR spectrum of the solid, it was confirmed that the solid was a copolymer comprising a monomer unit (a sulfonamide unit) which was derived from the perfluorovinyl ether obtained in Example 1 and a monomer unit (a TFE unit) which was derived from TFE. It was also confirmed that the ratio between sulfonamide units and TFE units (sulfonamide unit : TFE unit molar ratio) was 1 : 8.

Further, the copolymer was subjected to a press molding at 250 °C, to obtain a copolymer film.

Comparative Example 1

2.0 g of the sodium carboxylate synthesized in the step (I) of Example 1 was introduced into a flask equipped with a distillation head. The flask was heated under atmospheric pressure at 200 °C to generate a vapor. The vapor was condensed and recovered as a distillate, thereby obtaining 0.8 g of a colorless liquid. From the $^{19}$F-NMR spectrum of the solution, it was confirmed that the liquid was a cyclization reaction product represented by the following formula:

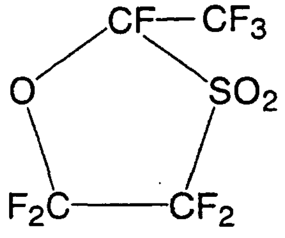

$^{19}$F-NMR: $\delta$(ppm)-125(1F), -120(1F), -115.3(1F), -90(1F), -80.5(3F), -78(1F).

It was found that the liquid contained no perfluorovinyl ether obtained in Example 1.

Example 3

(I) Amidation reaction

13.7 ml of a (2 moles/liter) solution of dimethylamine in THF was diluted with 60 ml of THF to obtain a solution. The obtained solution was cooled to -78 °C. 18.8 ml of a (1.6 moles/liter) solution of n-BuLi in n-hexane was dropwise added to the solution under a stream of nitrogen gas, and the resultant mixture was stirred at -78 °C for 1 hour, to obtain a solution. On the other hand, 10 g of the sodium carboxylate obtained in the step (II) of Example 1 was dissolved in 40 ml of anhydrous THF to obtain a solution. The obtained solution was dropwise added to the above-mentioned solution at -78 °C to obtain a mixture. The temperature of the obtained mixture was elevated to room temperature, and the obtained mixture was stirred at room temperature for 5 hours to effect a reaction, thereby obtaining a reaction mixture. The obtained reaction mixture was subjected to filtration to thereby remove a precipitate which was formed during the reaction. Then, the solvent in the reaction mixture was distilled off under reduced pressure to thereby obtain a residue. The residue was subjected to a vacuum drying at 70 °C, thereby obtaining 10.9 g of a yellow solid. From the $^{19}$F-NMR spectrum of the solid, it was confirmed that the solid was a sulfonamide represented by the following formula: $CF_3CF(CO_2Na)OCF_2CF_2SO_2N(CH_3)_2$.

$^{19}$F-NMR: δ(ppm)-125.8(1F), -115.2(2F), -82.1(3F), -82(1F), -80(1F).

(II) Decarboxylation-vinylation reaction

2.0 g of the sulfonamide obtained in the step (I) above was introduced into a flask equipped with a distillation head, and the flask was heated under a pressure of $4 \times 10^{-3}$ MPa at 250 °C, to generate a vapor. The vapor was condensed and recovered as a distillate, thereby obtaining 0.77 g of a pale yellow liquid. From the $^{19}$F-NMR spectrum of the liquid, it was confirmed that the main component of the liquid was a perfluorovinyl ether represented by the following formula: $CF_2=CFOCF_2CF_2SO_2N(CH_3)_2$.

$^{19}$F-NMR: δ(ppm)-137(1F), -124(1F), -116.7(2F), -116(1F), -85.8(2F).

Further, it was also confirmed that the above-mentioned liquid contained a small amount of a proton-substituted product represented by the following formula: $CF_3CHFOCF_2CF_2SO_2N(CH_3)_2$ (perfluorovinyl ether : proton-substituted product = 89 : 11). However, it was confirmed that the above-mentioned liquid contained no cyclization reaction product.

Example 4

(I) Amidation reaction

Amidation reaction was performed in substantially the same manner as in the step (I) of Example 3, except that 2.54 g of aniline was used instead of the solution of dimethylamine in THF, thereby obtaining 13.0 g of a yellow solid. From the $^{19}$F-NMR spectrum of the solid, it was confirmed that the solid was a sulfonamide represented by the following formula:

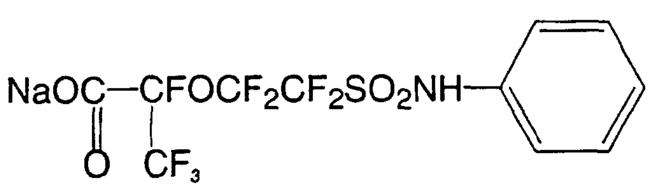

$^{19}$F-NMR: δ(ppm)-131.2(1F), -115.5(2F), -86(1F), -82.3(3F), -75(1F).

(II) Decarboxylation-vinylation reaction

2.0 g of the sulfonamide obtained in the step (I) above was dissolved in 10 ml of diglyme to obtain a solution. The obtained solution was heated under a stream of nitrogen gas at 150 °C, thereby obtaining a reaction mixture. The obtained reaction mixture was analyzed by gas chromatography (GC). As a result, the presence of two different products was confirmed. However, these products were different from the cyclization reaction product obtained in Comparative Example 1.

The solvent was distilled off from the reaction mixture under reduced pressure, and the residue was subjected to distillation under reduced pressure to thereby obtain 0.4 g of a yellow liquid. From the $^{19}F$-NMR spectrum of the liquid, it was confirmed that the main component of the solution was a perfluorovinyl ether represented by the following formula:

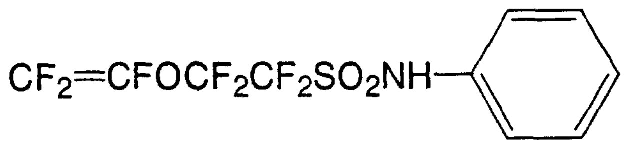

$^{19}F$-NMR: δ(ppm)-136(1F), -122(1F), -114.8(2F), -113(1F), -83.7(2F).

Further, it was also confirmed that the above-mentioned yellow liquid contained a small amount of a proton-substituted product represented by the following formula:

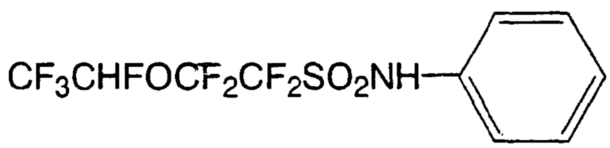

However, it was confirmed that the above-mentioned yellow liquid contained no cyclization reaction product.

Example 5

(I) Amidation reaction

Amidation reaction was performed in substantially the same manner as in the step (I) of Example 3, except that 2.0 g of t-butylamine was used instead of the solution of dimethylamine in THF, thereby obtaining 11.3 g of a yellow solid. From the $^{19}F$-NMR spectrum of the solid, it was confirmed that the solid was a sulfonamide represented by the following formula:

$$CF_3CF(CO_2Na)OCF_2CF_2SO_2NH^tBu$$

wherein $^tBu$ represents a t-butyl group and, hereinafter, a t-butyl group is represented by "$^tBu$".
$^{19}F$-NMR: δ(ppm)-131.2(1F), -116(2F), -86(1F), -82.4(3F), -76(1F).

(II) Decarboxylation-vinylation reaction

Decarboxylation-vinylation reaction was performed in substantially the same manner as in the step (II) of Example 3, except that 2.2 g of the sulfonamide obtained in the step (I) above was used instead of 2.0 g of the sulfonamide obtained in the step (I) of Example 3, thereby obtaining 0.57 g of a light yellow liquid. From the $^{19}F$-NMR spectrum of the liquid, it was confirmed that the main component of the liquid was a perfluorovinyl ether represented by the following formula: $CF_2=CFOCF_2CF_2SO_2NH^tBu$.

$^{19}F$-NMR: δ(ppm)-136(1F), -122(1F), -116.3(2F), -115(1F), -83.9(2F).

Further, it was also confirmed that the above-mentioned liquid contained a small amount of a proton-substituted product represented by the following formula: $CF_3CHFOCF_2CF_2SO_2NH^tBu$. However, it was confirmed that the above-mentioned liquid contained no cyclization reaction product.

Example 6

1.7 g of a dispersion of sodium hydride in a mineral oil (sodium hydride content: 60 %) was washed with n-hexane under a stream of nitrogen gas so as to remove the mineral oil and obtain a sodium hydride powder. 150 ml

of anhydrous acetonitrile was added to the obtained sodium hydride powder to obtain a mixture. The obtained mixture was cooled to 0 °C and 2.7 g of pyrrole was dropwise added to the mixture. Then, the temperature of the resultant solution was elevated to room temperature, and the solution was stirred for 1 hour, thereby obtaining a pyrrole sodium amide solution.

On the other hand, 15 g of the sodium carboxylate obtained in the step (II) of Example 1 was dissolved in 100 ml of anhydrous acetonitrile, to obtain a solution. To the obtained solution was dropwise added the above-mentioned pyrrole sodium amide solution at 0 °C. The temperature of the resultant mixture was elevated to room temperature, followed by stirring at room temperature for 12 hours to effect a reaction, thereby obtaining a reaction mixture. The obtained reaction mixture was subjected to filtration to thereby remove a precipitate which was formed during the reaction. Then, the solvent in the reaction mixture was distilled off under reduced pressure to thereby obtain a residue. The obtained residue was subjected to a vacuum drying at 50 °C, thereby obtaining 18.3 g of a brown solid. From the $^{19}$F-NMR spectrum of the solid, it was confirmed that the solid was a sulfonamide represented by the following formula:

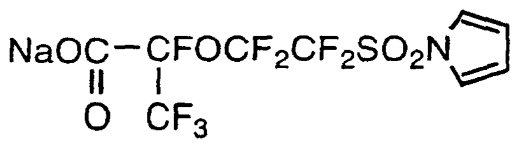

$^{19}$F-NMR: δ(ppm)-125.3(1F), -115.1(2F), -82.3(3F), -81(1F), -79(1F).

(II) Decarboxylation-vinylation reaction

2.1 g of sulfonamide obtained in the step (I) above was introduced into a flask equipped with a distillation head and the flask was heated under a pressure of 2.6 × $10^{-3}$ MPa at 230 °C to generate a vapor. The vapor was condensed and recovered as a distillate, thereby obtaining 0.4 g of a light yellow liquid. From the $^{19}$F-NMR and $^{1}$H-NMR spectra of the liquid, it was confirmed that the main component of the liquid was a perfluorovinyl ether represented by the following formula:

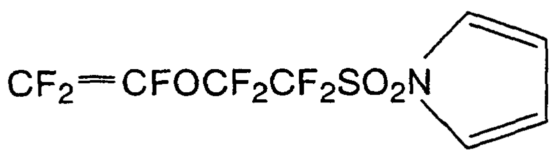

$^{19}$F-NMR: δ(ppm) -137(1F), -122(1F), -115.9(2F), -115(1F), -84.5(2F).
$^{1}$H-NMR: δ(ppm) 6.47(2H), 7.13(2H).

Further, it was also confirmed that the above-mentioned liquid contained a small amount of a proton-substituted product represented by the following formula:

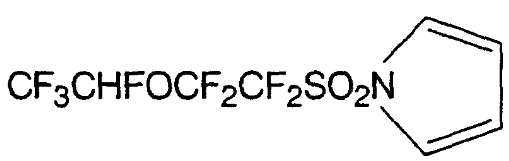

However, it was confirmed that the above-mentioned liquid contained no cyclization reaction product.

Example 7

(I) Neutralization reaction

Neutralization reaction was performed in substantially the same manner as in the step (I) of Example 1, except that 20.0 g of the compound represented by the following formula: $CF_3CF(COF)OCF_2CF_2CF_2SO_2F$ was used in stead of 103.8 g of the compound represented by the following formula: $CF_3CF(COF)OCF_2CF_2SO_2F$, thereby obtaining a white solid. As a result of the NMR and IR analyses, it was confirmed that the solid was a sodium carboxylate represented by the following formula: $CF_3CF(CO_2Na)OCF_2CF_2CF_2SO_2F$.

(II) Amidation reaction

Amidation reaction was performed in substantially the same manner as in the step (II) of Example 1, except that 15.0 g of the sodium carboxylate obtained in the step (I) above was used instead of 54.9 g of the sodium carboxylate obtained in the step (I) of Example 1, thereby obtaining a yellow solid. As a result of the NMR and IR analyses, it was confirmed that the solid was a sulfonamide represented by the following formula: $CF_3CF(CO_2Na)OCF_2CF_2CF_2SO_2N(C_2H_5)_2$.

(III) Decarboxylation-vinylation reaction

Decarboxylation-vinylation reaction was performed in substantially the same manner as in the step (III) of Example 1, except that 11.4 g of the sulfonamide obtained in the step (II) above was used instead of 20.4 g of the sulfonamide obtained in the step (II) of Example 1, thereby obtaining a light yellow liquid. As a result of the NMR and IR analyses, it was confirmed that the main component of the liquid was a perfluorovinyl ether represented by the following formula:

$$CF_2{=}CFOCF_2CF_2CF_2SO_2N(C_2H_5)_2.$$

Further, it was also confirmed that the above-mentioned liquid contained a small amount of a proton-substituted product represented by the following formula: $CF_3CHFOCF_2CF_2CF_2SO_2N(C_2H_5)_2$. However, it was confirmed that the above-mentioned solution contained no cyclization reaction product.

Example 8

(I) Amidation reaction

54.9 g of the sodium carboxylate obtained in the step (II) of Example 1 was dissolved in 150 ml of anhydrous THF to thereby obtain a solution. The obtained solution was cooled to 0 °C, and 150 ml of a (1M) solution of sodium hexamethyl disilazide in THF was dropwise added to the solution. The temperature of the resultant mixture was elevated to room temperature, followed by stirring for 12 hours to effect a reaction, thereby obtaining a reaction mixture. The obtained reaction mixture was subjected to filtration to thereby remove a precipitate which was formed during the reaction. Then, the solvent in the reaction mixture was distilled off under reduced pressure to thereby obtain a residue. The obtained residue was subjected to vacuum drying at 80 °C, thereby obtaining 67.5 g of a yellowish brown solid. As a result of the NMR and IR analyses, it was confirmed that the solid was a compound having a sulfonamide structure, and that the solid does not contain any unreacted sodium carboxylate.

(II) Decarboxylation-vinylation reaction

66 g of the compound obtained in step (I) above was dissolved in 300 ml of diglyme to thereby obtain a solution. The obtained solution was heated under a stream of nitrogen gas at 150 °C for 1 hour to effect a reaction, thereby obtaining a reaction mixture. From the $^{19}F$-NMR analysis of the reaction mixture, it was confirmed that the reaction mixture contained two different products each having a perfluorovinyl group present (both of which were not identified).

The solvent in the reaction mixture was distilled off under reduced pressure to thereby obtain a residue. To the obtained residue was added water, followed by addition of hydrochloric acid to thereby render acidic the resultant mixture. The obtained acidic mixture was subjected to extraction with HFC43-10mee. The solvent in the resultant extract solution was distilled off under reduced pressure to thereby obtain a residue. The obtained residue was subjected to distillation under reduced pressure of 1.3 × $10^{-3}$ MPa, so as to recover a fraction having a boiling point of from 130 to 133 °C, thereby obtaining 21.1 g of a slightly yellow liquid. From the $^{19}F$-NMR spectrum and GC-MS of the liquid, it was confirmed that the main component of the liquid was a perfluorovinyl ether represented by the following formula:

$$CF_2=CFOCF_2CF_2SO_2NH_2.$$

$^{19}F$-NMR: δ (ppm) -137 (1F), -124 (1F), -118.6 (2F), -116 (1F), -84.8 (2F).

EI-MS: m/z 180, 100, 97, 81, 80, 64, 16

Further, it was also confirmed that the above-mentioned liquid contained a small amount of a proton-substituted product represented by the following formula: $CF_3CHFOCF_2CF_2SO_2NH_2$. However, it was confirmed that the above-mentioned liquid contained no cyclization reaction product.

Example 9

Substantially the same procedures as in the steps (I) and (II) of Example 8 were repeated, except that the distillation of the residue (obtained by distilling off the solvent from the extract solution) under reduced pressure of 1.3 × $10^{-3}$ MPa was not performed in the step (II). The main component of the obtained residue was the same perfluorovinyl ether as obtained in Example 8. However, the residue contained impurities, such as the proton-substituted product mentioned in Example 8.

70 g of hexamethyl disilazane was added to 44 g of the above-mentioned residue to effect a reaction at 100 °C for 2 hours to thereby obtain a reaction mixture. From the obtained reaction mixture, the unreacted hexamethyl disilazane was distilled off to thereby obtain a residue. The obtained residue was subjected to distillation under reduced pressure of 3.9 × $10^{-4}$ MPa, so as to recover a fraction having a boiling point of from 115 to 118 °C, thereby obtaining a light yellow liquid. From the $^{19}F$-NMR and GC-MS of the liquid, it was confirmed that the liquid was a perfluorovinyl ether represented by the following formula: $CF_2=CFOCF_2CF_2SO_2NHSiMe_3$.

$^{19}F$-NMR: δ (ppm) -136 (1F), -123.5 (1F), -117.6 (2F), -116 (1F), -84.0 (2F).

Example 10

(I) Synthesis of $CF_3CHFOCF_2CF_2SO_2F$

135 g of sodium carbonate and 500 ml of diglyme were mixed together to obtain a slurry. The obtained slurry was charged into a flask equipped with a distillation head. 400 g of a compound represented by the following formula (which is the same compound as that used in the step (I) of Example 1):

$$CF_3CF(COF)OCF_2CF_2SO_2F$$

was dropwise added to the slurry at room temperature, and the resultant mixture was stirred at room temperature for 1 hour, and then at 40 °C for 1 hour, to effect a reaction, thereby obtaining a reaction mixture. 21 ml of water was added to the obtained reaction mixture, and the resultant mixture was heated to 100 °C to generate a vapor. The vapor was condensed and recovered as a distillate, and the distillate was washed and dried, thereby obtaining 176 g of a colorless liquid. From the $^{19}F$-NMR spectrum of the liquid, it was confirmed that the liquid was a compound represented by the following formula: $CF_3CHFOCF_2CF_2SO_2F$.

$^{19}F$-NMR: δ(ppm) -147.9 (1F), -114.2 (2F), -86.5 (3F), -87.0, -84.6 (2F), 42.7 (1F).

(II) Amidation reaction

25 g of a dispersion of sodium hydride in a mineral oil (sodium hydride content: 60 %) was washed with n-hexane under a stream of nitrogen gas to thereby remove the mineral oil and obtain a sodium hydride powder. 300 ml of anhydrous dimethoxyethane was added to the sodium hydride powder, and the resultant mixture was cooled to 0 °C. To the resultant mixture was dropwise added a solution obtained by dissolving 38.5 g of imidazole in 200 ml of dimethoxyethane. The temperature of the resultant mixture was then elevated to room temperature, followed by stirring for 1 hour, thereby obtaining an imidazole sodium amide solution.

The obtained solution was cooled to 0 °C, followed by dropwise addition of 170 g of the compound obtained

in the step (I) above. The temperature of the resultant mixture was elevated to room temperature, followed by stirring at room temperature for 12 hours, to effect a reaction, thereby obtaining a reaction mixture. A small amount of water was added to the obtained reaction mixture, and dimethoxyethane in the reaction mixture was distilled off under reduced pressure to obtain a residue. A small amount of water was added to the obtained residue, and extracted with HFC43-10mee to obtain an extract solution. The obtained extract solution was washed with diluted aqueous NaOH solution and dried. Then, the solvent was distilled off from the extract solution, and the resultant residue was subjected to distillation under a reduced pressure of $3.9 \times 10^{-4}$ MPa to recover a fraction having a boiling point of from 64 to 66 °C, thereby obtaining 112 g of a colorless liquid. From the $^{19}$F-NMR spectrum and GC-MS of the liquid, it was confirmed that the liquid was a sulfonamide represented by the following formula:

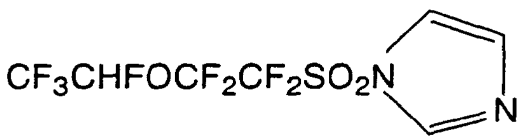

$^{19}$F-NMR: δ(ppm) -148.0 (1F), -115.5 (2F), -86.0 (3F), -84.5, -83.0 (2F).

(III) Vinylation reaction (dehydrofluorination reaction)

145 ml of hexamethyldisilazane was dissolved in 500 ml of anhydrous THF to obtain a solution. The obtained solution was cooled to -78 °C. To the resultant solution was dropwise added 431 ml of a (1.6 M) solution of n-BuLi in n-hexane under a stream of nitrogen gas, and the resultant mixture was stirred at -78 °C for 30 minutes, to obtain a lithium hexamethyldisilazide solution.

The temperature of the obtained solution was elevated to 0 °C. 104.4 g of the sulfonamide obtained in the step (II) above was dissolved in 200 ml of THF, and the resultant solution was dropwise added to the lithium hexamethyldisilazide solution to obtain a mixture. The obtained mixture was stirred at 0 °C for 1 hour to effect a reaction, thereby obtaining a reaction mixture.

A small amount of water was added to the obtained reaction mixture, and THF was distilled off from the reaction mixture. To the resultant residue was added small amounts of water and HFC43-10mee to obtain a mixture. The obtained mixture was subjected to filtration to remove insoluble, to obtain a filtrate. The organic phase of the filtrate was dried and the solvent was distilled off from the filtrate, to thereby obtain a residual liquid. The residual liquid was subjected to distillation under a reduced pressure of $3.9 \times 10^{-4}$ MPa, and a fraction having a boiling point of from 60 to 62 °C was recovered, thereby obtaining 57.6 g of a colorless liquid. From the $^{19}$F-NMR spectrum and GC-MS of the liquid, it was confirmed that the liquid was a perfluorovinyl ether represented by the following formula:

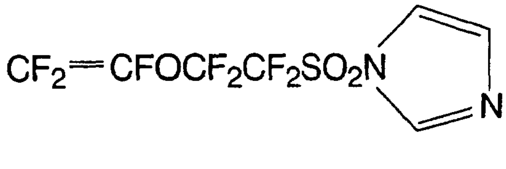

$^{19}$F-NMR: δ(ppm)-137.8(1F), -123.0(1F), -115.7(2F), -115.5(1F), -84.5(2F).

Example 11

Substantially the same procedure as in Example 10 was repeated, except that 96 ml of diisopropylamine was used instead of 145 ml of hexamethyl disilazane, namely lithium diisopropylamide was used instead of lithium di-hexamehyldisilazide. As a result, the same perfluorovinyl ether as obtained in Example 10 was obtained.

Example 12

0.7 g of the mixture of the perfluorovinyl ether and the proton-substituted product (perfluorovinyl ether : proton-substituted product = 89 : 11), which mixture was obtained in Example 3, was dissolved in 10 ml of THF, to thereby obtain a solution. To the obtained solution was dropwise added 1.5 ml of a (1M) solution of sodium hexamethyldisilazide in THF under a stream of nitrogen gas at 0 °C. The temperature of the resultant mixture was elevated to room temperature, and the resultant mixture was stirred for 12 hours to effect a reaction, thereby obtaining a reaction mixture.
By the GC analysis of the obtained reaction mixture, it was confirmed that the proton-substituted product had disappeared from the obtained reaction mixture. Further, by the $^{19}$F-NMR analysis, it has been confirmed that the obtained reaction mixture contained only the perfluorovinyl ether.

Example 13

A polymerization reaction was performed in substantially the same manner as in Example 2, except that 15 g of the perfluorovinyl ether obtained in Example 5 was used instead of 7.5 g of the perfluorovinyl ether obtained in Example 1, thereby obtaining a reaction mixture.
Methanol was added to the reaction mixture to obtain a precipitate. The obtained precipitate was recovered, and then washed and dried to thereby obtain 1.5 g of a white solid.
From the $^{19}$F-NMR spectrum of the solid, it was confirmed that the solid was a copolymer comprising a monomer unit (a sulfonamide unit) which was derived from the perfluorovinyl ether obtained in Example 5 and monomer unit (a TFE unit) which was derived from TFE. Further, it was also confirmed that the molar ratio of the sulfonamide unit to the TFE unit was 1 : 4.

Example 14

206 g of hexamethyldisilazane was dissolved in 700 ml of anhydrous THF to obtain a solution. The obtained solution was cooled to -78 °C. To the obtained solution was dropwise added 800 ml of a (1.6 M) solution of n-BuLi in n-hexane under a stream of nitrogen gas, and the resultant mixture was stirred at -78 °C for 30 minutes, to obtain a lithium hexamethyldisilazide solution.
The temperature of the obtained solution was elevated to 0 °C. 170 g of the compound obtained in the step (I) of Example 10 was dropwise added to the solution to obtain a mixture. The obtained mixture was stirred at 0 °C for 1 hour, thereby obtaining a reaction mixture.
A small amount of water was added to the obtained reaction mixture, and THF was distilled off from the reaction mixture. A diluted hydrochloric acid was added to the resultant residual liquid to obtain an acidic solution, and the acidic solution was extracted with HFC43-10mee to thereby obtain an extract solution. The obtained extract solution was dried and the solvent was distilled off from the dried extract. The resultant residue was subjected to distillation under a reduced pressure of 0.4 kPa, and a fraction having a boiling point of 87 °C was recovered, thereby obtaining 115 g of a colorless liquid. The $^{19}$F-NMR spectrum of the liquid was identical to the $^{19}$F-NMR spectrum of the perfluorovinyl ether obtained in Example 8. Further, the liquid contained no proton-substituted product which was mentioned in Example 8.

Example 15

A polymerization reaction was performed in substantially the same manner as in Example 2, except that 8 g of the perfluorovinyl ether obtained in Example 14 was used instead of 7.5 g of the perfluorovinyl ether obtained in Example 1, thereby obtaining a reaction mixture.
Methanol was added to the obtained reaction mixture, followed by removal of the solvent and the unreacted monomer by distillation under reduced pressure to recover a precipitate formed by the addition of the methanol. The obtained precipitate was washed and dried, thereby obtaining 0.8 g of a light brown solid.
From the $^{19}$F-NMR spectrum of the solid, it was confirmed that the solid was a copolymer comprising a monomer unit (a sulfonamide unit) which was derived from the perfluorovinyl ether obtained in Example 14 and monomer unit (a TFE unit) which was derived from TFE. Further, it was also confirmed that the molar ratio of the sulfonamide unit to TFE unit was 1 : 4.

Example 16

(I) Production of a terpolymer film

16 g of a monomer (hereinafter, referred to as "$SO_2F$ monomer") represented by the following formula:

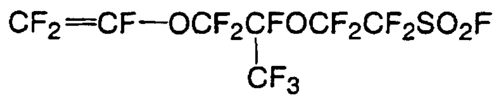

4 g of the perfluorovinyl ether monomer obtained in Example 14, 40 g of HFC43-10mee and 0.85 g of a 5 % $(CF_3CF_2CF_2COO)_2$ solution in HFC43-10mee (wherein the $(CF_3CF_2CF_2COO)_2$ is a polymerization initiator) were introduced into a 200 ml pressure resistant vessel which was made of a stainless steel and which was equipped with a gas introduction pipe. The internal atmosphere of the pressure resistant vessel was fully purged with nitrogen gas. Tetrafluoroethylene (TFE) was introduced into the pressure resistant vessel through the gas introduction pipe so that the internal pressure of the pressure resistant vessel was elevated to 0.3 MPa. Then, a reaction was performed at 25 °C for 4.5 hours while stirring and appropriately introducing TFE so as to maintain the internal pressure of the pressure resistant vessel at 0.3 MPa.

Thereafter, the introduction of TFE was stopped and the internal pressure of the pressure resistant vessel was lowered to atmospheric pressure, to obtain a reaction mixture (a white gel). Methanol was added to the obtained reaction mixture to precipitate a solid. The solid was recovered by filtration, followed by washing and subsequent drying, to obtain 6.5 g of a white solid.

From the $^{19}F$-NMR spectrum of the solid, it was confirmed that the solid was a terpolymer comprising a monomer unit (a sulfonamide unit) derived from the perfluorovinyl ether obtained in Example 14, a monomer unit (an $SO_2F$ unit) derived from the above-mentioned $SO_2F$ monomer, and a monomer unit (a TFE unit) derived from TFE. It was also confirmed that the ratio between sulfonamide unit, $SO_2F$ unit and TFE unit (sulfonamide unit : $SO_2F$ unit : TFE unit molar ratio) was 0.2 : 1.0 : 5.3. In addition, it was confirmed that peaks (at 3391, 3306 and 1544 $cm^{-1}$) ascribed to a sulfonamido group were observed in the IR spectrum of the solid.

The above-mentioned terpolymer was subjected to a press molding at 270 °C, thereby obtaining a colorless transparent terpolymer film (film thickness: 82 μm).

(II) Production of a solid polymer electrolyte membrane

The terpolymer film obtained in the step (I) above was immersed in a dioxane solution of triethylamine (dioxane: triethylamine = 5 : 3 (volume ratio)), and the terpolymer film in the solution was heated under reflux conditions for 3 hours, followed by washing and drying (hereinafter, this treatment is referred to as a "modification treatment"), thereby obtaining a modified terpolymer film.

Potassium hydroxide (KOH) was dissolved in a mixed solvent comprising dimethylsulfoxide (DMSO) and water to obtain a solution (KOH : DMSO : water = 3 : 6 : 11 (weight ratio)). The modified terpolymer film obtained above was immersed in the solution at 90 °C for 1 hour, followed by water washing and drying. In the IR spectrum of the film, a peak ascribed to a bissulfonylimido group was observed at 1350 $cm^{-1}$.

The film was immersed in 4N sulfuric acid at 90 °C for 1 hour, followed by water washing and drying, thereby obtaining a solid polymer electrolyte membrane.

The solid polymer electrolyte membrane exhibited a tensile modulus of $1.2 \times 10^8$ dyn/$cm^2$ at 150 °C and a tensile modulus of $6.3 \times 10^7$ dyn/$cm^2$ at 300 °C.

Example 17

(I) Production of a terpolymer film

A polymerization reaction was performed in substantially the same manner as in the step (I) of Example 16, except that the amounts of the $SO_2F$ monomer and perfluorovinyl ether monomer were changed to 22.1 g and 0.14 g, respectively, thereby obtaining 6.6 g of a white solid.

From the $^{19}F$-NMR spectrum of the solid, it was confirmed that the solid contained an $SO_2F$ unit and a TFE unit, and that the molar ratio of the $SO_2F$ unit to the TFE unit was 1.0 : 4.5.

In addition, from the IR spectrum of the solid, it was confirmed that the solid contained a sulfonamide unit,

and that the amount of the sulfonamide unit was 0.8 mol%, based on the total molar amount of the sulfonamide unit, $SO_2F$ unit and TFE unit.

Thus, it was confirmed that the obtained solid was a terpolymer comprising a sulfonamide unit, an $SO_2F$ unit and a TFE unit.

The terpolymer exhibited a melt index of 3.8, as measured by using, as a sample, 11.3 g of a terpolymer obtained by repeating the above-mentioned copolymerization reaction in a scale four times larger than mentioned above.

The above-mentioned terpolymer was subjected to a press molding at 270 °C, thereby obtaining a colorless transparent terpolymer film (film thickness: 83 μm).

(II) Production of a solid polymer electrolyte membrane

A solid polymer electrolyte membrane was produced in substantially the same manner as in the step (II) of Example 16, except that the terpolymer film obtained in the step (I) above was used instead of the terpolymer film obtained in the step (I) of Example 16.

The solid polymer electrolyte membrane exhibited a tensile modulus of $8.6 \times 10^7$ dyn/cm$^2$ at 150 °C and a tensile modulus of $2.0 \times 10^7$ dyn/cm$^2$ at 300 °C.

Comparative Example 2

A solid polymer electrolyte membrane was produced in substantially the same manner as in the step (II) of Example 16, except that a binary polymer film (film thickness: 48 μm) produced from the binary polymer (k : l = 5 : 1) represented by the following formula (36):

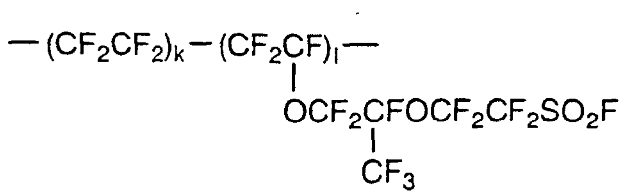

(36)

was used instead of the terpolymer film obtained in the step (I) of Example 16, and that the binarypolymer film was not subjected to modification treatment.

The obtained solid polymer electrolyte membrane exhibited a proton conduction ratio of 0.101 S/cm.

The solid polymer electrolyte membrane exhibited a tensile modulus of $3.1 \times 10^7$ dyn/cm$^2$ at 150 °C. However, since the solid polymer electrolyte membrane was melted at about 180 °C, the tensile modulus thereof at 300 °C could not be measured.

Further, another solid polymer electrolyte membrane was produced in substantially the same manner as in the step (II) of Example 16, except that the above-mentioned binarypolymer film was used instead of the terpolymer film obtained in the step (I) of Example 16.

The obtained solid polymer electrolyte membrane exhibited a tensile modulus of $3.1 \times 10^7$ dyn/cm$^2$ at 150 °C. However, since this solid polymer electrolyte membrane was also melted at about 180 °C, the tensile modulus thereof at 300 °C could not be measured.

Comparative Example 3

A solid polymer electrolyte membrane was produced in substantially the same manner as in the step (II) of Example 16, except that the terpolymer film was not subjected to modification treatment.

The obtained solid polymer electrolyte membrane exhibited a tensile modulus of $3.1 \times 10^7$ dyn/cm$^2$ at 150 °C. However, since the solid polymer electrolyte membrane was melted at about 200 °C, the tensile modulus thereof at 300 °C could not be measured.

Example 18

(I) Bromination reaction

54 g of a sulfonyl fluoride represented by the following formula (37):

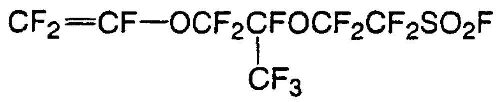

(37)

was dissolved in 40 ml of HFC43-10mee to obtain a solution. Bromine was dropwise added to the obtained solution while stirring at room temperature until the color of bromine did not disappear (12 g of bromine was added in total), to obtain a mixture. The obtained mixture was stirred at room temperature for 1 hour to effect a reaction, thereby obtaining a reaction mixture.

From the obtained reaction mixture, unreacted bromine and the solvent were distilled off, and the resultant residue was subjected to distillation under a reduced pressure of $6.7 \times 10^3$ Pa, and a fraction having a boiling point of 110 °C was recovered to thereby obtain 67 g of a liquid. From the $^{19}$F-NMR spectrum of the liquid, it was confirmed that the liquid was a bromine-added product represented by the following formula (38):

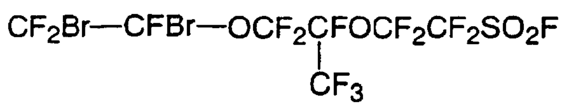

(38).

$^{19}$F-NMR: δ(ppm)-146.6(1F), -114.0(2F), -87.5, -83.6(2F), -81.5(3F), -81(2F), -73(1F), -65.0(2F), 43.4(1F).

(II) Amidation reaction

40 of the bromine-added product (38) obtained in the step (I) above was dissolved in 30 ml of glyme to obtain a solution. 20 g of diethylamine was dropwise added to the obtained solution at room temperature, and a reaction was performed at 50 °C for 5 hours, thereby obtaining a reaction mixture.

The obtained reaction mixture was pored into water and extracted with HFC43-10mee to obtain an extract solution. The obtained extract solution was washed with diluted hydrochloric acid, and then, the solvent was then distilled from the extract solution, thereby obtaining 41 g of a liquid. From the $^{19}$F-NMR spectrum of the liquid, it was confirmed that the liquid was a sulfonamide represented by the following formula (39):

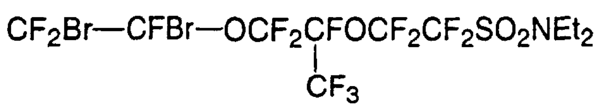

(39).

$^{19}$F-NMR: δ(ppm)-146.6(1F), -117.4(2F), -87, -83(2F), -81.2(3F), -80.6(2F), -72.7(1F), -64.8(2F).

(III) Vinylation reaction (dehalogenation reaction)

40 g of the sulfonamide (39) obtained in the step (II) above was dissolved in 160 g of dimethylformamide to obtain a solution. To the obtained solution was added 6.1 g of a zinc powder (which had been washed with diluted hydrochloric acid, followed by drying), and a reaction was performed at 80 °C for 2.5 hours to thereby obtain a reaction mixture.

The obtained reaction mixture was poured into water and extracted with HFC43-10mee to obtain an extract solution. The solvent was distilled off from the extract solution, and the resultant residue was subjected to distillation under a reduced pressure of $4.0 \times 10^2$ Pa, and a fraction having a boiling point of 128 °C was recovered to thereby obtain 15 g of a liquid. From the $^{19}$F-NMR spectrum of the liquid, it was confirmed that the liquid was a perfluorovinyl ether represented by the following formula (40):

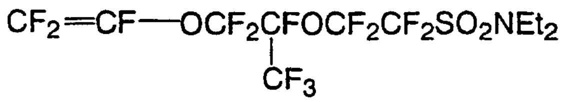 (40).

$^{19}$F-NMR: δ(ppm)-146.5(1F), -137.9(1F), -124.1(1F), -117.9(2F), -116.5(1F), 86.7(2F), -81.8(3F), -80.7(2F).

Example 19

7.5 g of the perfluorovinyl ether monomer (40) obtained in Example 18 (which had been purified by redistillation), 22 g of HFC43-10mee and 2.2 g of a 5 % $(CF_3CF_2CF_2COO)_2$ solution in HFC43-10mee (wherein the $(CF_3CF_2CF_2COO)_2$ is a polymerization initiator) were introduced into a 200 ml pressure resistant vessel which was made of a stainless steel and which was equipped with a gas introduction pipe. The internal atmosphere of the pressure resistant vessel was fully purged with nitrogen gas. Tetrafluoroethylene (TFE) was introduced into the pressure resistant vessel through the gas introduction pipe so that the internal pressure of the pressure resistant vessel was elevated to 0.4 MPa. Then, a reaction was performed at 25 °C for 3.5 hours while stirring and appropriately introducing TFE so as to maintain the internal pressure of the pressure resistant vessel at 0.4 MPa.

Thereafter, the introduction of TFE was stopped and the internal pressure of the pressure resistant vessel was lowered to atmospheric pressure, to obtain a reaction mixture (a white turbid liquid). Methanol was added to the obtained reaction mixture to precipitate a solid. The solid was recovered by filtration, followed by washing with methanol and subsequent drying, to obtain 1.2 g of a white solid.

From the $^{19}$F-NMR spectrum of the solid, it was confirmed that the solid was a copolymer comprising a monomer unit (a sulfonamide unit) which was derived from the perfluorovinyl ether monomer (40) obtained in Example 18, and a monomer unit (a TFE unit) which was derived from TFE. It was also confirmed that the molar ratio of the sulfonamide unit to the TFE unit was 1 : 4.

The above-mentioned copolymer was subjected to a press molding at 250 °C, thereby obtaining a copolymer film.

Comparative Example 4

3 g of the sulfonyl fluoride (37) used in the step (I) of Example 18 was dissolved in 3 ml of glyme to obtain a solution. 2 g of diethylamine was dropwise added to the obtained solution at room temperature. As a result, an exothermic reaction occurred, and a reaction mixture comprising a solid (i.e., an insoluble) was obtained.

The obtained reaction mixture was analyzed by gas chromatography (GC). The results of the analysis showed that the obtained reaction mixture was a complicated mixture and that the reaction mixture contained only a trace amount of the desired perfluorovinl ether (i.e., the perfluorovinyl ether (40) obtained in Example 18).

Example 20

(I) Chlorination reaction

14 g of a sulfonyl fluoride represented by the following formula:

$$CF_2=CFOCF_2CF_2SO_2F$$

was dissolved in 40 ml of HFC43-10mee to obtain a solution. The obtained solution was charged into a reaction vessel equipped with a gas introduction tube, and the solution was stirred at room temperature while introducing chlorine gas into the reaction vessel to thereby effect a reaction. During the reaction, the contents of the reaction vessel were analyzed by gas chromatography (GC), and a peak ascribed to the sulfonyl fluoride was observed. The reaction was continued until the peak ascribed to the sulfonyl fluoride disappeared.

The solvent in the resultant reaction mixture was distilled off, and the remainder of the reaction mixture was subjected to vacuum distillation, thereby obtaining 15 g of a liquid. Since the $^{19}$F-NMR spectrum of the liquid was in agreement with the data described in J. Fluorine Chem., 58, 59 (1992) (the Netherlands), it was confirmed that the liquid was a chlorine addition product represented by the following formula:

$$CF_2ClCFClOCF_2CF_2SO_2F.$$

(II) Amidation reaction

13 g of the chlorine addition product obtained in the step (I) above was dissolved in 15 ml of glyme to obtain a solution. 7 g of diethylamine was dropwise added to the obtained solution at room temperature, and a reaction was performed at 50 °C for 5 hours, thereby obtaining a reaction mixture.

The obtained reaction mixture was poured into water and extracted with HFC43-10mee to obtain an extract solution. The obtained extract solution was washed with diluted hydrochloric acid, and the solvent in the extract solution was then distilled off, thereby obtaining 15 g of a liquid. As a result of the $^{19}F$-NMR and IR analyses, it was confirmed that the liquid was a sulfonamide represented by the following formula:

$$CF_2ClCFClOCF_2CF_2SO_2N(C_2H_5)_2.$$

(III) Vinylation reaction (dehalogenation reaction)

15 g of the sulfonamide obtained in the step (II) above was dissolved in 60 g of dimethylformamide to obtain a solution. A zinc powder was washed with diluted hydrochloric acid and then dried, and 3.1 g of the thus treated zinc powder was added to the solution obtained above, and a reaction was performed at 80 °C for 2.5 hours to thereby obtain a reaction mixture.

The obtained reaction mixture was poured into water and extracted with HFC43-10mee to obtain an extract solution. The solvent in the obtained extract solution was distilled off, and the remainder of the extract solution was subjected to vacuum distillation under a pressure of $4.0 \times 10^3$ Pa, and a fraction having a boiling point of 130 °C was recovered, thereby obtaining 7.5 g of a liquid. From the $^{19}F$-NMR spectrum of the liquid, it was confirmed that the liquid was a perfluorovinyl ether represented by the following formula:

$$CF_2=CFOCF_2CF_2SO_2N(C_2H_5)_2.$$

$^{19}F$-NMR: δ(ppm) -137 (1F), -124 (1F), -117.6 (2F), -116 (1F), -85.3 (2F).

Reference Example 1

2.5 g of a dispersion of sodium hydride in a mineral oil (sodium hydride content: 60 %) was washed with n-hexane under a stream of nitrogen gas to thereby remove the mineral oil and obtain a sodium hydride powder. 30 ml of anhydrous dimethoxyethane was added to the obtained sodium hydride powder to obtain a mixture. The obtained mixture was cooled to 0 °C. 3.9 g of imidazole was dissolved in 20 ml of dimethoxyethane to obtain a solution. The obtained solution was dropwise added to the above-mentioned mixture. The temperature of the resultant mixture was elevated to room temperature, and the mixture was stirred for 1 hour, thereby obtaining an imidazole sodium amide solution.

On the other hand, 1 g of a powder of the copolymer (36) used in Comparative Example 2 was dispersed in anhydrous dioxane to obtain a dispersion. The imidazole sodium amide solution obtained above was dropwise added to the dispersion at room temperature, and the resultant mixture was stirred, first at room temperature for 4 hours and then at 70 °C for 8 hours, to effect a reaction, thereby obtaining a reaction mixture.

The obtained reaction mixture was subjected to filtration to recover the solid in the reaction mixture. The recovered solid was washed, firstly with water, secondly with dimethoxyethane, and finally with HFC43-10mee, and the solid was then dried.

From a comparison between the IR spectrum of the above-mentioned copolymer (36) and the IR spectrum of the obtained solid, it was found that a peak ascribed to an $SO_2F$ group was observed in the former, but not in the latter. In the IR spectrum of the solid, a peak ascribed to a sulfonamide group was observed, instead of a peak ascribed to an $SO_2F$ group. From these results, it was confirmed that, in the solid, the $SO_2F$ groups of the copolymer (36) were converted into sulfonamide groups through an amidation reaction with imidazole. Hereinafter, the solid is referred to as "amidated copolymer".

The amidated copolymer was subjected to a press molding at 280 °C, to obtain a copolymer film.

From the obtained copolymer film was cut out a square sample having a size of 3 cm × 3cm. The square sample was immersed in 30 ml of 3N sulfuric acid at 90 °C for 1 hour, and the square sample was then washed with

water and dried, thereby obtaining a solid polymer electrolyte membrane.

The IR spectrum of the obtained solid polymer electrolyte membrane was identical to the IR spectrum of the solid polymer electrolyte membrane obtained in Comparative Example 2. Further, in the IR spectrum of the obtained solid polymer electrolyte membrane, no peaks ascribed to amido groups were observed. From these results, it was confirmed that, in the obtained solid polymer electrolyte membrane, sulfonamido groups were converted into free sulfonic acid groups.

On the other hand, a binary copolymer film formed from the copolymer (36) (which was the same as used in Comparative Example 2) was immersed in 30 ml of 3N sulfuric acid at 90 °C for 1 hour, followed by water washing and drying, in substantially the same manner as mentioned above. However, no change was observed in the $SO_2F$ groups of the binary copolymer film.

Example 21

(I) Amidation reaction

The imidazole sodium amide solution obtained by the method described in Reference Example 1 was cooled to 0 °C. 10 g of the bromine addition product (38) obtained in the step (I) of Example 18 was dissolved in 20 ml of dimethoxyethane to obtain a solution. The obtained solution was dropwise added to the imidazole sodium amide solution to obtain a mixture. The temperature of the obtained mixture was elevated to room temperature, and the mixture was stirred at room temperature for 12 hours to effect a reaction, thereby obtaining a reaction mixture. A small amount of water was added to the obtained reaction mixture, and the dimethoxyethane was distilled off under reduced pressure to obtain a residual liquid. A small amount of water was added to the residual liquid, and the resultant mixture was extracted with HFC43-10mee to obtain an extract solution. The obtained extract solution was washed with water and dried, and then the solvent in the extract solution was distilled off, thereby obtaining 11 g of a liquid. From the $^{19}F$-NMR spectrum and GC-MS of the liquid, it was confirmed that the liquid was a sulfonamide represented by the following formula (41):

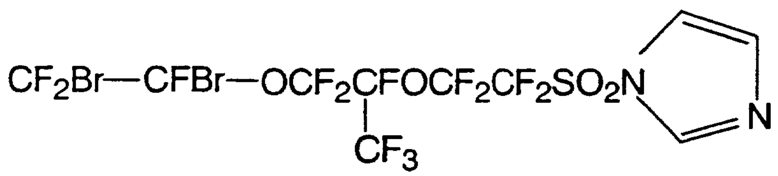

(41).

$^{19}F$-NMR: δ(ppm) -147 (1F), -117 (2F), -87, -83 (2F), -81 (3F), -80.5 (2F), -73 (1F), -65 (2F).

(II) Vinylation reaction (dehalogenation reaction)

10 g of the sulfonamide (41) obtained in the step (II) above was dissolved in 40 g of dimethylformamide to obtain a solution. A zinc powder was washed with diluted hydrochloric acid and then dried, and 1.5 g of the thus treated zinc powder was added to the solution obtained above, and a reaction was performed at 80 °C for 2.5 hours to thereby obtain a reaction mixture.

The obtained reaction mixture was poured into water and extracted with HFC43-10mee to obtain an extract solution. The solvent in the obtained extract solution was distilled off, and the remainder of the extract solution was subjected to vacuum distillation under a pressure of $4.0 \times 10^2$ Pa, and a fraction having a boiling point of from 140 to 150 °C was recovered, thereby obtaining 6 g of a liquid. From the $^{19}F$-NMR spectrum of the liquid, it was confirmed that the liquid was a perfluorovinyl ether represented by the following formula (42):

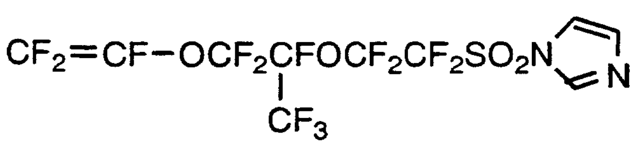

(42).

$^{19}F$-NMR: δ(ppm) -147 (1F), -138 (1F), -124 (1F), -118 (2F), -116.5 (1F), 86.5 (2F), -82 (3F), -81 (2F).

Example 22

(I) Neutralization reaction

10.6 g of sodium carbonate and 50 ml of acetonitrile were mixed together to obtain a slurry. 51.2 g of a compound represented by the following formula (43):

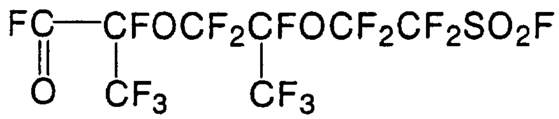

(43)

was dropwise added to the obtained slurry under a stream of nitrogen gas at room temperature. The resultant mixture was stirred at room temperature for 1 hour and then stirred at 40 °C for 1 hour to effect a reaction, thereby obtaining a reaction mixture. The obtained mixture was subjected to filtration to thereby remove a precipitate which was formed during the reaction. Then, the solvent in the reaction mixture was distilled off under reduced pressure, thereby obtaining 53.0 g of a white solid. As a result of the NMR and IR analyses, it was confirmed that the solid was a compound represented by the following formula (44):

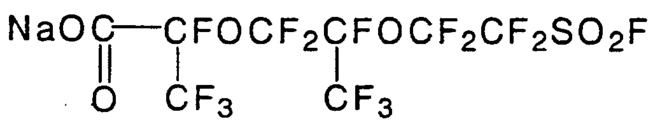

(44).

(II) Amidation reaction

47.9 g of the sodium carboxylate obtained in the step (I) above was dissolved in 100 ml of anhydrous THF to obtain a solution. The obtained solution was cooled to 0 °C. 90 ml of a (1M) solution of sodium hexamethyldisilazide in THF was dropwise added to the solution to thereby obtain a mixture. The temperature of the obtained mixture was elevated to room temperature, and the mixture was stirred at room temperature for 12 hours to effect a reaction, thereby obtaining a reaction mixture. The obtained reaction mixture was subjected to filtration to thereby remove a precipitate which was formed during the reaction. Then, the solvent in the reaction mixture was distilled off under reduced pressure to thereby obtain a residue. The residue was subjected to vacuum drying at 80 °C, thereby obtaining 55.4 g of a yellow brown solid. As a result of the NMR and IR analyses, it was confirmed that the solid was a compound having a sulfonamide structure. Further, it was confirmed that the solid contained no sodium carboxylate which was the starting material of the above-mentioned amidation reaction.

(III) Decarboxylation-vinylation reaction

50 g of the sulfonamide obtained in the step (II) above was dissolved in 200 ml of diglyme to obtain a solution. The obtained solution was heated at 150 °C for 1 hour under a stream of nitrogen gas to effect a reaction, thereby obtaining a reaction mixture. From the $^{19}$F-NMR spectrum of the obtained reaction mixture, it was confirmed that the reaction mixture contained 2 different products having perfluorovinyl groups (both of which were unidentified).

The solvent in the reaction mixture was distilled off under reduced pressure to obtain a residual liquid. Water was added to the residual liquid, and hydrochloric acid was added thereto, thereby obtaining an acidic liquid. The acidic liquid was extracted with HFC43-10mee to obtain an extract solution. The solvent in the obtained extract solution was distilled off under reduced pressure to thereby obtain a residual liquid. The obtained residual liquid was subjected to a vacuum distillation under a pressure of $1.3 \times 10^{-3}$ MPa, and a fraction having a boiling point of from 155 to 160 °C was recovered, thereby obtaining 24.5 g of a slightly yellowish liquid. From the $^{19}$F-NMR spectrum and GC-MS of the liquid, it was confirmed that the liquid was a perfluorovinyl ether represented by the following formula (45):

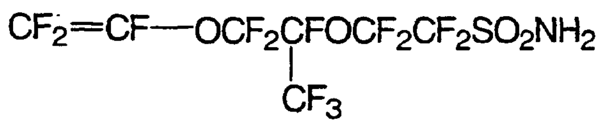 (45).

$^{19}$F-NMR: δ(ppm) -146.5 (1F), -137 (1F), -124 (1F), -118.9 (2F), -116 (1F), -86.4 (2F), -81.5 (3F), -80.5 (2F).

Example 23

30 g of hexamethyldisilazane was added to 10 g of the perfluorovinyl ether (45) obtained in Example 22, and a reaction was performed at 100 °C for 2 hours, thereby obtaining a reaction mixture. The unreacted hexamethyldisilazane in the reaction mixture was distilled off to obtain a residual liquid. The residual liquid was subjected to a vacuum distillation under a pressure of $3.9 \times 10^{-4}$ MPa, and a fraction having a boiling point of from 150 to 155 °C was recovered, thereby obtaining 6.2 g of a pale yellow liquid. From the $^{19}$F-NMR spectrum and GC-MS of the liquid, it was confirmed that the liquid was a perfluorovinyl ether represented by the following formula (46):

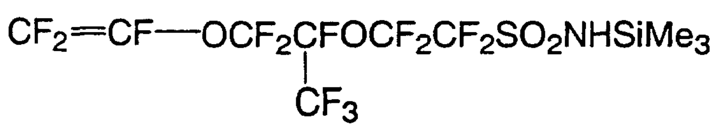 (46).

$^{19}$F-NMR: δ(ppm) -146.8 (1F), -136 (1F), -123.5 (1F), -117.9 (2F), -116 (1F), -86.0 (2F), -81.3 (3F), -80.2 (2F).

Example 24

104 ml of hexamethyldisilazane was dissolved in 500 ml of anhydrous THF to obtain a solution. The solution was cooled to -78 °C. 308 ml of a (1.6 M) solution of BuLi in n-hexane was dropwise added to the above-mentioned solution under a stream of nitrogen gas, and the resultant mixture was stirred at -78 °C for 30 minutes, thereby obtaining a lithium hexamethyldisilazide solution.

The temperature of the obtained solution was elevated to 0 °C, and 200 g of the sulfonylfluoride (37) used in the step (I) of Example 18 was dropwise added to the solution. The resultant mixture was stirred at room temperature for 2 hours to effect a reaction, thereby obtaining a reaction mixture.

A small amount of water was added to the obtained reaction mixture, and THF in the reaction mixture was distilled off to obtain a residual liquid. Diluted hydrochloric acid was added to the residual liquid to obtain an acidic liquid, and the acidic liquid was extracted with HFC43-10mee to thereby obtain an extract solution. The extract solution was dried, and the solvent in the extract solution was distilled off. The resultant residue was subjected to a vacuum distillation under a pressure of 0.4 kPa, and a fraction having a boiling point of 118 °C was recovered, thereby obtaining 170 g of a perfluorovinyl ether which was the same as the perfluorovinyl ether (45) obtained in Example 22, except that the perfluorovinyl ether obtained in this Example 24 was a colorless liquid.

Example 25

A reaction was performed in substantially the same manner as in Example 19, except that 10 g of the perfluoroviyl ether (45) obtained in Example 24 was used instead of 7.5 g of the perfluorovinyl ether (40) obtained in Example 18, thereby obtaining 1.2 g of a white solid.

From the $^{19}$F-NMR of the solid, it was confirmed that the solid was a copolymer comprising a monomer unit (a sulfonamide unit) which was derived from the perfluorovinyl ether (45) and a monomer unit (a TFE unit) which was derived from TFE. It was also confirmed that the ratio between sulfonamide unit and TFE unit (sulfonamide unit : TFE unit molar ratio) was 1 : 4.5.

Example 26

(I) Production of a terpolymer film

63.6 g of the sulfonyl fluoride (37) used in the step (I) of Example 18 (hereinafter, referred to as "$SO_2F$ monomer"), 3.3 g of the perfluorovinyl ether monomer (45) obtained in Example 24, 40 g of HFC43-10mee and 0.85 g of a 5 % $(CF_3CF_2CF_2COO)_2$ solution in HFC43-10mee (wherein the $(CF_3CF_2CF_2COO)_2$ is a polymerization initiator) were introduced into a 200 ml pressure resistant vessel which was made of a stainless steel and which was equipped with a gas introduction pipe. The internal atmosphere in the pressure resistant vessel was fully purged with nitrogen gas. Tetrafluoroethylene (TFE) was introduced into the pressure resistant vessel through the gas introduction pipe so that the internal pressure of the pressure resistant vessel was elevated to 0.3 MPa. Then, a reaction was performed at 23 °C for 4.5 hours while stirring and appropriately introducing TFE so as to maintain the internal pressure of the pressure resistant vessel at 0.3 MPa.
Thereafter, the introduction of TFE was stopped and the internal pressure of the pressure resistant vessel was lowered to atmospheric pressure, to obtain a reaction mixture (a white gel). Methanol was added to the obtained reaction mixture to precipitate a solid. The solid was recovered by filtration, followed by washing and subsequent drying, to obtain 11.6 g of a white solid.
From the $^{19}F$-NMR spectrum of the solid, it was confirmed that the solid was a terpolymer comprising a monomer unit (a sulfonamide units) derived from the perfluorovinyl ether monomer (45) obtained in Example 24, a monomer unit (an $SO_2F$ unit) derived from the above-mentioned $SO_2F$ monomer, and a monomer unit (a TFE unit) derived from the TFE. It was also confirmed that the ratio between TFE units, $SO_2F$ units and sulfonamide units (TFE unit : $SO_2F$ unit : sulfonamide unit molar ratio) was 4.1 : 1.0 : 0.04. Further, in the IR spectrum of the solid, peaks (at 3391, 3306 and 1544 $cm^{-1}$) ascribed to a sulfonamido group were observed.
The terpolymer exhibited a melt index of 7.5.
The above-mentioned terpolymer was subjected to a press molding at 270 °C, thereby obtaining a colorless transparent terpolymer film (film thickness: 88 μm).

(II) Production of a solid polymer electrolyte membrane

A solid polymer electrolyte membrane was obtained in substantially the same manner as in the step (II) of Example 16, except that the terpolymer film obtained in the step (I) above was used instead of the terpolymer film obtained in the step (I) of Example 16.
The solid polymer electrolyte membrane exhibited a tensile modulus of $4.1 \times 10^7$ dyn/cm$^2$ at 150 °C and a tensile modulus of $3.6 \times 10^7$ dyn/cm$^2$ at 300 °C.

Example 27

(I) Production of a terpolymer film

A polymerization reaction was performed in substantially the same manner as in the step (I) of Example 26, except that the amounts of $SO_2F$ monomer and perfluorovinyl ether monomer (45) were changed to 66.2 g and 0.67 g, respectively, thereby obtaining 13.7 g of a white solid.
From the $^{19}F$-NMR spectrum of the solid, it was confirmed that the solid contained an $SO_2F$ unit and a TFE unit, and that the molar ratio of the $SO_2F$ units to the TFE units was 1 : 3.7.
In addition, from the IR spectrum of the solid, it was confirmed that the solid contained a sulfonamide unit, and that the amount of the sulfonamide unit was 0.8 mol %, based on the total molar amount of the sulfonamide unit, $SO_2F$ unit and TFE unit.
Thus, it was confirmed that the obtained solid was a terpolymer comprising the sulfonamide unit, $SO_2F$ unit and TFE unit.
The terpolymer exhibited a melt index of 12.3. Further, the melt index of the terpolymer was measured again after keeping the terpolymer in a melt indexer at 270 °C for 1 hour without applying load, and was found to be the same as the previously measured value (12.3).
The above-mentioned terpolymer was subjected to a press molding at 270 °C, thereby obtaining a colorless transparent terpolymer film (film thickness: 82 μm).

(II) Production of a solid polymer electrolyte membrane

A solid polymer electrolyte membrane was obtained in substantially the same manner as in the step (II) of

Example 16, except that the terpolymer film obtained in the step (I) above was used instead of the terpolymer film obtained in the step (I) of Example 16.

The solid polymer electrolyte membrane exhibited a tensile modulus of $3.8 \times 10^7$ dyn/cm$^2$ at 150 °C and a tensile modulus of $1.2 \times 10^7$ dyn/cm$^2$ at 300 °C.

Example 28

(I) Synthesis of $CF_3CHFOCF_2CF(CF_3)OCF_2CF_2SO_2F$

124 g of sodium carbonate and 200 ml of tetraglyme were mixed together to obtain a slurry. The obtained slurry was charged into a flask equipped with a distillation head. 600 g of a compound represented by the following formula: $CF_3CF(COF)OCF_2CF(CF_3)OCF_2CF_2SO_2F$ was dropwise added to the slurry at 60 °C, and a reaction was performed at 60 °C for 1 hour while stirring, to thereby obtain a reaction mixture. 21 ml of water was added to the obtained reaction mixture and heated, first at 120 °C for 1 hour, then at 120 °C under reduced pressure, to thereby generate a vapor. The vapor was condensed and recovered as a distillate. The distillate was washed with water, followed by distillation under pressure of $133 \times 10^{-4}$ MPa, and a fraction having a boiling point of from 82 to 83 °C was recovered to thereby obtaining 219 g of a colorless liquid. From the $^{19}$F-NMR spectrum of the liquid, it was confirmed that the liquid was a compound represented by the following formula:

$$CF_3CHFOCF_2CF(CF_3)OCF_2CF_2SO_2F.$$

$^{19}$F-NMR: δ(ppm) -148.3 (1F), -146.6 (1F), -114.4 (2F), -88.3 (1F), -86.9 (3F), -85.5 (1F), -82.4 (3F), -81.4 (2F), 42.7 (1F).

$^1$H-NMR: δ(ppm) 6.0 (1H).

(II) Amidation reaction

20.8 g of a dispersion of sodium hydride in a mineral oil (sodium hydride content: 60 %) was washed with n-hexane under a stream of nitrogen gas so as to remove the mineral oil and obtain a sodium hydride powder. 300 ml of anhydrous dimethoxyethane was added to the obtained sodium hydride powder, and the resultant mixture was cooled to 0 °C. To the resultant mixture was dropwise added a solution obtained by dissolving 32 g of imidazol in 200 ml of dimethoxyethane. Then, the temperature of the resultant mixture was elevated to room temperature, followed by stirring for 1 hour, thereby obtaining an imidazol sodium amide solution.

The above-mentioned imidazol sodium amide solution was cooled to 0 °C, and then, 219 g of the compound obtained in the step (I) above was dropwise added to the solution, to obtain a mixture. The temperature of the mixture was elevated to room temperature, followed by stirring at room temperature for 12 hours to effect a reaction, thereby obtaining a reaction mixture. A small amount of water was added to the obtained reaction mixture, and then, dimethoxyethane was distilled off from the reaction mixture under reduced pressure to thereby obtain a residual liquid. To the obtained residual liquid was added a small amount of water and extracted with HFC43-10mee to obtain an extract solution. The obtained extract solution was washed with a diluted aqueous NaOH solution, followed by drying. Then, the solvent was distilled off from the dried extract to thereby obtain a residual liquid. The obtained residual liquid was subjected to distillation under a reduced pressure of $3.9 \times 10^{-4}$ MPa, and a fraction having a boiling point of from 102 to 104 °C was recovered, thereby obtaining 135 g of a colorless liquid. From the $^{19}$F-NMR and $^1$H-NMR spectra and GC-MS, it was confirmed that the solution was a sulfonamide represented-by the following formula:

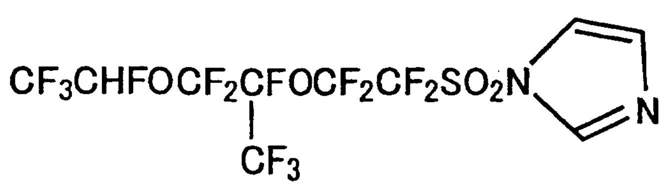

(47)

$^{19}$F-NMR: δ (ppm) -148.3 (1F), -146.6 (1F), -115.5 (2F), -88.3 (1F), -86.9 (3F), -85.5 (1F), -82.4 (3F), -81.4 (2F).

$^1$H-NMR: δ (ppm) 7.4 (1H), 6.8 (1H), 6.5 (1H), 6.0 (1H).

(III) Vinylation reaction (dehydrofluorination reaction)

126 ml of hexamethyldisilazane was dissolved in 500 ml of anhydrous THF to obtain a solution. The obtained solution was cooled to -78 °C. To the resultant solution was dropwise added 375 ml of a (1.6 M) solution of n-BuLi in n-hexane under a stream of nitrogen gas, to thereby obtain a mixture. The obtained mixture was stirred at -78 °C for 30 minutes, to obtain a lithium hexamethyldisilazide solution.
The temperature of the obtained solution was elevated to 0 °C. To the resultant solution was added a solution obtained by dissolving 135 g of the sulfonamide obtained in the step (II) above in 300 ml of THF, to thereby obtain a mixture. The obtained mixture was stirred at 0 °C for 1 hour to effect a reaction, thereby obtaining a reaction mixture.
A small amount of water was added to the obtained reaction mixture, and THF was distilled off from the reaction mixture, to obtain a residue. Then, small amounts of water and HFC43-10mee were added to the residue to obtain a mixture. The obtained mixture was subjected to filtration to remove insolubles, to obtain a filtrate. The organic phase of the filtrate was dried and the solvent was distilled off from the filtrate, to thereby obtain a residual liquid. The residual liquid was subjected to distillation under a reduced pressure of $4.0 \times 10^2$ Pa, and a fraction having a boiling point of from 140 to 150 °C was recovered, thereby obtaining 79 g of a colorless liquid. From the $^{19}$F-NMR spectrum and GC-MS, it was confirmed that the liquid was the same perfluorovinyl ether (42) as obtained in Example 21.

Comparative Example 5

30 g of a powder of the copolymer (36) used in Comparative Example 2 was immersed in 100 ml of HFC225ca/cb at room temperature for 30 minutes. The resultant mixture was cooled to -78 °C. 0.8 g of ammonia was condensed and added to the mixture, followed by stirring at -78 °C for 30 minutes. Then, the temperature of the resultant mixture was gradually elevated to room temperature, to effect a reaction, thereby obtaining a reaction mixture. During the reaction, the excess ammonia was removed from the reaction system by volatilization.
The obtained reaction mixture was dried by evaporation, to obtain a powdery residue. The powdery residue was washed twice with a 3N sulfuric acid at 80 °C, followed by further washing with heated water having a temperature of 80 °C, and subsequent drying, thereby obtaining a copolymer in which all $SO_2F$ groups have been amidated. Hereinafter, the obtained copolymer is referred to as "amidated copolymer".
10 parts by weight of the above-mentioned amidated copolymer and 90 parts by weight of the above-mentioned copolymer (36) were mixed together, and the resultant mixture was melt-kneaded using a kneader "Labo Plastomill" (trade name; manufactured and sold by Toyo Seiki Seisaku-sho, Ltd., Japan) at a revolution rate of 100 rpm at 270 °C for 20 minutes, thereby obtaining a composition.
The thus obtained composition was subjected to a press molding at 270 °C, thereby obtaining a copolymer film (thickness: 85 μm). However, the copolymer film was not uniform. More specifically, the copolymer film had a structure in which very small distinct particles were dispersed throughout the colorless matrix of the copolymer.
The above-mentioned copolymer film was analyzed by microscopic infrared spectroscopy. As a result, it was found that, in the copolymer film, the above-mentioned amidated copolymer and the above-mentioned copolymer (36) were completely separated from each other. More specifically, the amidated copolymer was contained only in the above-mentioned distinct particles, and the copolymer (36) was contained only in the above-mentioned matrix. This means that the amidated copolymer had an extremely poor compatibility with the copolymer (36).
Further, a solid polymer electrolyte membrane was obtained in substantially the same manner as in the step (I) of Example 16, except that the above-mentioned copolymer film was used instead of the terpolymer film obtained in the step (I) of Example 16.
The obtained membrane had a tensile modulus of $3.2 \times 10^7$ dyn/cm$^2$ at 150 °C. However, the membrane was melted at 180 °C, and hence, it was impossible to measure the tensile modulus at 300 °C.

Example 29

(I) Production of a terpolymer film

233 g of the sulfonyl fluoride (37) used in the step (I) of Example 18 (hereinafter, referred to as "$SO_2F$ monomer"), 4.4 g of the perfluorovinyl ether monomer obtained in Example 14, 711 g of HFC43-10mee and 3.7 g of a 5 % $(CF_3CF_2CF_2COO)_2$ solution in HFC43-10mee (wherein the $(CF_3CF_2CF_2COO)_2$ is a polymerization initiator) were introduced into a 1 liter pressure resistant vessel which was made of a stainless steel and which was equipped with a gas introduction pipe. The internal atmosphere in the pressure resistant vessel was fully purged with nitrogen gas. Tetrafluoroethylene (TFE) was introduced into the pressure resistant vessel through the gas introduction pipe so that the internal pressure of the pressure resistant vessel was elevated to 0.16 MPa. Then, a reaction was performed at 35 °C for 5.5 hours while stirring and appropriately introducing TFE so as to reduce the internal pressure of the pressure

resistant vessel gradually from 0.16 MPa to 0.14 MPa. Further, during the reaction, 1.9 g of a 5 % $(CF_3CF_2CF_2COO)_2$ solution in HFC43-10mee was added to the pressure resistant vessel.

Thereafter, the introduction of TFE was stopped and the internal pressure of the pressure resistant vessel was lowered to atmospheric pressure, to obtain a reaction mixture. From the reaction mixture, the solvent and unreacted monomers were distilled off, and the resultant residue was subjected to a filtration, to thereby recover the solid. The recovered solid was washed with HFC43-10mee, followed by drying, to obtain 50.0 g of a white solid.

From the $^{19}$F-NMR spectrum of the solid, it was confirmed that the solid was a terpolymer comprising a monomer unit (a sulfonamide unit) derived from the perfluorovinyl ether obtained in Example 14, a monomer unit (an $SO_2F$ units) derived from the above-mentioned $SO_2F$ monomer, and a monomer unit (a TFE unit) derived from TFE. It was also confirmed that the ratio between the TFE units, $SO_2F$ units and sulfonamide units (TFE unit : $SO_2F$ unit : sulfonamide unit molar ratio) was 3.2 : 1.0 : 0.019. Further, in the IR spectrum of the solid, peaks ascribed to a sulfonamido group were observed.

The terpolymer exhibited a melt index of 15.9.

The above-mentioned terpolymer was subjected to a press molding at 270 °C, thereby obtaining a colorless transparent terpolymer film (film thickness: 56 μm).

(II) Production of a solid polymer electrolyte membrane

A solid polymer electrolyte membrane was obtained in substantially the same manner as in the step (II) of Example 16, except that the terpolymer film obtained in the step (I) above was used instead of the terpolymer film obtained in the step (I) of Example 16.

With respect to the obtained solid polymer electrolyte membrane, the proton conductivity was measured, and found to be 0.099 S/cm. Further, the proton conductivity was also measured with respect to another solid polymer electrolyte membrane, which was obtained by using the terpolymer film obtained in the step (I) above in substantially the same manner as in the step (II) of Example 16, except that the terpolymer film was not subjected to the modification treatment. As a result, it was found that the proton conductivity of the thus obtained solid polymer electrolyte membrane was 0.107 S/cm.

The solid polymer electrolyte membrane (obtained using the terpolymer film which had been subjected to the modification treatment) exhibited a tensile modulus of $2.9 \times 10^7$ dyn/cm$^2$ at 150°C and a tensile modulus of $2.5 \times 10^7$ dyn/cm$^2$ at 300 °C.

Example 30

(I) Production of a terpolymer film

A polymerization reaction was performed in substantially the same manner as in Example 29, except that 7.1 g of the perfluorovinyl ether monomer (45) obtained in Example 24 was used instead of 4.4 g of the perfluorovinyl ether monomer obtained in Example 14, thereby obtaining 45.3 g of a white solid.

From the $^{19}$F-NMR spectrum of the solid, it was confirmed that the solid was a terpolymer comprising a monomer unit (a sulfonamide unit) which was derived from the perfluorovinyl ether (45) obtained in Example 24, a monomer unit (an $SO_2F$ unit) which was derived from the above-mentioned $SO_2F$ monomer, and a monomer unit (a TFE unit) which was derived from TFE. It was also confirmed that the ratio between TFE unit, $SO_2F$ unit and sulfonamide unit (TFE unit : $SO_2F$ unit : sulfonamide unit molar ratio) was 3.4 : 1.0 : 0.023. Further, in the IR spectrum of the solid, peaks ascribed to a sulfonamido group were observed.

The terpolymer exhibited a melt index of 17.5.

The above-mentioned terpolymer was subjected to a press molding at 270 °C. thereby obtaining a colorless transparent terpolymer film (film thickness: 56 μm).

(II) Production of a solid polymer electrolyte membrane

A solid polymer electrolyte membrane was obtained in substantially the same manner as in the step (II) of Example 16 (in which the terpolymer film is subjected to the modification treatment), except that the terpolymer film obtained in the step (I) above was used instead of the terpolymer film obtained in the step (I) of Example 16.

With respect to the above-mentioned solid polymer electrolyte membrane (obtained using a modified terpolymer film), the proton conductivity was measured, and found to be 0.105 S/cm. Further, the proton conductivity was also measured with respect to another solid polymer electrolyte membrane, which was obtained by using the above terpolymer film (obtained in the step (I) above) in substantially the same manner as in the step (II) of Example 16, except that the terpolymer film was not subjected to the modification treatment. As a result, it was found that the proton

conductivity of the thus obtained solid polymer electrolyte membrane was 0.091 S/cm.

With respect to the above-mentioned solid polymer electrolyte membrane obtained using the modified terpolymer film, the solid polymer electrolyte membrane exhibited a tensile modulus of $3.0 \times 10^7$ dyn/cm$^2$ at 150 °C and a tensile modulus of $2.6 \times 10^7$ dyn/cm$^2$ at 300 °C.

Example 31

(I) Production of a terpolymer film

A polymerization reaction was performed in substantially the same manner as in the step (I) of Example 29, except that the amount of the perfluorovinyl ether monomer obtained in Example 14 was changed to 10.9 g, thereby obtaining 40.3 g of a white solid.

From the $^{19}$F-NMR spectrum of the solid, it was confirmed that the solid was a terpolymer comprising a monomer unit (a sulfonamide unit) which was derived from the perfluorovinyl ether monomer obtained in Example 14, a monomer unit (an $SO_2F$ unit) which was derived from the $SO_2F$ monomer, and a monomer unit (a TFE unit) which was derived from TFE. The ratio between TFE unit, $SO_2F$ unit and sulfonamide unit (TFE unit : $SO_2F$ unit : sulfonamide unit molar ratio) was confirmed. In addition, it was confirmed that peaks ascribed to a sulfonamido group were observed in the IR spectrum of the solid.

The terpolymer exhibited a melt index of 40.3.

The terpolymer was subjected to a press molding at 270 °C, thereby obtaining a colorless transparent terpolymer film (film thickness: 40 μm).

(II) Production of a solid polymer electrolyte membrane

A solid polymer electrolyte membrane was obtained in substantially the same manner as in the step (II) of Example 16 (in which the terpolymer film is subjected to the modification treatment), except that the terpolymer film obtained in the step (I) above was used instead of the terpolymer film obtained in the step (I) of Example 16.

With respect to the above-mentioned solid polymer electrolyte membrane (obtained using a modified terpolymer film), the proton conductivity was measured, and found to be 0.097 S/cm. Further, the proton conductivity was also measured with respect to another solid polymer electrolyte membrane, which was obtained by using the above terpolymer film (obtained in the step (I) above) in substantially the same manner as in the step (II) of Example 16, except that the terpolymer film was not subjected to the modification treatment. As a result, it was found that the proton conductivity of the thus obtained solid polymer electrolyte membrane is 0.104 S/cm.

With respect to the above-mentioned solid polymer electrolyte membrane obtained using the modified terpolymer film, the solid polymer electrolyte membrane exhibited a tensile modulus of $3.3 \times 10^7$ dyn/cm$^2$ at 150 °C and a tensile modulus of $4.6 \times 10^7$ dyn/cm$^2$ at 300 °C.

Example 32

14 parts by weight of the copolymer obtained in Example 31 and 86 parts by weight of the copolymer (37) used in Comparative Example 2 were mixed together, and the resultant mixture was melt-kneaded using a kneader "Labo Plastomill" (trade name; manufactured and sold by Toyo Seiki Seisaku-sho, Ltd., Japan) at 270 °C for 20 minutes at a revolution rate of 100 rpm, thereby obtaining a composition.

The thus obtained composition was subjected to a press molding at 270 °C, thereby obtaining a uniform, colorless transparent copolymer film (film thickness: 56 μm).

A solid polymer electrolyte membrane was obtained in substantially the same manner as in the step (II) of Example 16, except that the copolymer film obtained above was used instead of the terpolymer film obtained in the step (I) of Example 16.

The obtained solid polymer electrolyte membrane exhibited a tensile modulus of $4.4 \times 10^7$ dyn/cm$^2$ at 150 °C and a tensile modulus of $4.1 \times 10^7$ dyn/cm$^2$ at 300 °C.

Comparative Example 6

(I) Amidation reaction

36.6 g of the sodium carboxylate obtained in the step (I) of Example 1 was dissolved in 100 ml of diglyme, to obtain a solution. The obtained solution was cooled to -50 °C. 17 g of ammonia was condensed and then added to the solution, to obtain a mixture.

The temperature of the obtained mixture was gradually elevated to room temperature over 7 hours while stirring, to effect a reaction, thereby obtaining a reaction mixture. During the reaction, excess ammonia was volatilized and removed from the mixture.

The obtained reaction mixture (a white turbid liquid) was subjected to filtration to obtain a filtrate. The solvent in the filtrate was distilled off under reduced pressure to obtain a residue. The obtained residue was subjected to a vacuum drying at 60 °C, thereby obtaining 43 g of a white viscous liquid. From the $^{19}$F-NMR spectrum of the liquid, it was confirmed that the liquid was a sulfonamide represented by the following formula:

$$CF_3CF(CO_2Na)OCF_2CF_2SO_2NH_2.$$

$^{19}$F-NMR: $\delta$ (ppm) -131.7 (1F), -122.3 (1F), -118.6 (1F), -87.5 (1F), -83.1 (3F), -74.6 (1F).

(II) Decarboxylation reaction

43 g of the sulfonamide obtained in the step (I) above was dissolved in 100 ml of diglyme to obtain a solution. The obtained solution was heated under a stream of nitrogen gas at 150 °C for 2 hours, thereby obtaining a reaction mixture. From the $^{19}$F-NMR spectrum of the reaction mixture, it was confirmed that the obtained reaction mixture contained a proton addition product represented by the following formula:

$$CF_3CHFOCF_2CF_2SO_2NH_2.$$

19F-NMR: $\delta$ (ppm) -146.7 (1F), -117.9 (2F), -84.7 (1F), -84.5 (3F), -82.6 (1F).

It was also confirmed that the reaction mixture contained no perfluorovinyl ether of the present invention represented by the following formula:

$$CF_2=CFOCF_2CF_2SO_2NH_2.$$

INDUSTRIAL APPLICABILITY

The perfluorovinyl ether monomer of the present invention can be used for producing a fluorinated polymer which exhibits excellent properties. The fluorinated polymer can be used in various fields; for example, the fluorinated polymer can be advantageously used as a raw material for producing a solid polymer electrolyte. The solid polymer electrolyte obtained from the perfluorovinyl ether monomer of the present invention exhibits not only excellent durability, but also excellent heat resistance and high proton conductivity, and, hence, the solid polymer electrolyte can be advantageously used in a fuel cell.

## Claims

1. A perfluorovinyl ether monomer represented by the following formula (1):

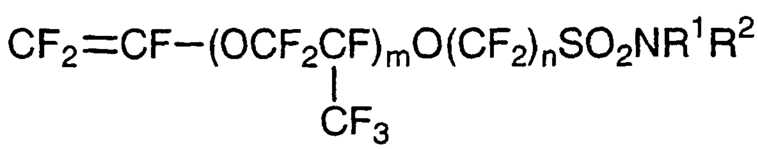

(1)

wherein:

m is an integer of from 0 to 5;
n is an integer of from 1 to 5; and
each of $R^1$ and $R^2$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a

sulfonyl group and an ether group, wherein said substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total,
with the proviso that, when each of $R^1$ and $R^2$ is independently the unsubstituted or substituted $C_1$-$C_{10}$ hydrocarbon group or the substituted silyl group, $R^1$ and $R^2$ are optionally bonded together to form a divalent group, thereby forming a saturated or unsaturated nitrogen-containing heterocyclic ring in cooperation with a nitrogen atom which is bonded to $R^1$ and $R^2$.

**2.** The monomer according to claim 1, wherein $R^1$ in formula (1) is a hydrogen atom, the unsubstituted or substituted $C_1$-$C_{10}$ hydrocarbon group or the substituted silyl group and $R^2$ in formula (1) is a hydrogen atom or the substituted silyl group.

**3.** The monomer according to claim 1, wherein at least one of $R^1$ and $R^2$ in formula (1) is the substituted silyl group.

**4.** The monomer according to claim 1, wherein at least one of $R^1$ and $R^2$ in formula (1) is a hydrogen atom.

**5.** The monomer according to claim 1, wherein each of $R^1$ and $R^2$ in formula (1) is a hydrogen atom.

**6.** A method for producing the monomer of claim 1, which comprises:

(i) converting an acyl fluoride represented by the following formula (2):

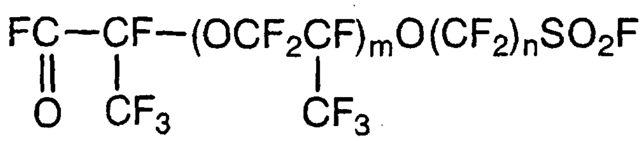

(2)

wherein m and n are as defined above for formula (1).
to a carboxylate represented by the following formula (3):

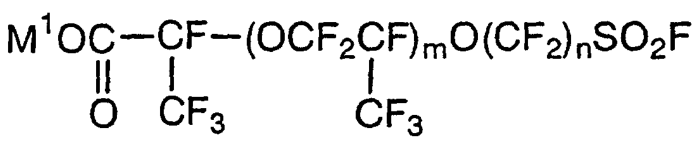

(3)

wherein:

m and n are as defined above for formula (1); and
$M^1$ is an alkali metal, an alkaline earth metal, a quaternary ammonium group or a quaternary phosphonium group;

(ii) effecting an amidation reaction of the fluorosulfonyl group of said carboxylate (3) to thereby obtain a sulfonamido represented by the following formula (4):

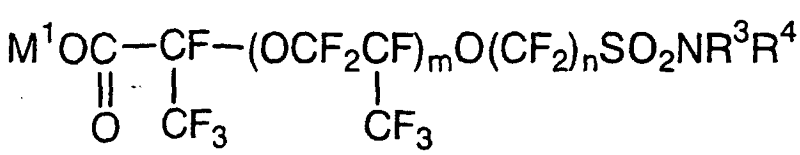

(4)

wherein:

m and n are as defined above for formula (1);
$M^1$ is as defined above for formula (3); and
each of $R^3$ and $R^4$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein said substituted $C_1$-$C_{10}$ hydrocarbon group has up to 15 carbon atoms in total; a substituted silyl group containing as a substituent at least one $C_1$-$C_{10}$ hydrocarbon group so as to have up to 10 carbon atoms in total; an alkali metal; an alkaline earth metal; an ammonium group; or a phosphonium group, with the proviso that, when each of $R^3$ and $R^4$ is independently the unsubstituted or substituted $C_1$-$C_{10}$ hydrocarbon group or the substituted silyl group, $R^3$ and $R^4$ are optionally bonded together to form a divalent group, thereby forming a saturated or unsaturated nitrogen-containing heterocyclic ring in cooperation with a nitrogen atom which is bonded to $R^3$ and $R^4$, and that $R^3$ and $R^4$ are not simultaneously hydrogen atoms,

optionally followed by treatment with an alkaline compound; and
(iii) subjecting said sulfonamido (4) to decarboxylation-vinylation, optionally followed by treatment with a protic compound.

**7.** The method according to claim 6, wherein each m in formulae (1), (2), (3) and (4) is 0.

**8.** A sulfonamide represented by the following formula (4):

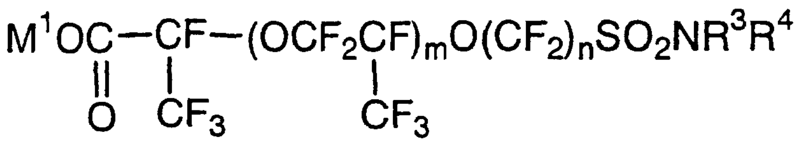

(4)

wherein:

m is an integer of from 0 to 5;
n is an integer of from 1 to 5;
$M^1$ is an alkali metal, an alkaline earth metal, a quaternary ammonium group or a quaternary phosphonium group; and
each of $R^3$ and $R^4$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein said substituted $C_1$-$C_{10}$ hydrocarbon group has up to 15 carbon atoms in total; a substituted silyl group containing as a substituent at least one $C_1$-$C_{10}$ hydrocarbon group so as to have up to 10 carbon atoms in total; an alkali metal; an alkaline earth metal; an ammonium group; or a phosphonium group, with the proviso that, when each of $R^3$ and $R^4$ is independently the unsubstituted or substituted $C_1$-$C_{10}$ hydrocarbon group or the substituted silyl group, $R^3$ and $R^4$ are optionally bonded together to form a divalent group, thereby forming a saturated or unsaturated nitrogen-containing heterocyclic ring in cooperation with a nitrogen atom which is bonded to $R^3$ and $R^4$, and that $R^3$ and $R^4$ are not simultaneously hydrogen atoms.

**9.** The sulfonamide according to claim 8, wherein m in formula (4) is 0.

**10.** A method for producing the monomer of claim 1, wherein each of $R^1$ and $R^2$ in formula (1) is a hydrogen atom, or wherein each of $R^1$ and $R^2$ in formula (1) is independently a hydrogen atom; a $C_1$-$C_{10}$ hydrocarbon group which is unsubstituted or substituted with at least one substituent selected from the group consisting of a N,N-disubstituted amino group containing as substituents two hydrocarbon groups, an alkoxy group and an ether group, wherein said substituted $C_1$-$C_{10}$ hydrocarbon group has up to 15 carbon atoms in total; or the substituted silyl group, with the proviso that at least one of $R^1$ and $R^2$ in formula (1) is a $C_3$-$C_{10}$ secondary or tertiary alkyl group or the substituted silyl group,

said method comprising subjecting a sulfonyl fluoride represented by the following formula (5):

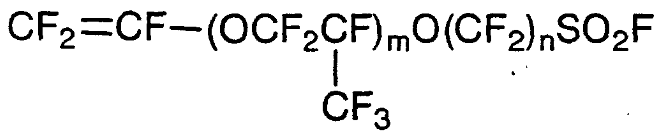 (5)

wherein m and n are as defined above for formula (1),
to amidation, optionally followed by treatment with a protic compound,

wherein said amidation is performed by reacting said sulfonyl fluoride (5) with an amine or metal amide, which is represented by the following formula (6):

$$M^2NR^5R^6 \quad (6)$$

wherein:

$M^2$ is a hydrogen atom, an alkali metal or an alkaline earth metal; and
each of $R^5$ and $R^6$ independently represents a $C_1$-$C_{10}$ hydrocarbon group which is unsubstituted or substituted with at least one substituent selected from the group consisting of a N,N-di-substituted amino group containing as substituents two hydrocarbon groups, an alkoxy group and an ether group, wherein said substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the proviso that at least one of $R^5$ and $R^6$ is a $C_3$-$C_{10}$ secondary or tertiary alkyl group or the substituted silyl group,

wherein $R^5$ and $R^6$ are optionally bonded together to form a divalent group, thereby forming a saturated or unsaturated nitrogen-containing heterocyclic ring in cooperation with a nitrogen atom which is bonded to $R^5$ and $R^6$.

**11.** A method for producing the monomer of claim 1, which comprises subjecting a compound represented by the following formula (7):

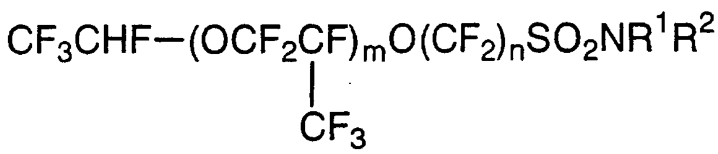 (7)

wherein m, n, $R^1$ and $R^2$ are as defined above for formula (1),
to dehydrofluorination, optionally followed by treatment with a protic compound,

wherein said dehydrofluorination is performed by contacting said compound (7) with a metal amide, which is represented by the following formula (8):

$$M^3NR^xR^y \quad (8)$$

wherein:

$M^3$ is an alkali metal or an alkaline earth metal; and
each of $R^x$ and $R^y$ independently represents a $C_1$-$C_{10}$ hydrocarbon group which is unsubstituted or substituted with at least one substituent selected from the group consisting of a N,N-disubstituted amino group containing as substituents two hydrocarbon groups, an alkoxy group and an ether group, wherein said substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total, with the proviso that at least one of $R^x$ and $R^y$ is a $C_3$-$C_{10}$ secondary or tertiary alkyl group or the substituted silyl group,

wherein $R^x$ and $R^y$ are optionally bonded together to form a divalent group, thereby forming a saturated or unsaturated nitrogen-containing heterocyclic ring in cooperation with a nitrogen atom which is bonded to $R^x$ and $R^y$.

**12.** A compound represented by the following formula (7):

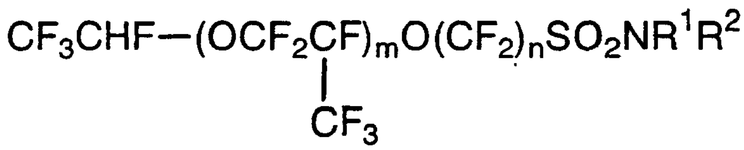

(7)

wherein:

m is an integer of from 0 to 5;
n is an integer of from 1 to 5; and
each of $R^1$ and $R^2$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein said substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total,
with the proviso that, when each of $R^1$ and $R^2$ is independently the unsubstituted or substituted $C_1$-$C_{10}$ hydrocarbon group or the substituted silyl group, $R^1$ and $R^2$ are optionally bonded together to form a divalent group, thereby forming a saturated or unsaturated nitrogen-containing heterocyclic ring in cooperation with a nitrogen atom which is bonded to $R^1$ and $R^2$ .

**13.** A method for producing the monomer of claim 1, which comprises subjecting a compound represented by the following formula (9):

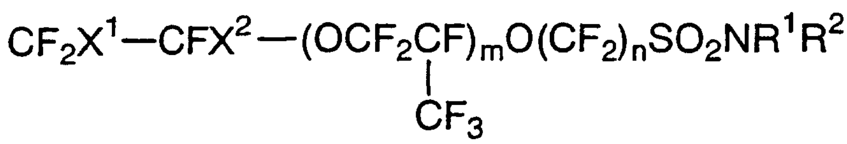

(9)

wherein:

m, n, $R^1$ and $R^2$ are as defined above for formula (1); and
each of $X^1$ and $X^2$ is independently a chlorine atom, a bromine atom or an iodine atom,
to dehalogenation, optionally followed by treatment with a protic compound.

**14.** A compound represented by the following formula (9):

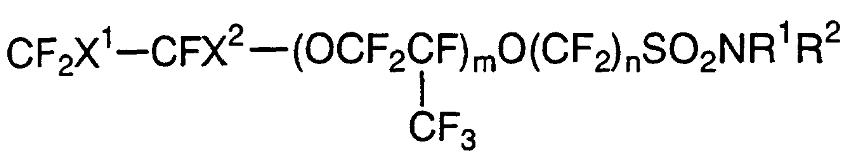

(9)

wherein:

m is an integer of from 0 to 5;
n is an integer of from 1 to 5;
each of $R^1$ and $R^2$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein said substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total,

with the proviso that, when each of $R^1$ and $R^2$ is independently the unsubstituted or substituted $C_1$-$C_{10}$ hydrocarbon group or the substituted silyl group, $R^1$ and $R^2$ are optionally bonded together to form a divalent group, thereby forming a saturated or unsaturated nitrogen-containing heterocyclic ring in cooperation with a nitrogen atom which is bonded to $R^1$ and $R^2$ ; and

each of $X^1$ and $X^2$ is independently a chlorine atom, a bromine atom or an iodine atom.

**15.** A method for producing a fluorinated polymer, which comprises subjecting a perfluorovinyl ether monomer represented by the following formula (1):

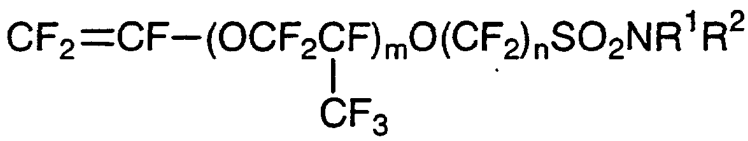

(1) .

wherein:

m is an integer of from 0 to 5;

n is an integer of from 1 to 5; and

each of $R^1$ and $R^2$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein said substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total,

with the proviso that, when each of $R^1$ and $R^2$ is independently the unsubstituted or substituted $C_1$-$C_{10}$ hydrocarbon group or the substituted silyl group, $R^1$ and $R^2$ are optionally bonded together to form a divalent group, thereby forming a saturated or unsaturated nitrogen-containing heterocyclic ring in cooperation with a nitrogen atom which is bonded to $R^1$ and $R^2$,

to homopolymerization or copolymerization with at least one comonomer having an olefinic unsaturated bond.

**16.** The method according to claim 15, wherein said monomer (1) is copolymerized with a comonomer comprising tetrafluoroethylene.

**17.** A fluorinated polymer produced by the method of claim 15 or 16.

**18.** A fluorinated polymer comprising monomer units derived from at least one perfluorovinyl ether monomer represented by the following formula (10):

$$CF_2{=}CFO(CF_2)_pSO_2NR^aR^b \qquad (10)$$

wherein:

p is an integer of from 1 to 5; and

each of $R^a$ and $R^b$ independently represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein said substituted $C_1$-$C_{10}$ hydrocarbon group has up to 15 carbon

atoms in total; or a substituted silyl group containing as a substituent at least one $C_1$-$C_{10}$ hydrocarbon group so as to have up to 10 carbon atoms in total, with the proviso that, when each of $R^a$ and $R^b$ is independently the unsubstituted or substituted $C_1$-$C_{10}$ hydrocarbon group or the substituted silyl group, $R^a$ and $R^b$ are optionally bonded together to form a divalent group, thereby forming a saturated or unsaturated nitrogen-containing heterocyclic ring in cooperation with a nitrogen atom which is bonded to $R^a$ and $R^b$.

**19.** The fluorinated polymer according to claim 18, which is a fluorinated copolymer comprising monomer units each derived from said monomer (10) and comonomer units each derived from tetrafluoroethylene.

**20.** A method for producing a fluorinated copolymer, which comprises subjecting to copolymerization:

(a) at least one monomer having a partially fluorinated or perfluorinated vinyl group and a group represented by the following formula (11):

$$-SO_2NR^7R^8 \quad (11)$$

wherein:

$R^7$ represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein said substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total; and
$R^8$ represents a hydrogen atom or the substituted silyl group;

(b) at least one monomer having a partially fluorinated or perfluorinated vinyl group and a group represented by the following formula (12):

$$-SO_2X^3 \quad (12)$$

wherein $X^3$ represents a fluorine atom, a chlorine atom or a $-OR^9$ group, wherein $R^9$ represents the unsubstituted or substituted $C_1$-$C_{10}$ hydrocarbon group or the substituted silyl group; and optionally
(c) at least one monomer other than said monomers (a) and (b), which has an olefinic unsaturated bond.

**21.** The method according to claim 20, wherein said monomer (a) is a monomer represented by the following formula (13):

$$CF_2{=}CF\text{-}Rf\text{-}SO_2NR^7R^8 \quad (13)$$

wherein:

$R^7$ and $R^8$ are as defined above for formula (11); and
Rf is a single bond; a $C_1$-$C_{20}$ fluoroalkylene group represented by the following formula (14):

$$-C_qX^4{}_{2q}- \quad (14)$$

wherein:

q is an integer of from 1 to 20; and

each $X^4$ is independently a fluorine atom; or a monovalent substituent selected from the group consisting of a hydrogen atom, a chlorine atom and an alkoxy group, with the proviso that the number of said monovalent

substituent is 35 % or less, based on the number of $X^4$; or

a $C_1$-$C_{20}$ oxyfluoroalkylene group represented by the following formula (15):

$$-OC_qX^4{}_{2q}- \qquad (15)$$

wherein q and $X^4$ are as defined above for formula (14),

wherein at least one single bond between two adjacent carbon atoms of said $C_1$-$C_{20}$ fluoroalkylene group (14) or $C_1$-$C_{20}$ oxyfluoroalkylene group (15) is optionally substituted with at least one divalent substituent selected from the group consisting of an oxygen atom, a carbonyl group, a sulfonyl group, a biscarbonylimide group, a bissulfonylimide group and a carbonylsulfonylimide group, with the proviso that the number of said divalent substituent is 50 % or less, based on the number q.

**22.** The method according to claim 20, wherein said monomer (a) is a monomer represented by the following formula (16):

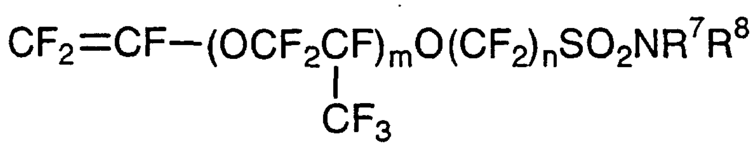

(16)

wherein:

m is an integer of from 0 to 5;
n is an integer of from 1 to 5; and
$R^7$ and $R^8$ are as defined above for formula (11).

**23.** The method according to any one of claims 20 to 22, wherein said monomers (a), (b) and (c) are subjected to copolymerization, said monomer (c) comprising tetrafluoroethylene.

**24.** A fluorinated copolymer obtained by the method of any one of claims 20 to 23.

**25.** A fluorinated copolymer comprising the following sulfonyl group-containing monomer units (A) and (B):

(A) monomer units derived from at least one monomer having a partially fluorinated or perfluorinated vinyl group and a group represented by the following formula (11):

$$-SO_2NR^7R^8 \qquad (11)$$

wherein:

$R^7$ represents a hydrogen atom; a $C_1$-$C_{10}$ hydrocarbon group which is unsubstituted or substituted with at least one substituent selected from the group consisting of a halogen atom, a hydroxyl group, an amino group, an alkoxy group, a carbonyl group, an ester group, an acid amido group, a sulfonyl group and an ether group, wherein said substituted $C_1$-$C_{10}$ hydrocarbon group has up to 15 carbon atoms in total; or a substituted silyl group containing as a substituent at least one $C_1$-$C_{10}$ hydrocarbon group so as to have up to 10 carbon atoms in total; and
$R^8$ represents a hydrogen atom or the substituted silyl group, and

(B) monomer units derived from at least one monomer having a partially fluorinated or perfluorinated vinyl group and a group represented by the following formula (12):

$$-SO_2X^3 \quad (12)$$

wherein $X^3$ represents a fluorine atom, a chlorine atom or a $-OR^9$ group, wherein $R^9$ represents the unsubstituted or substituted $C_1$-$C_{10}$ hydrocarbon group or the substituted silyl group.

**26.** The copolymer according to claim 25, which comprises said monomer units (A) and (B) and comonomer units each derived from tetrafluoroethylene.

**27.** The copolymer according to claim 25 or 26, wherein the amount of said monomer unit (A) is from 0.001 to 50 mol %, based on the total molar amount of said monomer units (A) and (B).

**28.** The copolymer according to any one of claims 25 to 27, wherein the weight of said copolymer per mole of sulfonyl groups in said monomer units (A) and (B), which is obtained by dividing the weight (g) of said copolymer by the total molar amount of said monomer units (A) and (B), is from 400 to 1400 g/mol.

**29.** The copolymer according to any one of claims 25 to 28, wherein each of said monomer units (A) is derived from a monomer represented by the following formula (13):

$$CF_2{=}CF\text{-}Rf\text{-}SO_2NR^7R^8 \quad (13)$$

wherein:

$R^7$ and $R^8$ are as defined above for formula (11); and
Rf is a single bond; a $C_1$-$C_{20}$ fluoroalkylene group represented by the following formula (14):

$$-C_qX^4{}_{2q}- \quad (14)$$

wherein:

q is an integer of from 1 to 20; and
each $X^4$ is independently a fluorine atom; or a monovalent substituent selected from the group consisting of a hydrogen atom, a chlorine atom and an alkoxy group, with the proviso that the number of said monovalent substituent is 35 % or less, based on the number of $X^4$; or

a $C_1$-$C_{20}$ oxyfluoroalkylene group represented by the following formula (15):

$$-OC_qX^4{}_{2q}- \quad (15)$$

wherein q and $X^4$ are as defined above for formula (14),
wherein at least one single bond between two adjacent carbon atoms of said $C_1$-$C_{20}$ fluoroalkylene group (14) or $C_1$-$C_{20}$ oxyfluoroalkylene group (15) is optionally substituted with at least one divalent substituent selected from the group consisting of an oxygen atom, a carbonyl group, a sulfonyl group, a biscarbonylimide group, a bissulfonylimide group and a carbonylsulfonylimide group, with the proviso that the number of said divalent substituent is 50 % or less of said integer q.

**30.** The copolymer according to any one of claims 25 to 28, wherein each of said monomer units (A) is derived from at least one monomer represented by the following formula (16):

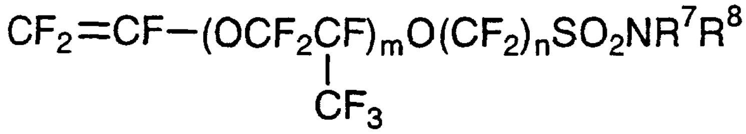

(16)

wherein:

m is an integer of from 0 to 5;
n is an integer of from 1 to 5; and
$R^7$ and $R^8$ are as defined above for formula (11).

**31.** A copolymer film produced from the copolymer of any one of claims 24 to 30 or a composition comprising the copolymer of any one of claims 24 to 30.

**32.** A method for producing the copolymer film of claim 31, which comprises forming the copolymer of any one of claims 24 to 30 or a composition comprising the copolymer of any one of claims 24 to 30 by melt processing.

**33.** A copolymer film produced by the method of claim 32.

**34.** The copolymer film according to claim 31 or 33, which is in the form of a single-layer film.

**35.** A method for producing a modified copolymer film, which comprises subjecting the copolymer film of any one of claims 31, 33 and 34 to treatment with a basic compound.

**36.** A modified copolymer film produced by the method of claim 35.

**37.** A method for producing a solid polymer electrolyte membrane, which comprises subjecting the modified copolymer film of claim 36 to at least one treatment selected from the group consisting of alkali treatment and acid treatment.

**38.** A solid polymer electrolyte membrane produced by the method of claim 37.

**39.** A method for producing a crosslinked copolymer film, which comprises subjecting the copolymer film of any one of claims 31, 33 and 34 to treatment with a basic compound.

**40.** A crosslinked copolymer film produced by the method of claim 39.

**41.** A method for producing a crosslinked solid polymer electrolyte membrane, which comprises subjecting the crosslinked copolymer film of claim 40 to at least one treatment selected from the group consisting of alkali treatment and acid treatment.

**42.** A crosslinked solid polymer electrolyte membrane produced by the method of claim 41.

Fig.1

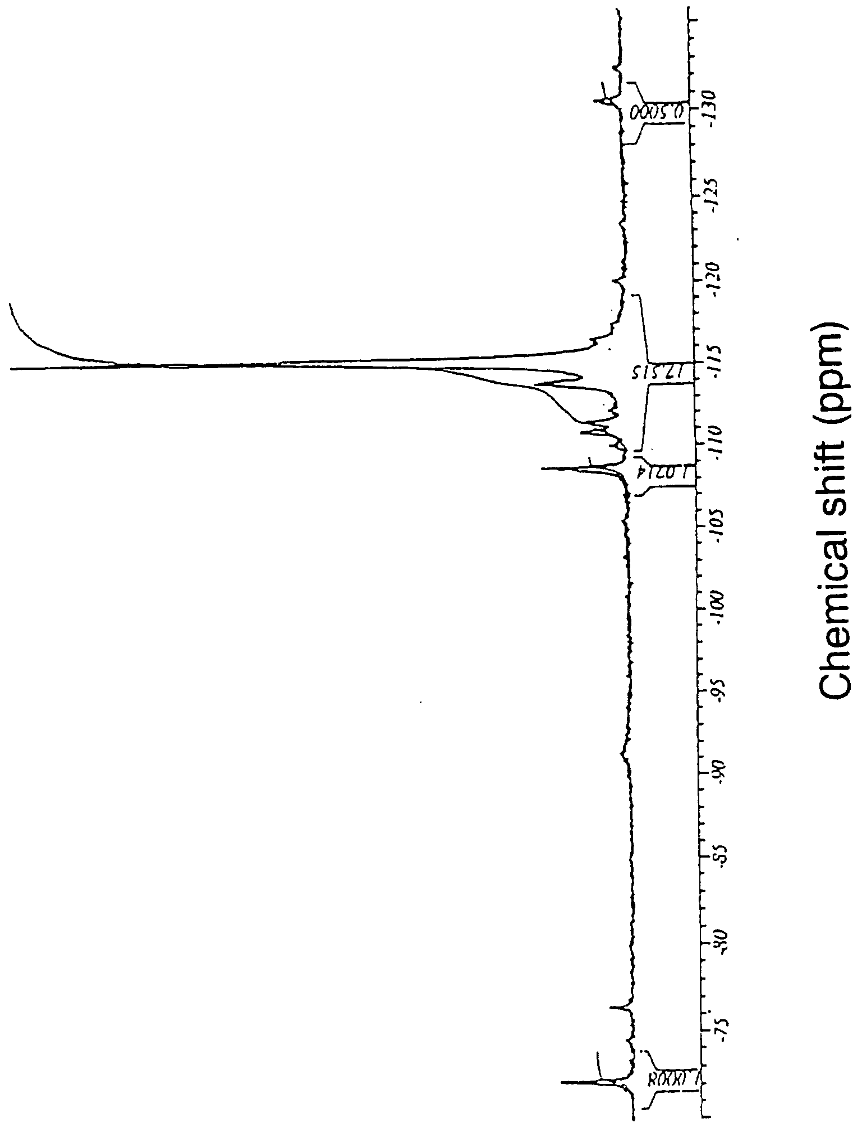

Fig.2

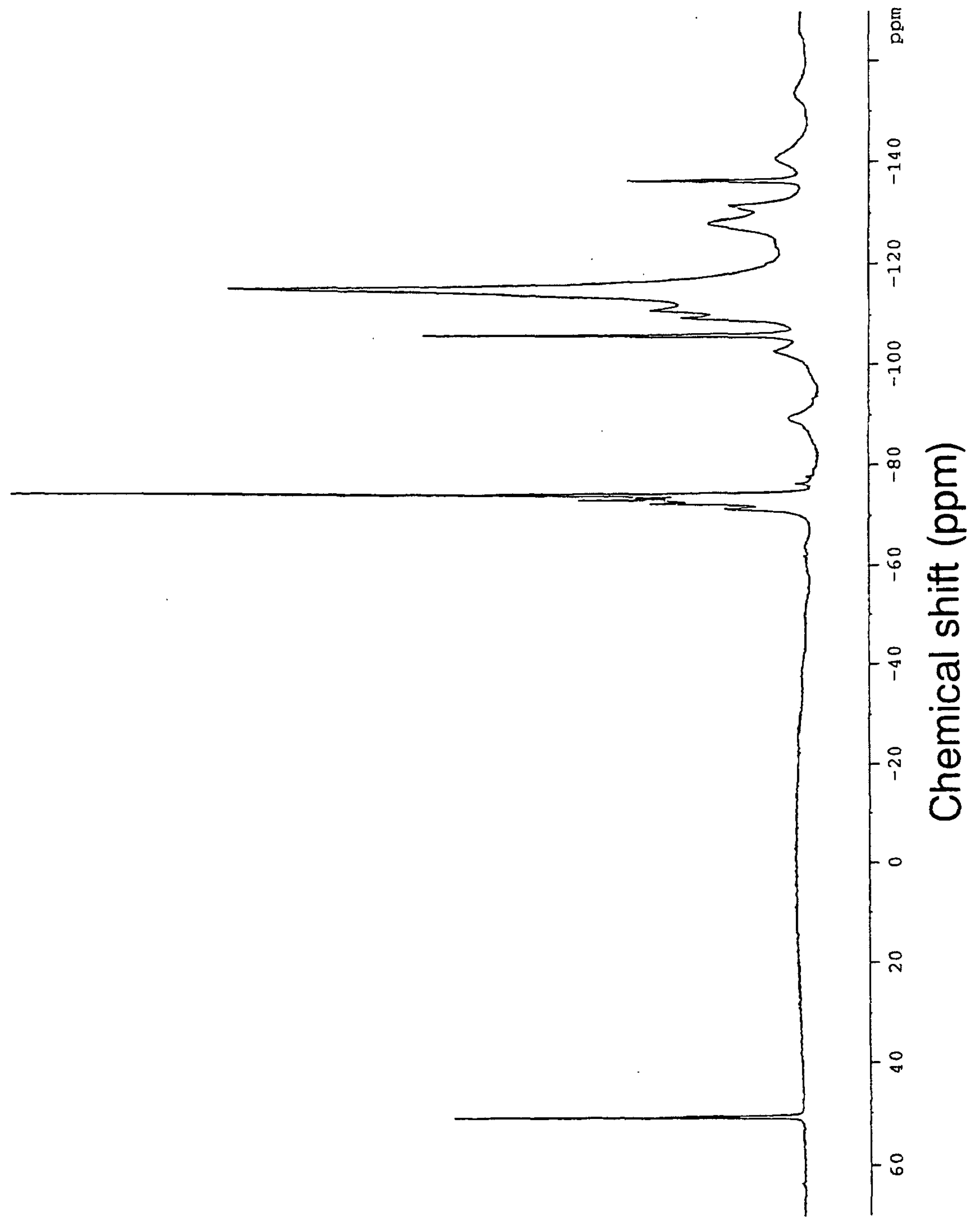

# INTERNATIONAL SEARCH REPORT

International application No.
PCT/JP02/00854

A. CLASSIFICATION OF SUBJECT MATTER
Int.Cl[7] C07C311/24, 303/36, C07F7/12, C08F214/26, C08F216/14, H01M8/02

According to International Patent Classification (IPC) or to both national classification and IPC

B. FIELDS SEARCHED

Minimum documentation searched (classification system followed by classification symbols)
Int.Cl[7] C07C311/00, 303/00, C07F7/00, C08F214/00, H01M8/00

Documentation searched other than minimum documentation to the extent that such documents are included in the fields searched

Electronic data base consulted during the international search (name of data base and, where practicable, search terms used)
REGISTRY(STN), CA(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | WO 00/52085 A1 (E.I. Du Pont De Nemours and Co.), 08 September, 2000 (08.09.00), & EP 1157061 A1 | 1-5,10, 15-42<br>6-9,11-14 |
| X<br><br>Y | WO 99/45048 A1 (E.I. Du Pont De Nemours and Co.), 10 September, 1999 (10.09.99), & EP 1060200 A1 & JP 2002-505356 A | 1-5,10, 15-42<br>6-9,11-14 |
| X<br><br>Y | WO 99/38842 A1 (Hydro-Quebec), 05 August, 1999 (05.08.99), & EP 973734 A1 & JP 2001-522376 A | 1-5,10, 15-42<br>6-9,11-14 |
| X<br><br>Y | EP 302478 A1 (Tosoh Corp.), 08 February, 1989 (08.02.89), & JP 1-40498 A & US 4944852 A | 1-5,10, 15-36<br>6-9,11-14 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 May, 2002 (28.05.02) | 11 June, 2002 (11.06.02) |
| Name and mailing address of the ISA/<br>Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/00854

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y | JP 62-104891 A (Toyo Soda Manufacturing Co., Ltd.),<br>15 May, 1987 (15.05.87),<br>Pages 2 to 6<br>(Family: none) | 1-5,10,<br>15-36<br>6-9,11-14 |
| Y | US 4578512 A (Dow Chemical Co.),<br>25 March, 1986 (25.03.86),<br>& JP 57-28025 A | 6-9 |
| Y | JP 56-138127 A (Daikin Industries, Ltd.),<br>28 October, 1981 (28.10.81),<br>Pages 3 to 4<br>(Family: none) | 11,12 |
| Y | WO 94/27945 A1 (E.I. Du Pont De Nemours and Co.),<br>08 December, 1994 (08.12.94),<br>& US 5350497 A & JP 9-501406 A<br>& EP 699176 A1 | 13,14 |
| X | ZHU, Shizheng et al., "Synthesis of diethyl N-(perfluoroalkanesulfonyl) phosphoramidates and N-(perfluoroalkanesulfonyl) phosphoramidic acids", Phosphorus, Sulfur and Silicon and the Related Elements, 1998, Vol.140, pages 53 to 61 | 8 |
| X | KENNEDY, Gerald L. Jr., "Increase in mouse liver weight following feeding of ammonium perfluoro-octanoate and related fluorochemicals", Toxicol. Lett., 1987, Vol.39, Nos. 2 to 3, pages 295 to 300 | 14 |

Form PCT/ISA/210 (continuation of second sheet) (July 1998)

## INTERNATIONAL SEARCH REPORT

International application No.
PCT/JP02/00854

**Box I Observations where certain claims were found unsearchable (Continuation of item 1 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

The subject matters of the claims are classified into the following groups.

① Claims 1-19 and
that part of claims 22-24 and 30-42 which relates to the compound (1)

② Claims 20-21 and 25-29 and
that part of claims 22-24 and 30-42 which does not relate to the compound (1)

Between these groups, there is no relationship involving any special technical feature common to these. Consequently, these groups are not considered to be so linked as to form a single general inventive concept.

Therefore, the number of inventions disclosed in the claims of this

1. ☒ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.

☒ No protest accompanied the payment of additional search fees.

Form PCT/ISA/210 (continuation of first sheet (1)) (July 1998)

**INTERNATIONAL SEARCH REPORT**

International application No.
PCT/JP02/00854

Continuation of Box No.II of continuation of first sheet(1)

international application is 2.

Form PCT/ISA/210 (extra sheet) (July 1998)